(19) Europäisches Patentamt
European Patent Office
Office européen des brevets

(11) EP 1 410 784 B1

(12) **FASCICULE DE BREVET EUROPEEN**

(45) Date de publication et mention de la délivrance du brevet:
**07.02.2007 Bulletin 2007/06**

(51) Int Cl.:
***A61Q 1/02*** *(2006.01)* ***A61K 8/49*** *(2006.01)*

(21) Numéro de dépôt: **03292466.4**

(22) Date de dépôt: **07.10.2003**

(54) **Composition cosmétique comprenant une phase huileuse et un colorant de type naphtopyranne et procédé de traitement cosmétique**

Kosmetische Zusammensetzung enthaltend eine Ölphase und einen Farbstoff aus der Gruppe der Naphthopyrane sowie Methode

Cosmetic composition comprising an oil phase and a naphthopyran dye and method

(84) Etats contractants désignés:
**AT BE BG CH CY CZ DE DK EE ES FI FR GB GR HU IE IT LI LU MC NL PT RO SE SI SK TR**

(30) Priorité: **18.10.2002 FR 0213003**
**18.10.2002 FR 0213004**

(43) Date de publication de la demande:
**21.04.2004 Bulletin 2004/17**

(73) Titulaire: **L'ORÉAL**
**75008 Paris (FR)**

(72) Inventeurs:
- **Blin, Xavier**
**75015 Paris (FR)**
- **Simon, Jean-Christophe**
**Shinjuku-ku**
**Tokyo 162-0842 (JP)**

(74) Mandataire: **Dodin, Catherine**
**L'OREAL - D.I.P.I.**
**25-29 Quai Aulagnier**
**92600 Asnières (FR)**

(56) Documents cités:
**EP-A- 0 970 689** **EP-B- 0 973 761**
**WO-A-02/078665** **US-A1- 2002 122 780**

- **DATABASE WPI Derwent Publications Ltd., London, GB; AN 2002-073025 [10] XP002246063 & KR 2001 069 807 A (MAGICHITECH), 25 juillet 2001 (2001-07-25)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 410 784 B1

## Description

La présente invention a trait à de nouvelles compositions cosmétiques, et notamment de nouvelles compositions de maquillage, comprenant des colorants organiques notamment photochromes.

Les compositions de maquillage telles que les poudres libres ou compactes, les fonds de teint, les fards à joues ou à paupières ou les rouges à lèvres, sont généralement constituées d'un véhicule approprié et d'un ou plusieurs agents de coloration destinés à conférer une certaine couleur auxdites compositions avant et/ou après leur application sur la peau, les muqueuses, les semi-muqueuses et/ou les phanères telles que les ongles ou les cheveux.

Pour créer des couleurs, on utilise aujourd'hui une gamme d'agents de coloration assez limitée comprenant notamment des laques, des pigments minéraux, des pigments organiques et des pigments nacrés. Les laques permettent d'obtenir des couleurs vives, mais pour la plupart sont instables à la lumière, à la température ou au pH. Certaines présentent également l'inconvénient de tacher la peau de manière disgracieuse après application, par dégorgement du colorant. Les pigments minéraux, en particulier les oxydes minéraux sont au contraire très stables mais donnent des couleurs plutôt ternes et pâles. Les pigments nacrés quant à eux permettent d'obtenir des couleurs variées mais peu intenses, qui conduisent à des effets irisés mais le plus souvent assez faibles.

Dans le domaine de la coloration capillaire temporaire ou fugace, qui donne lieu à une modification légère de la couleur naturelle de la chevelure qui tient d'un shampooing à l'autre et qui sert à embellir ou corriger une nuance déjà obtenue, on a déjà proposé une coloration avec des pigments usuels pour apporter un reflet temporaire aux cheveux, mais les nuances obtenues par cette coloration restent assez ternes, trop uniformes et peu ludiques. On peut notamment assimiler une telle coloration à un "maquillage" des cheveux.

Depuis peu, il est également proposé d'utiliser des composés photochromes dans les compositions de maquillage ou capillaire, de manière à obtenir des changements agréables, ludiques et variables dans le 'rendu couleur' des maquillages de la peau et/ou des cheveux.

Les composés photochromes sont des composés qui possèdent la propriété de changer de couleur lorsqu'ils sont irradiés par une source lumineuse, puis de reprendre leur couleur initiale, ou une couleur proche, lorsque l'on arrête l'irradiation. De tels composés trouvent notamment une application particulièrement intéressante dans les compositions cosmétiques, en particulier dans les compositions de maquillage telles que les fonds de teint, les fards à joues ou à paupières et les rouges à lèvres

En effet, on a constaté que le 'rendu maquillage' d'une peau maquillée est différent selon que l'on se trouve en éclairage naturel ou en éclairage artificiel. Ainsi, un maquillage réalisé en éclairage artificiel paraîtra plus clair en lumière naturelle. A l'inverse, un maquillage réalisé à l'extérieur paraîtra plus sombre dans un endroit éclairé artificiellement.

Pour pallier ce problème, il a été proposé, par exemple par le brevet EP359909, des compositions cosmétiques comprenant des composés photochromes minéraux particulier, choisis parmi les oxydes métalliques, leurs hydrates et leurs complexes. En particulier, ce document mentionne l'utilisation d'oxyde de titane traité de manière à le rendre photochrome, dans des compositions de maquillage telles que des poudres et des fonds de teint.

Toutefois, on a constaté que ces composés photochromes minéraux, bien qu'ils permettent d'obtenir un maquillage qui semble rester de couleur constante quel que soit l'éclairage, ne permettent toutefois pas d'obtenir un véritable changement de la couleur du maquillage, ou, en d'autres termes, un véritable changement du 'rendu maquillage'.

D'autre part, on a également constaté qu'après l'arrêt de l'irradiation lumineuse, la couleur du maquillage ne revenait pas toujours d'une manière acceptable à sa couleur initiale, en particulier qu'elle ne revenait pas complètement à une couleur identique à ladite couleur initiale et/ou pas de manière assez rapide.

Il a également été proposé d'utiliser des composés photochromes organiques, tels que les composés de la famille des spiropyrannes ou des naphtooxazines. Ces composés photochromes sont particulièrement intéressants dans la mesure où ils permettent d'obtenir un changement rapide de coloration du support sur lequel ils sont appliqués, lorsque ledit support est exposé aux UV par exemple, avec un retour rapide à la couleur initiale lorsque l'exposition aux UV cesse.

On peut ainsi citer le brevet FR1604929 qui décrit des compositions cosmétiques notamment capillaires sous forme d'aérosol, qui contiennent des composés photo-tropes tels que des nitrobenzylpyridines, des thiosemicarbazones ou des dérivés de spiropyrannes. Après sprayage de ces compositions sur les cheveux et exposition à la lumière du soleil, on obtient une coloration bleu-violet qui redevient jaune pâle dans l'obscurité.

On connaît également par EP970689 un produit cosmétique de maquillage comprenant des première et seconde compositions conditionnées séparément, la première composition contenant un agent de coloration photochrome susceptible de produire au moins une couleur en présence d'UV et la seconde composition contenant au moins un filtre UV. Toutefois, la dynamique de ces compositions est faible : notamment, le retour à l'état initial se fait lentement.

On connaît également par la demande WO02/078665, une composition cosmétique comprenant des matières colorantes liquides, absorbeurs d'UV, de type naphtopyrannes ou naphtoxazines, et qui sont encapsulées dans une capsule à base de copolymères acrylates, et qui peuvent être employées dans le but d'absorber les UV. Toutefois, il n'y est nullement fait mention d'une quelconque propriété photochrome de ces compositions ou matières colorantes.

La présente invention propose de pallier certains des inconvénients de l'art antérieur, grâce à de nouvelles

compositions cosmétiques comprenant des colorants organiques particuliers, en association avec une phase huileuse particulière, qui peut être, mais pas obligatoirement, au moins partiellement siliconée.

Il est connu que, parmi les huiles susceptibles d'être employées en cosmétique, les huiles de silicone apportent des propriétés particulières telles qu'un certain glissant, et une bonne filmogénicité. En particulier, par application sur la peau ou les lèvres, elles conduisent à la formation d'un dépôt présentant une homogénéité, une douceur et une brillance satisfaisante. D'autre part, leur bonne propriété de mouillage leur confère une résistance aux agressions, notamment à l'eau, qui participe à la bonne tenue de la composition.

Il peut donc être particulièrement intéressant de disposer d'une composition cosmétique comprenant à la fois de telles huiles de silicone et des colorants photochromes tels que ci-après définis.

Or, la demanderesse a constaté qu'il n'était pas possible de formuler une composition cosmétique comprenant l'association desdits colorants photochromes et des huiles siliconées usuellement employées, notamment parce que lesdits colorants n'étaient pas solubilisables dans ces huiles de silicone.

Il revient à la demanderesse d'avoir mis en évidence un moyen pour permettre la solubilisation, et donc la formulation, de ces colorants photochromes dans les huiles de silicone.

Un premier objet de la présente invention est une composition cosmétique comprenant, dans un milieu cosmétiquement acceptable, au moins une phase huileuse, notamment siliconée et au moins un colorant tel que ci-après défini.

On a en effet constaté de manière surprenante que les colorants notamment photochromes étaient tout particulièrement solubles dans les huiles de silicone phénylées; il devient ainsi possible de les formuler, soit par exemple en n'utilisant que des huiles siliconées phénylées dans la phase huileuse de la composition, soit en les 'pré-solubilisant' dans lesdites huiles siliconées phénylées puis en ajoutant ensuite à ladite solution, les autres huiles, siliconées ou non, que l'on souhaite employer. Dans ce second cas, les huiles siliconées phénylées sont donc employées comme 'agent solubilisant' ou 'agent compatibilisant' desdits colorants.

En outre, on a constaté de manière surprenante que selon la polarité de l'huile ou du mélange d'huiles formant la phase huileuse de la composition cosmétique, il est possible de moduler la couleur de la composition initiale, avant irradiation.

Ainsi, lorsque la phase huileuse est polaire ou plutôt polaire, elle présente en présence des colorants photochromes selon l'invention une coloration qui peut aller, par exemple, du rose clair au violet en passant par l'orangé, en fonction bien évidemment de la nature chimique du colorant, de sa quantité, ainsi que, notamment, de la polarité et de la nature chimique de ladite phase huileuse.

Lorsque la phase huileuse est plutôt apolaire ou faiblement polaire, on a constaté que sa couleur n'est que peu ou pas perturbée par la présence des colorants photochromes avant irradiation; la phase huileuse reste quasiment incolore.

Les colorants photochromes selon l'invention sont donc également solvatochromes, c'est-à-dire qu'ils changent de couleur en fonction de la nature (polarité) du solvant dans lequel ils se trouvent.

Ceci permet par exemple notamment d'obtenir un produit cosmétique photochrome complètement incolore sans excitation UV, puis un effet tranché (incolore / coloré) après irradiation.

De plus, si la composition est pigmentée par ailleurs, l'absence de couleur apportée par le colorant photochrome selon l'invention permet de ne pas perturber la couleur initiale.

On peut également obtenir un produit cosmétique photochrome légèrement coloré sans UV, ce qui peut permettre d'en visualiser le dépôt et d'en faciliter l'application avant excitation et intensification de la couleur.

Grâce aux colorants notamment photochromes et solvatochromes selon l'invention, il est possible non seulement de moduler la couleur conférée par une composition cosmétique comprenant lesdits colorants, par irradiation (photochromisme) mais également de moduler la couleur de la composition initiale, avant irradiation.

Un autre objet de l'invention est une composition cosmétique comprenant, dans un milieu cosmétiquement acceptable, au moins une phase huileuse comprenant par exemple une huile polaire et/ou apolaire, et au moins un colorant tel que ci-après défini.

A titre avantageux, les compositions cosmétiques selon l'invention permettent l'obtention d'un maquillage susceptible d'un changement de couleur rapide en fonction de la nature et/ou de l'intensité de la lumière.

L'utilisation des colorants selon l'invention permet également d'obtenir un maquillage de couleur nette et pure, avec un 'rendu maquillage' qui parait plus intense.

Le changement de couleur obtenu à l'aide de ces colorants est rapide; il est également réversible et permet de revenir, après l'arrêt de l'irradiation par une source lumineuse, à la couleur initiale, et ce également de manière rapide.

Par ailleurs, il est ainsi possible d'utiliser, dans la composition cosmétique selon l'invention, une quantité de colorants photochromes plus faible que celle généralement utilisée dans l'art antérieur, par exemple de l'ordre de 0,01 à 2% en poids, tout en obtenant un effet de maquillage et une transparence comparables.

En outre, les bonnes propriétés cosmétiques des compositions selon l'invention sont maintenues.

De plus, bien que de structure chimique proche, les colorants photochromes selon l'invention présentent une grande variété de couleur qui peut aller du rouge au bleu en passant par le gris, avec des variations plus ou moins foncées.

Ceci permet notamment une formulation plus aisée desdits colorants, ne nécessitant pas, pour chaque modification de teinte, une adaptation de la composition.

Les colorants organiques selon l'invention sont des naphtopyrannes, et notamment des 3H-naphto-[2,1-b]-pyrannes qui peuvent être représentés par la formule (I) ou des 2H-naphto-[1,2-b]-pyrannes qui peuvent être représentés par la formule (IIa) :

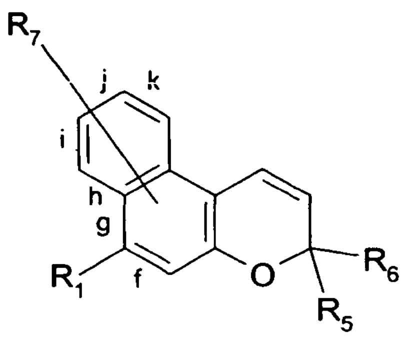

(I)

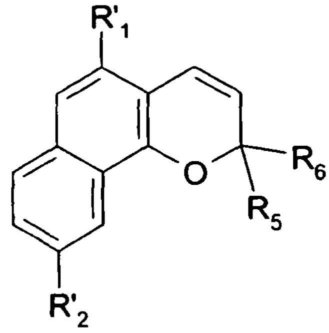

(IIa)

dans lesquelles :

* R1 représente :

- (i) un groupement hydrocarboné ayant 1 à 30, de préférence 1-18, et mieux 1 à 12, voire 1-6, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl;
- (ii) un cycle hydrocarboné formé avec l'une des liaisons "f" ou "gh" et le radical R7; ou
- (iii) un groupement choisi parmi -COOR4, -C(O)NR2R3, -NR2R3, -OR4 et -SR4, dans lequel :

  - R2 et R3 soit représentent, indépendamment l'un de l'autre, un groupement hydrocarboné ayant 1 à 20, de préférence 1-12, et mieux 1 à 6, atomes de carbone, linéaire, ramifié ou cyclique, saturé ou insaturé, éventuellement comportant 1 à 5 hétéroatomes choisis parmi N, O, S, Si et P,
    soit pris ensemble avec l'atome d'azote auquel ils sont reliés, forment un hétérocycle hydrocarboné, saturé ou insaturé, comportant 3-10, de préférence 4-6, atomes de carbone et éventuellement 1-5 autres hétéroatomes choisis parmi N, O, S, Si et P, ledit cycle étant éventuellement substitué par au moins un radical hydrocarboné ayant 1 à 20, de préférence 1-15, et mieux 1 à 6, atomes de carbone, linéaire, ramifié ou cyclique, saturé ou insaturé, comportant éventuellement 1 à 5 hétéroatomes choisis parmi N, O, S, Si et P;
  - R4 représente un groupement hydrocarboné ayant 1 à 20, de préférence 1-15, et mieux 1 à 6, atomes de carbone, linéaire, ramifié ou cyclique, saturé ou insaturé, éventuellement halogéné ou perhalogéné, notamment par Fl, Br et/ou Cl, et/ou éventuellement comportant 1 à 5 hétéroatomes choisis parmi N, O, S, Si et P;

* R5 et R6 représentent, indépendamment l'un de l'autre, un groupement choisi parmi :

- (i) les groupements aminoaryles cycliques saturés de formule (IIa) ou (IIb) :

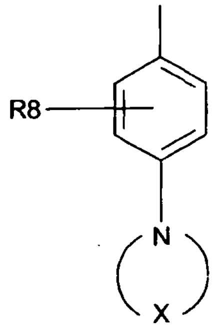

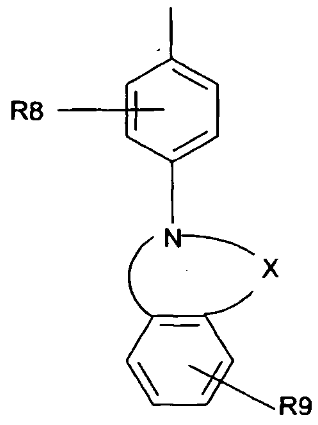

**(IIa)** **(IIb)**

dans lesquelles le cycle comportant N et X est un cycle saturé qui comprend au total 3 à 30 atomes, de préférence 4-10 et encore mieux 5, 6 ou 7 atomes, inclus l'azote, le reste étant des atomes de carbone et/ou des hétéroatomes choisis parmi O, S, Si, P et/ou des groupements choisis parmi -NH et -NR avec R représentant un radical hydrocarboné ayant 1 à 20, de préférence 1-15, et mieux 1 à 6, atomes de carbone, linéaire, ramifié ou cyclique, saturé ou insaturé, éventuellement comportant 1 à 5 hétéroatomes choisis parmi N, O, S, Si et P;

- (ii) les groupements indolinoaryles de formule (III) :

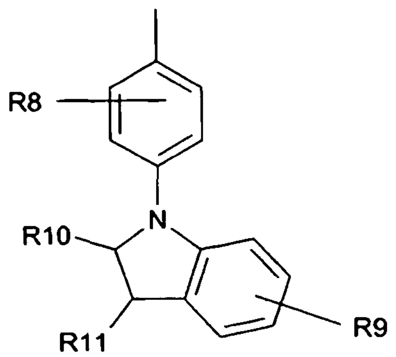

**(III)**

dans laquelle R10 et R11 représentent, indépendamment l'un de l'autre, un groupement choisi parmi (i) les groupements hydrocarbonés ayant 1 à 30, de préférence 1-18, et mieux 1 à 12, voire 1-6, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl; (ii) les atomes d'halogène, et notamment Fl, Br et/ou Cl; (iii) les groupements -CN (nitrile), -COOH (carboxylate), $-NO_2$ (nitro); (iv) un atome d'hydrogène; (v) un groupement choisi parmi -C(O)NR2R3, -NR2R3, -OR4 ou -SR4 avec R2, R3 et R4 ayant les significations données ci-dessus; (vi) les radicaux R10 et R11 pouvant former ensemble un cycle hydrocarboné saturé ou insaturé ayant au total 5 à 8 atomes (incluant les atomes du cycle indoline), lesdits atomes étant choisis parmi C, O, S et/ou NR avec R représentant H ou un radical hydrocarboné ayant 1 à 20, voire 1-12, atomes de carbone, linéaire, ramifié ou cyclique, saturé ou insaturé, éventuellement comportant 1 à 5 hétéroatomes choisis parmi N, O, S, Si et P,

- (iii) les groupements de formule (IV) :

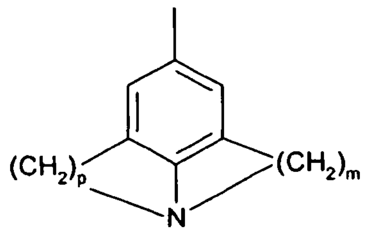

(IV)

dans laquelle m et p sont, indépendamment l'un de l'autre, des entiers allant de 2 à 5;

- (iv) les groupements aminoaryles cycliques insaturés de formules (Va), (Vb) ou (Vc) :

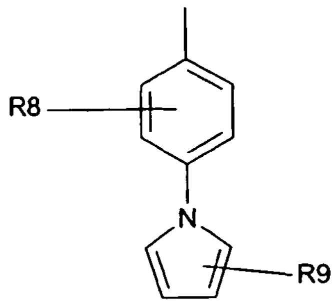

(Va)

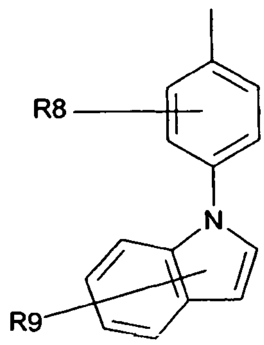

(Vb)

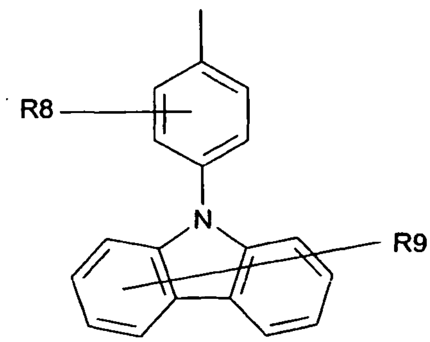

(Vc)

dans lesquelles R8 et R9, représentent, indépendamment l'un de l'autre, un groupement choisi parmi (i) les groupements hydrocarbonés ayant 1 à 30, de préférence 1-18, et mieux 1 à 12, voire 1-6, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl; (ii) les atomes d'halogène, et notamment Fl, Br et/ou Cl; (iii) les groupements -CN (nitrile), -COOH (carboxylate), $-NO_2$ (nitro); (iv) un atome d'hydrogène; (v) un groupement choisi parmi -C(O)NR2R3, -NR2R3, -OR4 ou -SR4 avec R2, R3 et R4 ayant les significations données ci-dessus;

- (v) un groupement hydrocarboné ayant 1 à 30, de préférence 2-18, et mieux 3 à 12, atomes de carbone, linéaire, ramifié ou cyclique, saturé ou insaturé, éventuellement comportant 1 à 5 hétéroatomes choisis parmi N, O, S, Si et P; et notamment un groupement choisi parmi $-C_6H_4$-CONR2R3, $-C_6H_4$-NR2R3 et $-C_6H_4$-OR4 avec R2,

R3 et R4 ayant les significations données ci-dessus;

* R7 représente un groupement choisi parmi :

- (i) les groupements hydrocarbonés ayant 1 à 30, de préférence 1-18, et mieux 1 à 12, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl;
- (ii) les atomes d'halogène, et notamment Fl, Br et/ou Cl;
- (iii) les groupements -CN (nitrile), -COOH (carboxylate), $-NO_2$ (nitro); -N=N- (azo); =NH (imino); $-CONH_2$ (amide);
- (iv) un atome d'hydrogène;
- (v) un groupement choisi parmi -C(O)NR2R3, -NR2R3, -OR4 ou -SR4 avec R2, R3 et R4 ayant les significations données ci-dessus;
- (vi) le radical R7 pouvant en outre former, avec l'une des liaisons "i", "j", "k", ou "g,h" prises avec le radical R1, ou "f" prise avec le radical R1, un cycle hydrocarboné saturé ayant au total 3 à 8, de préférence 4 à 7, et mieux 5 ou 6, atomes de carbone, éventuellement comportant 1 à 5 hétéroatomes choisis parmi N, O, S, Si et P;

* R'1 représente un groupement choisi parmi :

- (i) un atome d'hydrogène;
- (ii) un groupement hydrocarboné ayant 1 à 30, de préférence 1-18, et mieux 1 à 12, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl;
- (iii) un groupement choisi parmi -C(O)NR2R3, -NR2R3, -OR4 et -SR4, avec R2, R3 et R4 ayant les significations données ci-dessus;

* R'2 représente un groupement choisi parmi :

- (i) les groupements hydrocarbonés ayant 1 à 30, de préférence 1-18, et mieux 1 à 12, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl;
- (ii) les atomes d'halogène, et notamment Fl, Br et/ou Cl;
- (iii) les groupements -CN (nitrile), -COOH (carboxylate), $-NO_2$ (nitro); -N=N- (azo); =NH (imino); $-CONH_2$ (amide);
- (iv) un atome d'hydrogène;
- (v) un groupement choisi parmi -C(O)NR2R3, -NR2R3, -OR4 ou -SR4 avec R2, R3 et R4 ayant les significations données ci-dessus.

De préférence, les colorants organiques selon l'invention répondent à la formule (Ia) suivante :

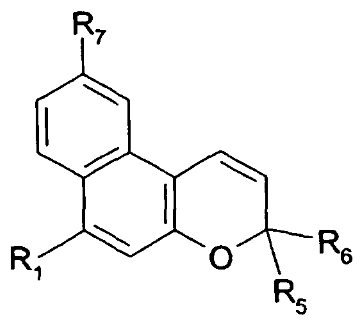

(Ia) ,

dans lesquelles R1, R5, R6 et R7 sont définis tels que précédemment.

De préférence, R1 représente un atome d'hydrogène; un cycle hydrocarboné avec l'une des liaisons "f" ou "gh" et le radical R7; ou un groupement choisi parmi - COOR4, -NR2R3, -OR4 et -SR4, dans lequel :

- R2 et R3 soit représentent, indépendamment l'un de l'autre, un groupement hydrocarboné ayant 1 à 20, de préférence 1-12, et mieux 1 à 6, atomes de carbone, linéaire, ramifié ou cyclique, saturé ou insaturé, éventuellement comportant 1 à 5 hétéroatomes choisis parmi N, O, S, Si et P, soit pris ensemble avec l'atome d'azote auquel ils sont reliés, forment un hétérocycle hydrocarboné, saturé ou insaturé, comportant 3-10, de préférence 4-6, atomes de carbone et éventuellement 1-5 autres hétéroatomes choisis parmi N, O, S, Si et P, ledit cycle étant éventuellement substitué par au moins un radical hydrocarboné ayant 1 à 20, de préférence 1-15, et mieux 1 à 6, atomes de carbone, linéaire, ramifié ou cyclique, saturé ou insaturé, comportant éventuellement 1 à 5 hétéroatomes choisis parmi N, O, S, Si et P;
- R4 représente un groupement hydrocarboné ayant 1 à 20, de préférence 1-15, et mieux 1 à 6, atomes de carbone, linéaire, ramifié ou cyclique, saturé ou insaturé, éventuellement halogéné ou perhalogéné, notamment par Fl, Br et/ou Cl, et/ou éventuellement comportant 1 à 5 hétéroatomes choisis parmi N, O, S, Si et P.

De préférence R5 et R6 représentent, indépendamment l'un de l'autre, un groupement choisi parmi :

- les groupements aminoaryles cycliques saturés de formule (IIa) ou (IIb) :

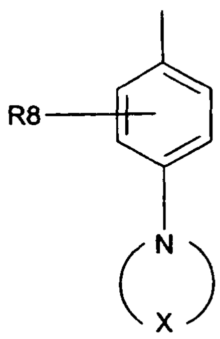

(IIa)

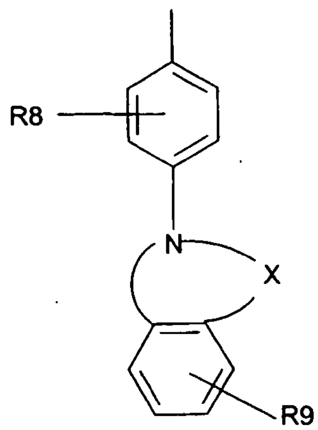

(IIb)

dans lesquelles le cycle comportant N et X est un cycle saturé qui comprend au total 3 à 30 atomes, de préférence 4-10 et encore mieux 5, 6 ou 7 atomes, inclus l'azote, le reste étant des atomes de carbone et/ou des hétéroatomes choisis parmi O, S, Si, P et/ou des groupements choisis parmi -NH et -NR avec R représentant un radical hydrocarboné ayant 1 à 20, de préférence 1-15, et mieux 1 à 6, atomes de carbone, linéaire, ramifié ou cyclique, saturé ou insaturé, éventuellement comportant 1 à 5 hétéroatomes choisis parmi N, O, S, Si et P;

- un groupement hydrocarboné ayant 1 à 30, de préférence 2-18, et mieux 3 à 12, atomes de carbone, linéaire, ramifié ou cyclique, saturé ou insaturé, éventuellement comportant 1 à 5 hétéroatomes choisis parmi N, O, S, Si et P; et notamment un groupement choisi parmi $-C_6H_4$-CONR2R3, $-C_6H_4$-NR2R3 et $-C_6H_4$-OR4 avec R2, R3 et R4 ayant les significations données ci-dessus;

De préférence, R7 représente un groupement choisi parmi :

- (i) les groupements hydrocarbonés ayant 1 à 30, de préférence 1-18, et mieux 1 à 12, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl;
- (ii) les atomes d'halogène, et notamment Fl, Br et/ou Cl;
- (iii) les groupements -CN (nitrile), -COOH (carboxylate), $-NO_2$ (nitro); -N=N- (azo); =NH (imino); $-CONH_2$ (amide);
- (iv) un atome d'hydrogène;
- (v) un groupement choisi parmi -NR2R3, -OR4 ou -SR4 avec R2, R3 et R4 ayant les significations données ci-dessus;
- (vi) le radical R7 pouvant en outre former, avec l'une des liaisons "i", "j", "k", ou "g,h" prises avec le radical R1, ou "f" prise avec le radical R1, un cycle hydrocarboné saturé ayant au total 3 à 8, de préférence 4 à 7, et mieux 5 ou 6, atomes de carbone, éventuellement comportant 1 à 5 hétéroatomes choisis parmi N, O, S, Si et P.

De préférence, dans les formules (I) ou (la), au moins l'un des groupements R1 ou R7 est différent de l'hydrogène.
De préférence, dans les formules (I) ou (la), au moins l'un des groupements R5 ou R6 est différent d'un radical phényle ($-C_6H_5$).
De préférence également, dans les formules (I) ou (la), au moins l'un des groupements R1, R7, R5 ou R6, comprend un atome d'azote.
De préférence, dans les formules (I) ou (la), au moins l'un des groupements R5 ou R6 représente un radical phényl substitué.
De préférence, R'1 représente l'hydrogène ou un groupement hydrocarboné ayant 1 à 30, de préférence 1-18, et mieux 1 à 12, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl.
De préférence, R'2 représente l'hydrogène ou un groupement choisi parmi $-NO_2$, -NR2R3 et -C(O)NR2R3, dans lesquels R2 et R3, soit représentent, indépendamment l'un de l'autre, un groupement hydrocarboné ayant 1 à 20, de préférence 1-12, et mieux 1 à 6, atomes de carbone, linéaire, ramifié ou cyclique, saturé ou insaturé, éventuellement comportant 1 à 5 hétéroatomes choisis parmi N, O, S, Si et P; soit pris ensemble avec l'atome d'azote auquel ils sont reliés, forment un hétérocycle hydrocarboné, saturé ou insaturé, comportant 3-10, de préférence 4-6, atomes de carbone et éventuellement 1-5 autres hétéroatomes choisis parmi N, O, S, Si et P, ledit cycle étant éventuellement substitué par au moins un radical hydrocarboné ayant 1 à 20, de préférence 1-15, et mieux 1 à 6, atomes de carbone, linéaire, ramifié ou cyclique, saturé ou insaturé, comportant éventuellement 1 à 5 hétéroatomes choisis parmi N, O, S, Si et P.
On peut plus particulièrement citer les colorants organiques de formule (I) ou (la) pour lesquelles :

* R1 représente l'hydrogène; ou un groupement -COOR avec R étant un radical hydrocarboné saturé ayant 1 à 12, de préférence 1-6, atomes de carbone, et notamment un radical méthyle ou éthyle; ou un groupement

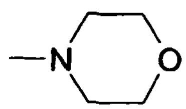

et/ou
* R5 et R6 représentent, indépendamment l'un de l'autre, soit (i) un groupement de formule (IIa) :

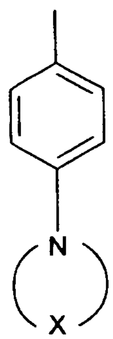

(IIa)

dans laquelle le cycle comportant N et X est un cycle saturé qui comprend au total 4 à 7 atomes, notamment 5 ou 6 atomes, inclus l'azote, et notamment 3-5 atomes de carbone et 0-1 atome d'oxygène; et en particulier un groupement de formule:

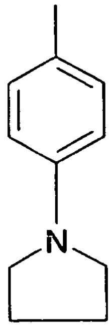

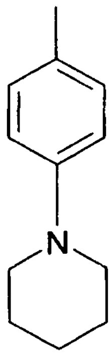

ou

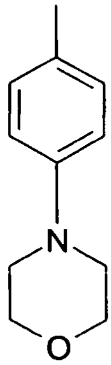

ou

soit (ii) un groupement hydrocarboné ayant 5 à 14, de préférence 6 à 10 atomes de carbone, linéaire, ramifié ou cyclique, saturé ou insaturé, éventuellement comportant 1 à 2 hétéroatomes choisis parmi N, O ou S;
en particulier un groupement

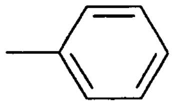

ou

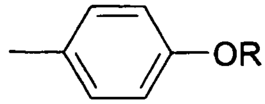

ou

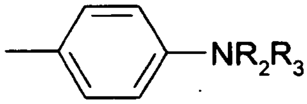

dans lesquelles R est un radical hydrocarboné saturé ayant 1 à 12, de préférence 1-6, atomes de carbone, et notamment un radical méthyle ou éthyle; et R2 et R3 sont, indépendamment l'un de l'autre, un groupement hydrocarboné ayant 1 à 20, de préférence 1-15, et mieux 1 à 6, atomes de carbone, linéaire, ramifié ou cyclique, saturé ou insaturé, éventuellement comportant 1 à 5 hétéroatomes choisis parmi N, O, S, Si et P;
et/ou
* R7 représente un atome d'hydrogène ou un groupement -NR2R3, avec R2 et R3 représentant, indépendamment l'un de l'autre, un groupement hydrocarboné saturé, linéaire ou ramifié, ayant 1 à 12, de préférence 1-6, atomes de carbone, et notamment un groupement méthyle et/ou éthyle.

On peut également citer les colorants organiques de formule (II) ou (IIa) pour lesquelles :

* R'1 représente l'hydrogène ou un groupement -COOR avec R étant un radical hydrocarboné saturé ayant 1 à 12, de préférence 1-6, atomes de carbone, et notamment un radical méthyle ou éthyle;
et/ou
* R5 et R6 représentent, indépendamment l'un de l'autre, soit (i) un groupement de formule (IIa) :

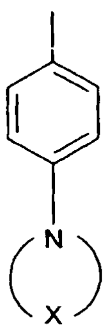

(IIa)

dans laquelle le cycle comportant N et X est un cycle saturé qui comprend au total 4 à 7 atomes, notamment 5 à 6 atomes, inclus l'azote, et notamment 4-5 atomes de carbone et 0-1 atome d'oxygène; et en particulier un groupement de formule:

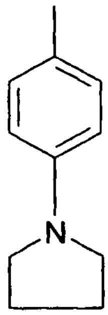 ou 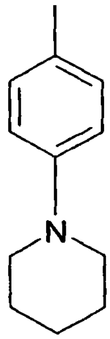 ou 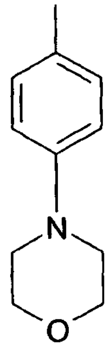

soit (ii) un groupement hydrocarboné ayant 5 à 14, de préférence 6 à 10 atomes de carbone, linéaire, ramifié ou cyclique, saturé ou insaturé, éventuellement comportant 1 à 2 hétéroatomes choisis parmi N, O ou S;
en particulier un groupement :

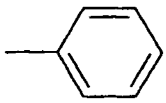 ou 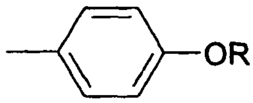 ou 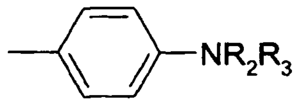

dans lesquelles R est un radical hydrocarboné saturé ayant 1 à 12, de préférence 1-6, atomes de carbone, et notamment un radical méthyle ou éthyle; et R2 et R3 sont, indépendamment l'un de l'autre, un groupement hydrocarboné ayant 1 à 20, de préférence 1-15, et mieux 1 à 6, atomes de carbone, linéaire, ramifié ou cyclique, saturé ou insaturé, éventuellement comportant 1 à 5 hétéroatomes choisis parmi N, O, S, Si et P;
et/ou
* R'2 représente l'hydrogène, ou un groupement -NR'R" avec R' et R", identiques ou différents, représentant un groupement hydrocarboné saturé, linéaire ou ramifié, ayant 1 à 12, de préférence 1-6, atomes de carbone, et notamment un groupement méthyle et/ou éthyle; ou un groupement

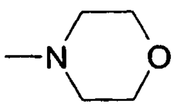

Parmi ces colorants organiques, on peut citer ceux décrits dans les demandes WO94/22850, WO98/45281 et WO00/18755.

On peut notamment citer les composés suivants, plus particulièrement préférés :

- le 3,3-di(4-méthoxyphényl)-6-morpholino-3H-naphto[2,1-b]pyran de formule :

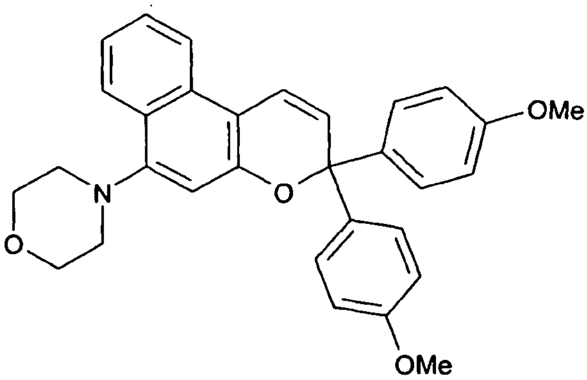

- le 3-phényl-3-(4-morpholinophényl)-6-morpholino-3H-naphto[2,1-b]pyran de formule :

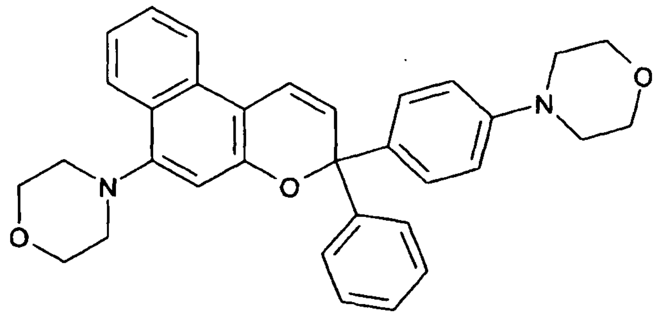

- le 3-phényl-3-(4-pipéridinophényl)-6-morpholino-3H-naphto[2,1-b]pyran de formule :

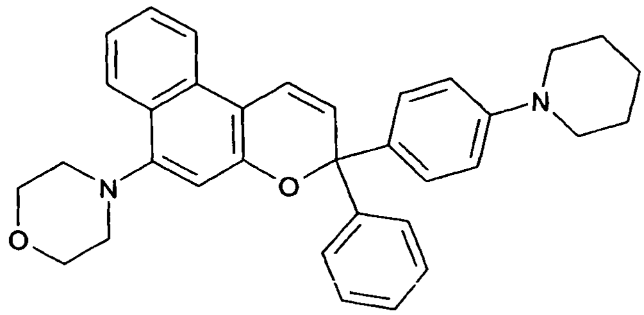

- le 3-phényl-3-(4-pipéridinophényl)-6-carboxyméthyl-9-N-diméthyl-3H-naphto[2,1-b]pyran de formule :

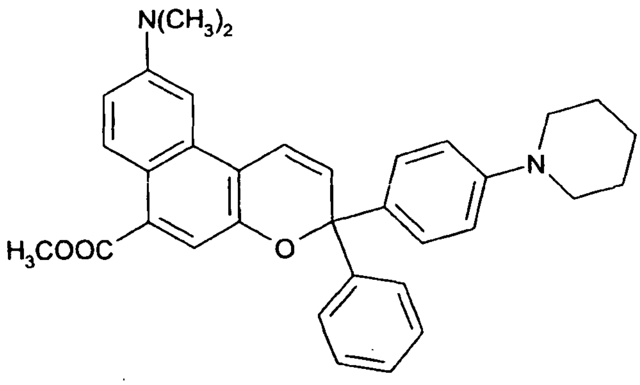

- le 2-phényl-2-(4-piperidinophényl)-5-carboxyméthyl-9-N-diméthyl-2H-naphto[1,2-b]pyran de formule :

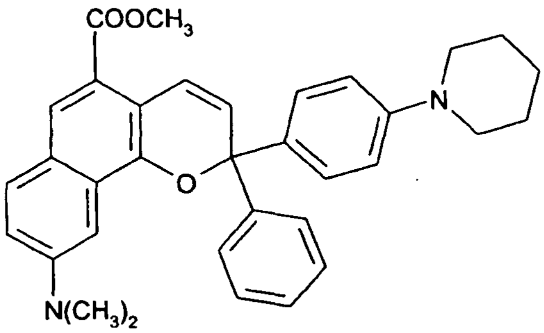

- leurs mélanges.

Les colorants organiques selon l'invention sont de préférence compris, seul ou en mélange, en une quantité de 0,001 à 20% en poids, notamment de 0,005 à 10 % en poids, de préférence 0,01 à 4,8% en poids, et encore mieux de 0,05 à 3% en poids, préférentiellement de 0,1 à 2% en poids, notamment, 0,15-1% en poids, voire 0,2-0,8% en poids, par rapport au poids total de la composition cosmétique.

De préférence, ces colorants organiques sont photochromes, c'est-à-dire qu'ils présentent un $\Delta E$ supérieur ou égal à 5, de préférence supérieur ou égal à 10, mieux supérieur ou égal à 25, encore mieux supérieur ou égal 35, voire supérieur ou égal à 45; le $\Delta E$ est mesuré de la manière définie avant les exemples.

Avantageusement, les colorants organiques selon l'invention sont solubles dans la phase huileuse de la composition.

Par soluble, on entend que les colorants forment un mélange homogène, sans particules apparentes à l'oeil nu, à 25°C, 1 atm, à raison de 0,05% en poids, dans la phase huileuse.

La composition selon l'invention comprend en outre une phase huileuse qui peut comprendre une ou plusieurs huiles cosmétiquement acceptables.

Par huile cosmétiquement acceptable selon l'invention, on entend tout corps gras liquide à 25°C, 1 atm, et ayant de préférence un poids moléculaire supérieur ou égal à 160, notamment compris entre 170 et $10^6$, voire entre 200 et $5.10^5$, compatible avec une application sur la peau, les muqueuses (lèvres) et/ou les phanères (ongles, cils, sourcils, cheveux).

De préférence, la phase huileuse est macroscopiquement homogène, c'est-à-dire homogène à l'oeil nu.

Dans un premier mode de réalisation particulier de l'invention, la phase huileuse est polaire et peut comprendre principalement, voire exclusivement, une ou plusieurs huiles polaires (plutôt ou franchement polaires) en mélange, qui peuvent donc représenter 5 à 100% en poids, notamment 10-90%, voire 15-60% et encore mieux 20-50% en poids du poids total de ladite phase huileuse.

Selon ce premier mode préféré, la phase huileuse polaire présente de préférence un paramètre moyen de solubilité $\delta_a$ selon l'espace de solubilité de Hansen, à 25°C, supérieur ou égal à 5,0 $(J/cm^3)^{½}$, notamment supérieur ou égal à 5,3, voire supérieur ou égal à 5,5 et encore mieux supérieur ou égal à 6,0 $(J/cm^3)^{½}$, voire supérieur ou égale à 7,0 $(J/cm^3)^{½}$.

On entend par huile polaire, une huile composée de composés chimiques comportant au moins un groupement polaire. Les groupements polaires sont bien connus de l'homme du métier : il peut s'agir notamment de groupement de type alcool, ester ou acide carboxylique.

En particulier, on peut définir les huiles polaires selon l'invention comme ayant un paramètre moyen de solubilité $\delta_a$ selon l'espace de solubilité de Hansen, à 25°C, de : $\delta_a \geq 5,0$ $(J/cm^3)^{½}$.

Les huiles polaires comprennent les huiles plutôt polaires qui ont un paramètre moyen de solubilité à 25°C de : $5,0 \leq \delta_a \leq 7,0$ $(J/cm^3)^{½}$, et les huiles franchement polaires qui ont un paramètre moyen de solubilité à 25°C de : $\delta_a > 7,0$ $(J/cm^3)^{½}$.

De la même manière, les huiles apolaires au sens de l'invention ont un paramètre moyen de solubilité $\delta_a$ selon l'espace de solubilité de Hansen, à 25°C, de : $0 < \delta_a < 5,0$ $(J/cm^3)^{½}$.

Les huiles apolaires au sens de l'invention comprennent les huiles franchement apolaires ($\delta_a = 0$) et les huiles peu polaires qui ont un paramètre moyen de solubilité à 25°C de : $0 < \delta_a < 5,0$ $(J/cm^3)^{½}$.

Ainsi, plus la valeur de $\delta_a$ est élevée, plus la polarité de l'huile est élevée.

La définition et le calcul des paramètres de solubilité dans l'espace de solubilité tridimensionnel de HANSEN sont décrits dans l'article de C. M. HANSEN : "The three dimensionnal solubility parameters" J. Paint Technol. 39, 105 (1967).

Selon cet espace de Hansen :

- $\delta_D$ caractérise les forces de dispersion de LONDON issues de la formation de dipôles induits lors des chocs moléculaires ;

- $\delta_p$ caractérise les forces d'interactions de DEBYE entre dipôles permanents ainsi que les forces d'interactions de KEESOM entre dipôles induits et dipôles permanents;
- $\delta_h$ caractérise les forces d'interactions spécifiques (type liaisons hydrogène, acide/base, donneur/accepteur, etc.) ;
- $\delta_a$ est déterminé par l'équation : $\delta_a = (\delta_p^2 + \delta_h^2)^{½}$

Les paramètres $\delta_p$, $\delta_h$, $\delta_D$ et $\delta_a$ sont exprimés en $(J/cm^3)^{½}$.
Lorsque la phase huileuse est un mélange de différentes huiles, les paramètres de solubilité du mélange sont déterminés à partir de ceux des composés pris séparément, selon les relations suivantes :

$$\delta_{Dmel} = \sum_i xi\, \delta_{Di} \quad ; \quad \delta_{pmel} = \sum_i xi\, \delta_{pi} \quad \text{et} \quad \delta_{hmel} = \sum_i xi\, \delta_{hi}$$

$$\delta_{amel} = (\delta^2_{pmel} + \delta^2_{hmel})^{1/2}$$

où xi représente la fraction volumique du composé i dans le mélange.
Il est à la portée de l'homme du métier de déterminer les quantités de chaque huile pour obtenir une phase huileuse satisfaisant aux critères souhaités.

Selon un second mode particulier de réalisation de l'invention, la phase huileuse est apolaire et peut comprendre 5 à 100% en poids, notamment 10-90%, voire 15-60% et encore mieux 20-50% en poids d'une ou plusieurs huiles apolaires (apolaires ou peu polaires); elle présente un paramètre moyen de solubilité $\delta_a$ selon l'espace de solubilité de Hansen, à 25°C, inférieur à 5,0, notamment inférieur ou égal à 4,9, encore mieux inférieur ou égal à 4,5 et encore mieux inférieur ou égal à 4,0 $(J/cm^3)^{½}$.

La phase huileuse, polaire ou apolaire, peut comprendre une ou plusieurs huiles, qui peuvent être elles-même polaires ou apolaires, volatiles ou non volatiles, et de préférence, hydrocarbonées.
Ces huiles peuvent être choisies parmi, seule ou en mélange, les huiles d'origine animale, végétale, minérale ou synthétique; volatiles ou non.
On entend par huile volatile, les huiles ayant, à 25°C, une pression de vapeur comprise entre 0,02 et 300 mm Hg (soir 2,66 à 40000 Pa). De préférence, on utilise des huiles volatiles dont le point éclair est de l'ordre de 30-100°C.
On peut notamment citer :

- les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule $R_1COOR_2$ dans laquelle $R_1$ représente le reste d'un acide gras supérieur comportant de 7 à 19 atomes de carbone et $R_2$ représente une chaîne hydrocarbonée ramifiée contenant de 3 à 20 atomes de carbone, par exemple, l'huile de Purcellin; l'huile de paraffine, de vaseline, de calophyllum, de macadamia, de colza, de coprah, d'arachide, de palme, de ricin, de jojoba, d'olive ou de germes de céréales;l'huile de beurre de karité; le perhydrosqualène;
- les esters et les éthers de synthèse notamment d'acides gras comme les huiles de formule $R_1COOR_2$ dans laquelle $R_1$ représente le reste d'un acide gras supérieur comportant de 7 à 29 atomes de carbone et $R_2$ représente une chaîne hydrocarbonée contenant de 3 à 30 atomes de carbone comme par exemple le myristate d'isopropyle, le palmitate d'éthyl-2hexyle, le stéarate d'octyl-2-dodécyle, l'érucate d'octyl-2-dodécyle, l'isononanoate d'isononyle, l'isostéarate d'isostéaryle; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol; les esters du pentaérythritol comme le tétraisostéarate de pentaérythrithyle ; des esters du type trimellitate de tridécyle;
- des alcools gras ayant de 12 à 26 atomes de carbone comme l'octyldodécanol, le 2-butyloctanol, le 2-hexyldécanol, le 2-undécylpentadécanol, l'alcool oléique.
- des hydrocarbures linéaires ou ramifiés, d'origine minérale ou synthétique tels que les huiles de paraffine et leurs dérivés, la vaseline, les polydécènes, le polyisobutène hydrogéné tel que le parléam; les isoparaffines comme l'isohexadécane et l'isodécane ;
- des glycérides et notamment des acétylglycérides ou des triglycérides d'acides gras ayant 4 à 10 atomes de carbone, comme les triglycérides des acides heptanoïque, octanoïque et des acides caprique/caprylique.

Parmi les huiles polaires particulièrement préférées, on peut citer l'octyldodécanol, l'hexyldécanol, l'octyldécanol, l'alcool oléique, l'huile de ricin, le malate de diisostéaryle, le triheptanoate de glycéryle, le trioctanoate de glycéryle, le triglycéride des acides caprique/caprylique, le triisononanoïn, le trimellitate de tridécyle, et leurs mélanges.

Parmi les huiles apolaires particulièrement préférées, on peut citer les hydrocarbures aliphatiques notamment en C6-C40 comme les huiles de paraffine, volatiles, telles que l'isohexadecane ou l'isododécane, ou non volatiles, et leurs dérivés; la vaseline, les polydécènes hydrogénés ou non, le polyisobutène hydrogéné tel que l'huile de Parléam, le squalane, les polybutylènes, l'isononanoate d'isononyle; les huiles fluorées notamment perfluorées, et leurs mélanges.

On peut notamment citer les huiles suivantes :

| | $\delta_a$ $(J/cm^3)^{1/2}$ |
|---|---|
| huile de ricin | 9,09 |
| 2-hexyldécanol | 8,55 |
| alcool oléïque | 8,17 |
| octyldodécanol | 7,69 |
| triglycéride de l'acide heptanoïque | 7,29 |
| diisostéarylmalate | 7,19 |
| triglycéride de l'acide octanoïque | 6,87 |
| triglycéride des acides caprique/caprylique | 6,69 |
| Triisononanoïn | 6,54 |
| Trimellilate de tridécyle | 5,35 |
| Isononanoate d'isononyle | 4,87 |
| Polyisobutène hydrogéné | 0 |
| Isododécane | 0 |

Selon un troisième mode particulier de réalisation de l'invention, la phase huileuse peut comprendre au moins une huile de siliconé phénylée notamment de formule (VI), ou un mélange de telles huiles :

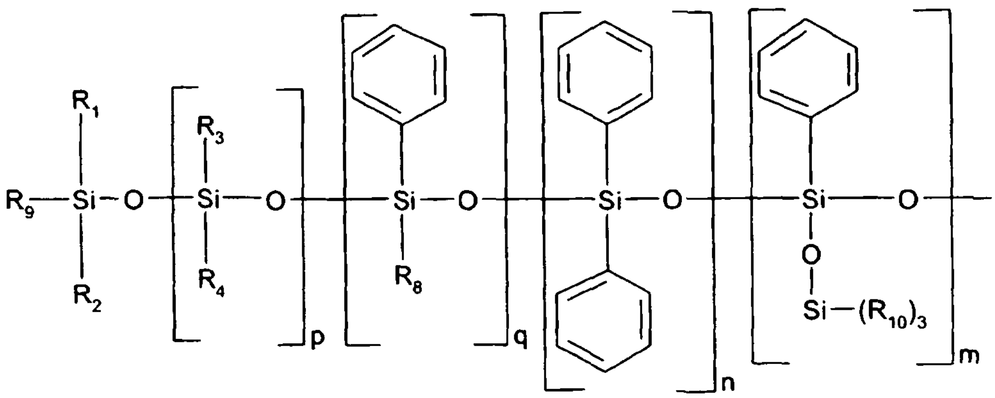

(VI)

dans laquelle

- R1 à R10, indépendamment les uns des autres, sont des radicaux hydrocarbonés, saturés ou insaturés, linéaires, cycliques ou ramifiés, en C1-C30,
- m, n, p et q sont, indépendamment les uns des autres, des nombres entiers compris entre 0 et 900, sous réserve que la somme 'm+n+q' est différente de 0.

De préférence, la somme 'm+n+q' est comprise entre 1 et 100.

De préférence, la somme 'm+n+p+q' est comprise entre 1 et 900, encore mieux entre 1 et 800.
De préférence, q est égal à 0.

De préférence, les huiles de silicone phénylées sont de formule (VII) :

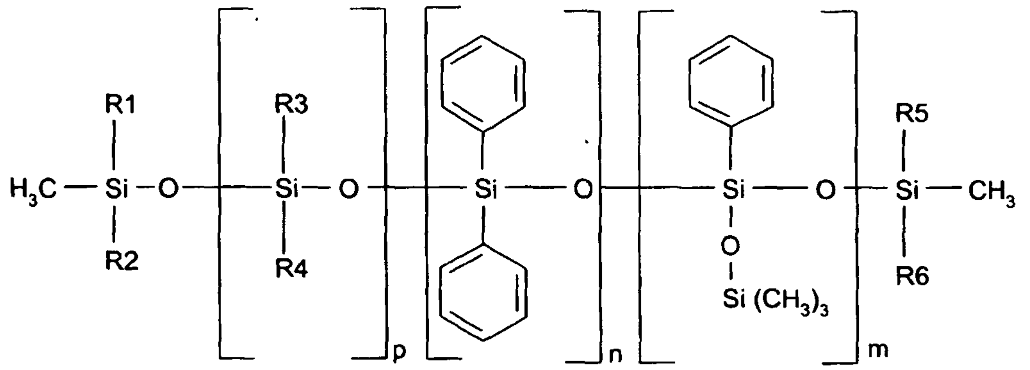
(VII)

dans laquelle :

- R1 à R6, indépendamment les uns des autres, sont des radicaux hydrocarbonés, saturés ou insaturés, linéaires cycliques ou ramifiés, en C1-C30,
- m, n et p sont, indépendamment les uns des autres, des nombres entiers compris entre 0 et 100, sous réserve que la somme 'n + m' est comprise entre 1 et 100.

De préférence, R1 à R6, indépendamment les uns des autres, représentent un radical hydrocarboné saturé, linéaire ou ramifié, en C1-C30, notamment en C1-C12, et en particulier un radical méthyle, éthyle, propyle ou butyle.

Notamment, R1 à R6 peuvent être identiques, et en outre peuvent être un radical méthyle.

De préférence, on peut avoir m=1 ou 2 ou 3, et/ou n=0 et/ou p=0 ou 1.

Les huiles siliconées phénylées peuvent être choisies parmi les phényl trimethicones, les phényl dimethicones, les phényl triméthylsiloxy diphénylsiloxanes, les diphényl diméthicones, les diphényl méthyldiphényl trisiloxanes, et leurs mélanges.

De préférence, le poids moléculaire en masse de l'huile siliconée phénylée est compris entre 500 et 10 000.

On peut notamment citer les huiles de silicones phénylées de formule (VI) ayant une viscosité à 25°C comprise entre 3 et 1500 $mm^2/s$ (soit 3 à 1500 cSt), de préférence ayant une viscosité comprise entre 5 et 1000 $mm^2/s$ (soit 5 à 1000 cSt).
On peut notamment citer les phényltriméthicones telles que la DC556 de Dow Corning (22,5 cSt), l'huile Silbione 70663V30 de Rhône Poulenc (28 cSt), l'huile Abil AV8853 de Goldschmidt (4-6 cSt), ou les diphényldiméthicones telles que les huiles Belsil, notamment Belsil PDM1000 (1000cSt), Belsil PDM 200 (200 cSt) et Belsil PDM 20 (20cSt) de Wacker. Les valeurs entre parenthèses représentent les viscosités à 25°C.

Dans ce troisième mode de réalisation, la phase huileuse peut comprendre, en plus de l'huile siliconée phénylée, une ou plusieurs autres huiles additionnelles, cosmétiquement acceptables, qui peuvent être polaires ou apolaires, volatiles ou non volatiles, siliconées ou hydrocarbonées.
Parmi les huiles additionnelles susceptibles d'être employées, on peut citer, seule ou en mélange, les huiles d'origine animale, végétale, minérale ou synthétique, polaires ou apolaires, telles que ci-dessus mentionnées.
On peut également citer, parmi les huiles additionnelles, les huiles siliconées volatiles telles que :

- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE , qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane, de l'hexylheptaméthyltrisiloxane ou de l'octylheptaméthyltrisiloxane.

On peut également utiliser des huiles siliconées non volatiles telles que :

- les polyalkyl($C_1$-$C_{20}$) siloxanes et notamment ceux à groupements terminaux triméthylsilyle, de préférence ceux dont la viscosité est inférieure à 0,06 $m^2/s$; on peut notamment citer les polydiméthylsiloxanes linéaires ou cycliques,

éventuellement comportant un groupement alkyl alcoxy, pendant ou en bout de chaîne; les alkylméthylpolysiloxanes tels que la cétyldiméthicone (nom CTFA),

- les silicones modifiées par des groupements aliphatiques et/ou aromatiques, éventuellement fluorés, ou par des groupements fonctionnels tels que des groupements hydroxyles, thiols et/ou amines.

Dans ce 3$^{ème}$ mode de réalisation particulier, la composition comprend de préférence la ou les huile(s) siliconée(s) phénylée(s) en une quantité comprise entre 1-99% en poids, notamment 5-90% en poids, de préférence 7-50% en poids, voire 13-40% en poids, par rapport au poids total de la composition cosmétique.
La ou les huile(s) additionnelle(s) sont alors présentes en une quantité de préférence comprise entre 0,1-50% en poids, notamment 4-30% en poids, de préférence 10-25% en poids, par rapport au poids total de la composition cosmétique.

Quel que soit le mode de réalisation, la phase huileuse est de préférence comprise en une quantité de 1 à 99% en poids, notamment 10 à 90% en poids, de préférence 15 à 80% en poids, par rapport au poids total de la composition cosmétique.

La composition selon l'invention peut comprendre en outre d'autres corps gras que les huiles ci-dessus, qui peuvent être choisis par l'homme du métier sur base de ses connaissances générales, de manière à conférer à la composition finale les propriétés souhaitées, par exemple en consistance et/ou en texture.
Ces corps gras additionnels peuvent être des cires, des gommes et/ou des corps gras pâteux d'origine animale, végétale, minérale ou synthétique, ainsi que leurs mélanges.
Une cire, au sens de la présente invention, est un composé gras lipophile, solide à température ambiante (25°C), à changement d'état solide/liquide réversible, ayant une température de fusion supérieure à 40°C et pouvant aller jusqu'à 200°C, généralement une dureté supérieure à 0,5 MPa et présentant à l'état solide une organisation cristalline anisotrope.
On peut notamment citer les cires d'origine animale, végétale, minérale ou synthétique telles que les cires microcristallines, la cire de paraffine, le pétrolatum, la vaseline, l'ozokérite, la cire de montan; la cire d'abeilles, la cire de lanoline et ses dérivés; les cires de Candellila, d'Ouricury, de Carnauba, du Japon, le beurre de cacao, les cires de fibres de lièges ou de canne à sucre, la cire de lignite, la cire de son de riz, la cire de sapin, la cire de coton; les huiles hydrogénées ayant une température de fusion supérieure à 40°C (environ), comme l'huile de jojoba hydrogénée; les esters gras et les glycérides concrets à 25°C; les cires de polyéthylène et les cires obtenues par synthèse de Fischer-Tropsch; des huiles hydrogénées concrètes à 25°C; des lanolines.
Lorsque la phase huileuse comprend des huiles de silicone, on peut également employer des cires de silicone telles que les alkyl- bu les alcoxydiméthicones ayant une chaîne alkyle ou alcoxy comportant 10-45 atomes de carbone; les esters de polysiloxane, notamment de polydiméthylsiloxane, solides à 30°C dont la chaîne ester comporte 10-45 atomes de carbone, et/ou les résines de silicone.
Les corps gras pâteux ont généralement un point de fusion compris entre 25 et 60°C, de préférence entre 30 et 45°C, et/ou une dureté allant de 0,001 et 0,5 MPa, de préférence entre 0,005 et 0,4 MPa. On peut notamment citer les lanolines et leurs dérivés, ou les esters de cholestérol.
Ces corps gras additionnels peuvent être présents en une quantité de 0,1-50% en poids, notamment 3-40% en poids, encore mieux 5-30% en poids, par rapport au poids total de la composition.

La composition selon l'invention peut être anhydre, ou peut également comprendre une phase aqueuse, qui peut comprendre de l'eau, une eau florale telle que l'eau de bleuet et/ou une eau minérale telle que l'eau de VITTEL, l'eau de LUCAS ou l'eau de LA ROCHE POSAY.
Ladite phase aqueuse peut comprendre de 0,1 à 14% en poids, par rapport au poids total de la phase aqueuse, d'un monoalcool en $C_2$-$C_6$ comme l'éthanol, le propanol, le butanol, l'isopropanol et l'isobutanol.

La composition selon l'invention peut éventuellement comprendre un tensioactif, notamment lorsqu'elle se présente sous forme d'émulsion, de préférence en une quantité de 0,01 à 30% en poids par rapport au poids total de la composition.
On peut citer, seuls ou en mélange, les sels alcalins, les sels d'ammonium, les sels d'amines ou les sels d'aminoalcools des composés suivants : les alcoylsulfates, alcoyléther sulfates, alcoylamides sulfates et éthers sulfates, alcoylarylpolyéthersulfates, monoglycérides sulfates, alcoylsulfonates, alcoylamides sulfonates, alcoylarylsulfonates, $\alpha$-oléfines sulfonates, paraffines sulfonates, alcoylsulfosuccinates, alcoyléthersulfosuccinates, alcoylamides sulfosuccinates, alcoylsulfosuccinamates, alcoylsulfoacétates, alcoylpolyglycérol carboxylates, alcoylphosphates/alcoylétherphosphates, acylsarcosinates, alcoylpolypeptidates, alcoylamidopolypeptidates, acyliséthionates, alcoyllaurates. Le radical alcoyle ou acyle dans tous ces composés désigne généralement une chaîne de 12 à 18 atomes de carbone.
On peut aussi citer les savons et les sels d'acides gras tels que les acides oléique, ricinoléique, palmitique, stéarique, les acides d'huile de coprah ou d'huile de coprah hydrogénée et notamment les sels d'amines tels que les stéarates d'amines; les acyl lactylates dont le radical acyle comprend 8-20 atomes de carbone; les acides carboxyliques d'éthers polyglycoliques.
On peut encore citer les alcools, les alcoylphénols et acides gras polyéthoxylés, polypropoxylés ou polyglycérolés à chaîne grasse comportant 8 à 18 atomes de carbone; des copolymères d'oxydes d'éthylène et de propylène, des

condensats d'oxyde d'éthylène et de propylène sur des alcools gras, des amides gras polyéthoxylés, des amines grasses polyéthoxylées, des éthanolamides, des esters d'acides gras de glycol, des esters d'acides gras (stéarate, oléate) du sorbitan oxyéthylénés ou non, des esters d'acides gras du saccharose, des esters d'acides gras de polyéthylèneglycol (monostéarate ou monolaurate de polyéthylène glycol); des triesters phosphoriques, des esters d'acides gras de dérivés de glucose; les alkylpolyglycosides et les alkylamides des sucres aminés; les produits de condensation d'un $\alpha$-diol, d'un monoalcool, d'un alcoylphénol, d'un amide ou d'un diglycolamide avec le glycidol ou un précurseur de glycidol.
On peut également citer le trioleyl phosphate; les ester d'acides gras de polyols comme les mono-, di-, tri- ou sesqui-oléates ou stéarates de sorbitol ou de glycérol, les laurates de glycérol ou de polyéthylène glycol; les alkyl ou alkoxy diméthicones copolyols à chaîne alkyle ou alkoxy pendante ou en bout de squelette siliconé ayant par exemple de 6 à 22 atomes de carbone; les alkyl (lauryl, cétyl, stéaryl, octyl) éthers polyoxyéthylénés et les diméthicones copolyols.
La composition selon l'invention peut encore comprendre un ou plusieurs agents épaississants, par exemple dans des concentrations préférentielles de 0,01 à 6% en poids par rapport au poids total de la composition. L'agent épaississant peut être choisi parmi, seul ou en mélange :

- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose et la carboxyméthylcellulose, les dérivés de l'amidon, les dérivés d'éthers de cellulose possédant des groupes ammonium quaternaires, les polysaccharides cationiques;
- les polymères synthétiques comme les acides polyacryliques tels que les polymères poly(méth)acrylates de glycéryl tels que l'HISPAGEL ou le LUBRAGEL des sociétés HISPANO QUIMICA ou GARDIAN, la polyvinylpyrrolidone, l'alcool polyvinylique, les polymères réticulés d'acrylamide et d'acrylate d'ammonium tels que le PAS 5161 ou BOZEPOL C de HOECHST; les copolymères acrylate/octylacrylamide tels que le Dermacryl de National Starch; les polymères à base de polyacrylamide tels que le SEPIGEL 305 de SEPPIC, les polymères réticulés d'acrylamide et de chlorure de méthacryloyloxyéthyltriméthylammonium tels que le SALCARE SC 92 de ALLIED COLLOIDS,
- le silicate de magnésium et d'aluminium;
- les argiles éventuellement modifiées comme les hectorites modifiées par un chlorure d'ammonium d'acide gras en $C_{10}$ à $C_{22}$, comme l'hectorite modifiée par du chlorure de di-stéaryl di-méthyl ammonium ;
- la silice éventuellement modifiée;
- les galactomannanes comportant un à six et mieux de deux à quatre groupes hydroxyle par ose, substitués par une chaîne alkyle saturée ou non, comme la gomme de guar alkylée par des chaînes alkyle en $C_1$ à $C_6$ et mieux en $C_1$ à $C_3$ et plus particulièrement la guar éthylée ayant un degré de substitution de 2 à 3 telle que celle vendue par la société Aqualon sous le nom N-HANCE-AG ;
- les dérivés de cellulose tel que l'éthylcellulose.
- les copolymères séquencés, notamment de type "dibloc" ou "tribloc" du type polystyrène/polyisoprène, polystyrène/polybutadiène, polystyrène/copoly(éthylène-butylène) ou encore polystyrène/copoly(éthylène-propylène) tels que ceux vendus sous le nom de 'Kraton' par Shell Chemical;
- les polymères de type polyamide, par exemple comportant un squelette polymérique ayant des motifs répétitifs amide, et éventuellement au moins une chaîne grasse pendante et/ou au moins une chaîne terminale éventuellement fonctionnalisées, ayant de 8 à 120 atomes de carbone et étant liées à ces motifs amide, parmi lesquels on peut citer les produits commerciaux vendus par la société Arizona Chemical sous les noms Uniclear 80 et Uniclear 100 qui sont un mélange de copolymères d'un diacide en $C_{36}$ condensé sur l'éthylène diamine, de masse moléculaire moyenne en poids d'environ 6000, les groupes ester terminaux résultant de l'estérification des terminaisons d'acide restantes par l'alcool cétylique, stéarylique ou leurs mélanges (appelés aussi alcool cétylstéarylique).

Selon l'application envisagée, la composition peut comprendre en outre un polymère filmogène. Ceci est généralement le cas lorsque l'on souhaite préparer une composition de type vernis à ongles, mascara, eye-liner ou laque à lèvres.
Les polymères peuvent être dissous ou dispersés dans le milieu cosmétiquement acceptable. Les polymères peuvent être présents à une teneur allant de 0,01 % à 40 % en poids par rapport au poids total de la composition.
La composition peut également comprendre un plastifiant choisi parmi les plastifiants usuels et qui peut être présent à une teneur allant de 0,1 à 40% en poids par rapport au poids total de la composition.
La composition peut comprendre en outre une phase particulaire, qui peut comprendre des pigments et/ou des nacres et/ou des charges habituellement utilisés dans les compositions cosmétiques.
Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière, notamment produites par certains mollusques dans leur coquille ou bien synthétisées.
Les pigments peuvent être présents dans la composition à raison de 0,01 à 25% en poids de la composition finale, et

de préférence à raison de 3 à 10% en poids. Ils peuvent être blancs ou colorés, minéraux ou organiques, de taille usuelle ou nanométrique. Ils peuvent se présenter sous forme de poudre ou de pâte pigmentaire. On peut citer les oxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent
ou d'aluminium. On peut encore citer les pigments D&C et les laques couramment employées pour conférer aux lèvres et à la peau un effet de maquillage, qui sont des sels de calcium, de baryum, d'aluminium, de strontium ou de zirconium.
Les nacres peuvent être présentes dans la composition à raison de 0,01 à 20% en poids, de préférence à un taux de l'ordre de 3 à 10% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Les charges, qui peuvent être présentes à raison de 0,01 à 60% en poids, de préférence 3 à 10%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères de polymère telles que l'Expancel (Nobel Industrie), le polytrap (Dow Corning) et les microbilles de résine de silicone (Tospearls de Toshiba, par exemple), le carbonate de calcium précipité, le carbonate ou l'hydrocarbonate de magnésium, des savons métalliques dérivés d'acides organiques carboxyliques ayant de 8 à 22 atomes de carbone.

La composition peut en outre comprendre un colorant, notamment un colorant organique naturel tel que le carmin de cochenille, et/ou un colorant de synthèse tel que les colorants halogéno-acides, azoïques, anthraquinoniques. On peut également citer des colorants minéraux tels que le sulfate de cuivre ou de fer. On peut encore citer le brun Soudan, le rouge Soudan et le rocou, ainsi que le jus de betterave, le carotène et le bleu de méthylène. Le colorant peut être présent dans la composition, seul ou en mélange, à raison de 0,001 à 15% en poids, de préférence 0,01 à 5% en poids et notamment de 0,1 à 2% en poids, par rapport au poids total de la composition.

La composition peut en outre comprendre un composé photochrome autre que les colorants organiques photochromes de formule (I) ou (II). On peut notamment citer les composés photochromes minéraux, et plus particulièrement les aluminosilicates dopés tels que la sodalite dopée par des halogènes, ou les oxydes ou hydrates métalliques tels que les oxydes de titane rendu photochrome à l'aide d'un métal choisi parmi le fer, le chrome, le cuivre, le nickel, le manganèse, le cobalt, le molybdène, tel quel ou sous forme de sel tel qu'un sulfate, un chlorate, un nitrate, un acétate. Le composé photochrome peut être incorporé dans la composition en une quantité de 0,001-20% poids par rapport au poids total de la composition, de préférence en une quantité de 0,1-10% en poids.

La composition peut en outre comprendre un filtre UV qui peut être incorporé dans la composition en une quantité de 0,01-20% en poids par rapport au poids total de la composition, de préférence en une quantité de 0,1-10% en poids. On peut notamment citer, parmi les filtres solaires susceptibles d'être employés, les composés appartenant aux familles des para-aminobenzoïques; des salicylates; des dibenzoylméthanes; des cinnamates; des dérivés de β, β'-diphénylacrylate; des benzophénones; des benzylidène camphres; des phenyl benzimidazoles; des triazines; des phenyl benzotriazoles; des anthraniliques; des imidazolines et/ou des benzalmalonates.

La composition peut comprendre en outre tout additif usuellement utilisé dans le domaine cosmétique, tel que des antioxydants, des parfums, des huiles essentielles, des conservateurs, des actifs cosmétiques lipophiles ou hydrophiles, des hydratants, des vitamines, des sphingolipides, des agents autobronzants tels que la DHA, des azurants optiques, des agents antimousses, des agents séquestrants.

Bien entendu l'homme du métier veillera à choisir les éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention est avantageusement photochrome, c'est-à-dire qu'elle présente un ΔE supérieur ou égal à 5, de préférence supérieur ou égal à 10, mieux supérieur ou égal à 25, encore mieux supérieur ou égal 35, voire supérieur ou égal à 45; le ΔE est mesuré de la manière définie avant les exemples.

La composition cosmétique selon l'invention trouve notamment une application particulièrement intéressante dans le domaine du maquillage de la peau, des semi-muqueuses, des muqueuses et/ou des phanères (ongles, cils, sourcils, poils et cheveux).

Elle peut comprendre ou se présenter sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; une poudre libre, compacte ou coulée; une pâte anhydre. Cette composition peut avoir l'aspect d'une lotion, d'une crème, d'une pommade, d'une pâte souple, d'un onguent, d'un solide coulé ou moulé et notamment en stick ou en coupelle, ou encore de solide compacté.

La composition cosmétique selon l'invention peut se présenter sous la forme d'un produit de soin et/ou de maquillage de la peau du corps ou du visage, des lèvres et des cheveux, d'un produit solaire ou autobronzant, voire d'un produit capillaire. Elle trouve une application particulière dans le domaine des rouges à lèvres, des fonds de teint, des fards à joues ou à paupières, des poudres libres ou compactes, des crèmes teintées, des produits de maquillage

du corps, des produits de coloration de la peau, des eye-liners et des mascaras.
L'invention a également pour objet un procédé de traitement cosmétique d'un support choisi parmi les muqueuses, les semi-muqueuses, la peau et/ou les phanères, dans lequel on applique sur ledit support une composition cosmétique telle que définie ci-dessus.
Un autre objet de l'invention est un procédé de maquillage et/ou de coloration temporaire d'un support choisi parmi les muqueuses, les semi-muqueuses, la peau et/ou les phanères, dans lequel on applique sur ledit support une composition cosmétique telle que définie ci-dessus.
L'invention est illustrée plus en détail dans les exemples suivants.

**Mesure du ΔE**

Les mesures de L, a et b ont été effectuées sur carte de contraste de marque LENETA et de référence FORM 1A PENOPAC, pour un étalement de composition de 50 microns d'épaisseur.
Les mesures en réflexion ont été effectuées grâce à un spectrocolorimètre MINOLTA 3700D.
On a ainsi déterminé les coordonnées trichromatiques initiales de la composition avant irradiation ($L_0$, $a_0$ et $b_0$). On a ensuite soumis la composition à une irradiation aux UV (flux de 2 mW/cm$^2$ d'un rayonnement UVA) pendant deux minutes puis aussitôt après l'arrêt de l'irradiation, déterminé les coordonnées trichromatiques après irradiation (L, a et b). Il s'est écoulé moins de 5 secondes entre l'arrêt de l'irradiation et la détermination des nouvelles coordonnées.
On a alors calculé le ΔE de la manière suivante :

$$\Delta E = [ (L - L_0)^2 + (a - a_0)^2 + (b - b_0)^2 ]^{1/2}$$

Lorsque l'on mesure le ΔE d'un colorant et non d'une composition le comprenant, on effectue la mesure sur une solution comprenant 0,05% en poids de colorant dans du malate de diisostéaryle. Puis on effectue la mesure comme indiqué ci-dessus.

**Exemple 1**

On a étudié la solubilité d'un colorant selon l'invention dans différentes huiles de silicone.
On a donc préparé plusieurs mélanges comprenant 0,05% en poids de 3-phényl-3-(4-pypéridinophényl)-6-morpholino-3H-naphto[2,1-b]pyran (Reversacol Ruby de James Robinson) dans différentes huiles, puis chauffé le mélange sous agitation à 60°C.
On a observé à l'oeil nu le mélange du colorant avec l'huile, à chaud (60°C) puis après retour à 25°C du mélange chauffé.
On obtient les résultats suivants :

| Huile | A chaud (60°C) | Après retour à 25°C |
|---|---|---|
| Phényltriméthylsiloxy trisiloxane (viscosité: 20 cSt) DC556 de Dow Corning | solubilisation totale | solubilisation quasi totale : quelques grains restent non solubilisés |
| Polyphényltriméthylsiloxy diméthylsiloxane (viscosité: 1000 cSt) BELSIL PDM 1000 de Wacker | solubilisation totale | solubilisation quasi totale : quelques grains restent non solubilisés |
| Cyclopentadiméthylsiloxane (viscosité : 4cSt) | dépôt de poudre au fond; presque pas de solubilisation; mélange trouble (suspension de particules de colorant non solubilisées) | dépôt du colorant non solubilisé (poudre) au fond; solution incolore trouble avec des particules en suspension |
| PDMS (viscosité 5 cSt) DC200 fluid de Dow Corning | dépôt de poudre au fond; presque pas de solubilisation; mélange trouble (suspension de particules de colorant non solubilisées) | dépôt du colorant non solubilisé (poudre) au fond; solution incolore trouble avec des particules en suspension |

(suite)

| Huile | A chaud (60°C) | Après retour à 25°C |
|---|---|---|
| Décaméthyltétrasiloxane (viscosité 1,5 cSt) | dépôt de poudre au fond; presque pas de solubilisation; mélange très légèrement trouble (suspension de particules de colorant non solubilisées) | dépôt du colorant non solubilisé (poudre) au fond; solution incolore transparente avec des particules en suspension |

On constate donc que le colorant photochrome se solubilise mal à chaud dans les huiles non phénylées, bien qu'elles soient de viscosité faible, plus propice à une bonne solubilisation qu'une viscosité plus élevée.
Par contre, la solubilisation est totale à chaud dans les huiles phénylées, quelle que soit leur viscosité.

Après retour à température ambiante, les observations à chaud sont confirmées. Les silicones phénylées permettent une solubilisation quasi totale du colorant photochrome après retour à température ambiante. La solubilisation est là encore bien meilleure que dans les autres milieux siliconés, dans lesquels le colorant n'est pratiquement pas solubilisé (dépôt de poudre au fond du flacon).

Par ailleurs, on a observé une coloration rose de la solution du colorant dans les silicones phénylées, et non pour les autres huiles; ceci montre qu'il existe également pour ces huiles un effet solvatochrome.

**Exemple 2**

On a préparé une solution comprenant 0,05% en poids de 3-phényl-3-(4-pypéridinophényl)-6-morpholino-3H-naphto[2,1-b]pyran (Reversacol Ruby de James Robinson) dans une huile de silicone phénylée (DC556 de Dow Corning). Puis on a irradié ledit mélange aux UV (soleil) pendant 2 minutes.

On a observé visuellement le passage très net d'une coloration rose initiale du mélange avant irradiation, à une coloration rouge orangé soutenu, après irradiation. Ceci confirme bien l'existence de l'effet photochrome.

**Exemple 3**

On a préparé une composition comprenant :

- 0,05% en poids de 3-phényl-3-(4-pypéridinophényl)-6-morpholino-3H-naphto[2,1-b]pyran (Reversacol Ruby de James Robinson),
- 15 % en poids de cire de polyéthylène (cire apolaire),
- qsp 100% d'huile de silicone phénylée (DC556 de Dow Corning).

Les mesures de L, a et b ont été effectuées sur carte de contraste de marque LENETA et de référence FORM 1A PENOPAC, pour un étalement de composition de 50 microns d'épaisseur.
Les mesures en réflexion ont été effectuées grâce à un spectrocolorimètre MINOLTA 3700D.
On a ainsi déterminé les coordonnées trichromatiques initiales de la composition avant irradiation ($L_0$, $a_0$ et $b_0$). On a ensuite soumis la composition à une irradiation aux UV (flux de 2 $mW/cm^2$ d'un rayonnement UVA) pendant deux minutes puis aussitôt après l'arrêt de l'irradiation, déterminé les coordonnées trichromatiques après irradiation (L, a et b). Il s'est écoulé moins de 5 secondes entre l'arrêt de l'irradiation et la détermination des nouvelles coordonnées.
On a alors calculé le ΔE de la manière suivante :

$$\Delta E = [ (L - L_0)^2 + (a - a_0)^2 + (b - b_0)^2 ]^{1/2}$$

On obtient le résultat suivant : ΔE = 37,38

On constate donc que l'effet photochrome existe toujours dans un système structuré, c'est-à-dire en présence d'une cire.

**Exemple 4**

On a préparé un stick pour les lèvres comprenant (% en poids):

| | |
|---|---|
| - Reversacol Ruby | 0,05% |

(suite)

| | |
|---|---|
| - cire de polyéthylène | 15% |
| - huile de silicone phénylée (DC556) | 30% |
| - huile de parléam qsp | 100% |

**Exemple 5**

On a déterminé le ΔE (avant et après irradiation UV pendant 2 minutes) de différents colorants photochromes selon l'invention, à 1% en poids dans une base blanche de rouge à lèvres comprenant :

| | |
|---|---|
| - octyl-2 dodecanol | 0,5% |
| - hectorite modifiée par chlorure de di-stearyl di-methyl ammonium | 0,6% |
| - lanoline liquide | 27,2% |
| - cire microcristalline | 10,5% |
| - cire d'abeille polyglycérolée (3 moles) | 4,2% |
| - lanoline acétylée | 6,7% |
| - huile d'arara (esters d'acide oléique) | 13,5% |
| - cire de lanoline oxypropylénée (5 op) | 6,7% |
| - érucate d'oléyle | 13,5% |
| - triglycérides d'acides oleique-linoleique-linolenique | 1,7% |
| - triglycérides d'acides palmitique-oleique-linoleique | 13,5% |
| - hyaluronate de sodium | 0,1% |
| - conservateurs | 0,1% |
| - vitamine | 0,5% |
| - filtre UV | 0,7% |

On prépare donc une composition comprenant donc 1 % de colorant selon l'invention et 99% de la base ci-dessus.

Les mesures de L, a et b ont été effectuées sur carte de contraste de marque LENETA et de référence FORM 1A PENOPAC, pour un étalement de composition de 50 microns d'épaisseur.

Les mesures en réflexion ont été effectuées grâce à un spectrocolorimètre MINOLTA 3700D.

On a ainsi déterminé les coordonnées trichromatiques initiales de la composition avant irradiation ($L_0$, $a_0$ et $b_0$). On a ensuite soumis les compositions à une irradiation aux UV (flux de 2 mW/cm$^2$ d'un rayonnement UVA) pendant deux minutes puis aussitôt après l'arrêt de l'irradiation, déterminé les coordonnées trichromatiques après irradiation (L, a et b). Il s'est écoulé moins de 5 secondes entre l'arrêt de l'irradiation et la détermination des nouvelles coordonnées.

On a alors calculé le ΔE de la manière suivante :

$$\Delta E = [\,(L - L_0)^2 + (a - a_0)^2 + (b - b_0)^2\,]^{1/2}$$

On obtient les résultats suivants :

| Colorant | ΔE |
|---|---|
| 3,3-di(4-methoxyphenyl)-6-morpholino-3H-naphto[2,1-b]pyran | 51 |
| 3-phényl-3-(4-morpholinophényl)-6-morpholino-3H-naphto[2,1-b]pyran | 53 |
| 3-phényl-3-(4-piperidinophényl)-6-morpholino-3H-naphto[2,1-b]pyran | 49 |

Une mesure de ΔE supérieure à 30 environ signifie que le colorant donne une couleur vive; on constate que cela est particulièrement le cas avec les colorants selon l'invention testés ici.

**Exemple 6**

Afin de mettre en évidence le solvatochromisme des colorants selon l'invention, c'est dire l'influence de la nature de l'huile sur l'expression colorielle desdits colorants photochromes, sans exposition aux UV, on a préparé plusieurs mélanges simplex comprenant 0,05% en poids de 3-phényl-3-(4-pypéridinophényl)-6-morpholino-3H-naphto [2,1-b]pyran (Reversacol Ruby de James Robinson) dans différentes huiles.

On a ainsi déterminé les coordonnées trichromatiques ($L_h$, $a_h$ et $b_h$) de l'huile seule et du mélange huile + colorant ($L_m$, $a_m$ et $b_m$) et calculé le ΔE' de la manière suivante:

$$\Delta E' = [ (L_h - L_m)^2 + (a_h - a_m)^2 + (b_h - b_m)^2 ]^{1/2}$$

Pour ce faire, on a employé un spectrocolorimètre Minolta 3700D en mode transmission, avec une cuve en plastique (trajet optique "parois cuve + huile + éventuellement colorant " = 1 cm).

On obtient les résultats suivants :

| Huile | $\delta_a$ de l'huile seule $(J/cm^3)^{1/2}$ | ΔE' du mélange huile + colorant |
|---|---|---|
| huile de ricin | 9,09 | 33,6278 |
| Octyldodécanol | 7,69 | 37,6049 |
| Diisostéarylmalate | 7,19 | 12,9041 |
| triglycéride des acides caprique/caprylique | 6,69 | 12,4093 |
| Triisononanoïn | 6,54 | 12,8122 |
| Trimellilate de tridécyle | 5,35 | 14,2178 |
| Isononanoate d'isononyle | 4,87 | 7,7386 |
| Polyisobutène hydrogéné | 0 | 6,6920 |
| Isododécane | 0 | 4,2421 |

On constate donc que l'effet solvatochrome, à savoir la modification de la couleur par l'huile, est d'autant plus important que l'huile est polaire.

**Exemple 7**

Les mélanges préparés dans l'exemple 6 ont été irradiés aux UV (flux de 2 $mW/cm^2$ d'UVA) pendant 2 minutes. On a ensuite à nouveau déterminé, comme à l'exemple 2, les coordonnées trichromatiques du mélange, après irradiation ($L_{m'}$, $a_{m'}$ et $b_{m'}$) et calculé le ΔE de la manière suivante :

$$\Delta E = [ (L_{m'} - L_m)^2 + (a_{m'} - a_m)^2 + (b_{m'} - b_m)^2 ]^{1/2}$$

Il s'est écoulé moins de 5 secondes entre l'arrêt de l'irradiation et la détermination des nouvelles coordonnées.

On obtient les résultats suivants :

| Huile | ΔE |
|---|---|
| huile de ricin | 19,02 |
| octyldodécanol | 20,88 |
| diisostéarylmalate | 38,79 |
| triglycéride des acides caprique/caprylique | 23,70 |
| Triisononanoïn | 21,62 |
| Trimellilate de tridécyle | 13,74 |

(suite)

| Huile | ΔE |
|---|---|
| Isononanoate d'isononyle | 25,47 |
| Polyisobutène hydrogéné | 17,69 |
| Isododécane | 19,17 |

On constate donc bien que l'effet photochrome existe bien pour le colorant solubilisé dans différents huiles.

**Exemple 8**

On a ajouté aux mélanges préparés à l'exemple 6 (huiles + 0,05% en poids de colorant Reversacol Ruby) 15 % en poids de cire de polyéthylène (cire apolaire), puis on a mesuré, avant et après irradiation 2 minutes aux UV (flux de 2 $mW/cm^2$ d'UVA), les coordonnées trichromatiques L,a,b sur des films minces de composition, d'épaisseur 50 microns, coulés sur carte de contraste.

Pour ce faire, on a employé un spectrocolorimètre Minolta 3700D en mode réflexion; il s'est écoulé moins de 5 secondes entre l'arrêt de l'irradiation et la détermination des nouvelles coordonnées.

Comme précédemment, on a calculé le ΔE et on obtient les résultats suivants :

| Nature de l'huile dans la composition (15% de cire + 0,05% de colorant) | ΔE |
|---|---|
| huile de ricin | 26,75 |
| octyldodécanol | 31,09 |
| diisostéarylmalate | 43,12 |
| Triisononanoïn | 33,70 |
| Trimellilate de tridécyle | 31,98 |
| Isononanoate d'isononyle | 27,74 |
| Polyisobutène hydrogéné | 18,36 |
| Isododécane | 27,21 |

On constate donc que l'effet photochrome existe toujours dans un système structuré, c'est-à-dire en présence d'une cire.

**Exemple 9**

On a préparé un gloss apolaire pour les lèvres comprenant (% en poids):

| | |
|---|---|
| - Reversacol Ruby | 0,05% |
| - polybutylène ($\delta_a$ = 0) | 99,95% |

On obtient un gel de couleur initiale saumon très léger, qui devient saumon soutenu après exposition quelques secondes au soleil. L'effet photochrome est net, d'autant plus que la composition de départ est peu colorée et transparente. La rapidité de l'effet à l'excitation aux UV comme à la relaxation est remarquable.

**Exemple 10**

On a préparé un gloss polaire pour les lèvres comprenant (% en poids):

| | |
|---|---|
| - Reversacol Ruby | 0,2% |
| - octyl-2 dodécanol | 10% |
| - silice pyrogénée | 6% |
| - huile de ricin | qsp 100% |

$\delta_a$ de la phase huileuse > 7 $(J/cm^3)^{½}$

On obtient un gel semi-épais de couleur initiale grenat foncé. L'effet solvatochrome est très visible.

**Exemple 11**

On a préparé un stick pour les lèvres comprenant (% en poids):

| | |
|---|---|
| - Reversacol Ruby | 0,2% |
| - cire de polyéthylène | 15% |
| - polyisobutène hydrogéné ($\delta_a$ = 0) | qsp 100% |

On obtient un stick de couleur initiale blanc très légèrement orangé qui devient orangé foncé très soutenu après exposition quelques secondes au soleil. Le retour à la couleur initiale est également rapide.

**Exemple 12**

On a préparé un stick pour les lèvres comprenant (% en poids):

| | |
|---|---|
| - Reversacol Ruby | 0,05% |
| - octyl-2 dodécanol | 10% |
| - huile de lanoline fluide | 10% |
| - cire de polyéthylène | 15% |
| - huile de ricin | qsp 100% |

-
-
-
-
-

On obtient un stick de couleur initiale rose clair qui devient orangé foncé très soutenu après exposition quelques secondes au soleil.

On constate un effet photochrome très important avec une qualité importante de retour à l'état non excité en terme de rapidité et de recouvrance de l'absence de couleur (le retour se fait comme l'excitation en quelques secondes).

**Revendications**

1. Composition cosmétique comprenant, dans un milieu cosmétiquement acceptable, au moins une phase huileuse et au moins un colorant organique photochrome de formule (I) ou (II) :

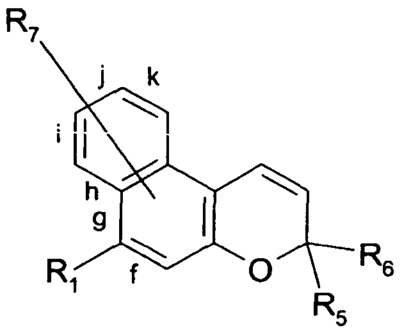

(I)

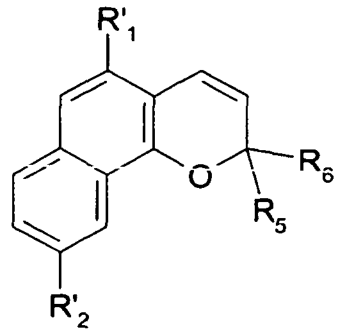

(IIa)

dans lesquelles :

* R1 représente :

- (i) un groupement hydrocarboné ayant 1 à 30, de préférence 1-18, et mieux 1 à 12, voire 1-6, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl;
- (ii) un cycle hydrocarboné formé avec l'une des liaisons "f" ou "gh" et le radical R7; ou
- (iii) un groupement choisi parmi -COOR4, -C(O)NR2R3, -NR2R3, -OR4 et -SR4, dans lequel :

  - R2 et R3 soit représentent, indépendamment l'un de l'autre, un groupement hydrocarboné ayant 1 à 20, de préférence 1-12, et mieux 1 à 6, atomes de carbone, linéaire, ramifié ou cyclique, saturé ou insaturé, éventuellement comportant 1 à 5 hétéroatomes choisis parmi N, O, S, Si et P,
  soit pris ensemble avec l'atome d'azote auquel ils sont reliés, forment un hétérocycle hydrocarboné, saturé ou insaturé, comportant 3-10, de préférence 4-6, atomes de carbone et éventuellement 1-5 autres hétéroatomes choisis parmi N, O, S, Si et P, ledit cycle étant éventuellement substitué par au moins un radical hydrocarboné ayant 1 à 20, de préférence 1-15, et mieux 1 à 6, atomes de carbone, linéaire, ramifié ou cyclique, saturé ou insaturé, comportant éventuellement 1 à 5 hétéroatomes choisis parmi N, O, S, Si et P;
  - R4 représente un groupement hydrocarboné ayant 1 à 20, de préférence 1-15, et mieux 1 à 6, atomes de carbone, linéaire, ramifié ou cyclique, saturé ou insaturé, éventuellement halogéné ou perhalogéné, notamment par Fl, Br et/ou Cl, et/ou éventuellement comportant 1 à 5 hétéroatomes choisis parmi N, O, S, Si et P;

* R5 et R6 représentent, indépendamment l'un de l'autre, un groupement choisi parmi :

- (i) les groupements aminoaryles cycliques saturés de formule (IIa) ou (IIb) :

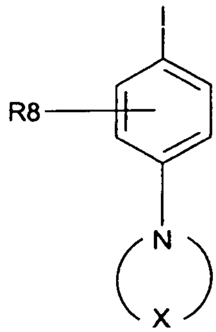

(IIa)

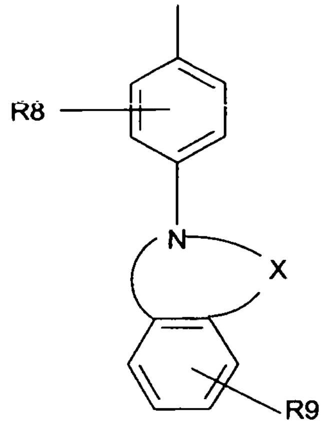

(IIb)

dans lesquelles le cycle comportant N et X est un cycle saturé qui comprend au total 3 à 30 atomes, de préférence 4-10 et encore mieux 5, 6 ou 7 atomes, inclus l'azote, le reste étant des atomes de carbone et/ou des hétéroatomes choisis parmi O, S, Si, P et/ou des groupements choisis parmi -NH et -NR avec R représentant un radical hydrocarboné ayant 1 à 20, de préférence 1-15, et mieux 1 à 6, atomes de carbone, linéaire, ramifié ou cyclique, saturé ou insaturé, éventuellement comportant 1 à 5 hétéroatomes choisis parmi N, O, S, Si et P;

- (ii) les groupements indolinoaryles de formule (III) :

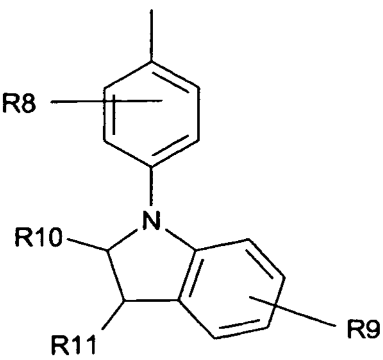

(III)

dans laquelle R10 et R11 représentent, indépendamment l'un de l'autre, un groupement choisi parmi (i) les groupements hydrocarbonés ayant 1 à 30, de préférence 1-18, et mieux 1 à 12, voire 1-6, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl; (ii) les atomes d'halogène, et notamment Fl, Br et/ou Cl; (iii) les groupements -CN (nitrile), -COOH (carboxylate), $-NO_2$ (nitro); (iv) un atome d'hydrogène; (v) un groupement choisi parmi -C(O)NR2R3, -NR2R3, - OR4 ou -SR4 avec R2, R3 et R4 ayant les significations données ci-dessus; (vi) les radicaux R10 et R11 pouvant former ensemble un cycle hydrocarboné saturé ou insaturé ayant au total 5 à 8 atomes (incluant les atomes du cycle indoline), lesdits atomes étant choisis parmi C, O, S et/ou NR avec R représentant H ou un radical hydrocarboné ayant 1 à 20 atomes de carbone, linéaire, ramifié ou cyclique, saturé ou insaturé, éventuellement comportant 1 à 5 hétéroatomes choisis parmi N, O, S, Si et P,

- (iii) les groupements de formule (IV) :

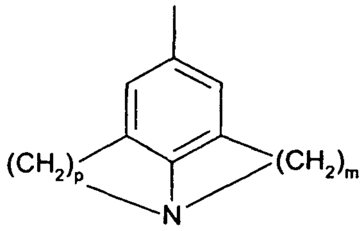

(IV)

dans laquelle m et p sont, indépendamment l'un de l'autre, des entiers allant de 2 à 5;

- (iv) les groupements aminoaryles cycliques insaturés de formules (Va), (Vb) ou (Vc) :

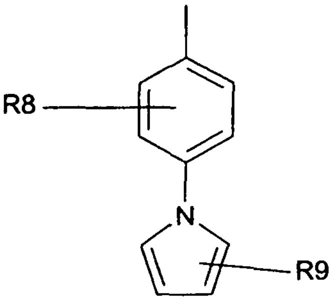

(Va)

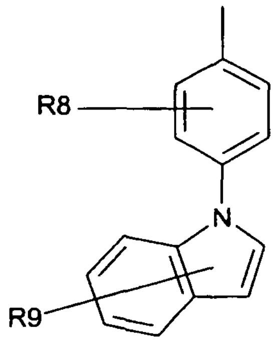

(Vb)

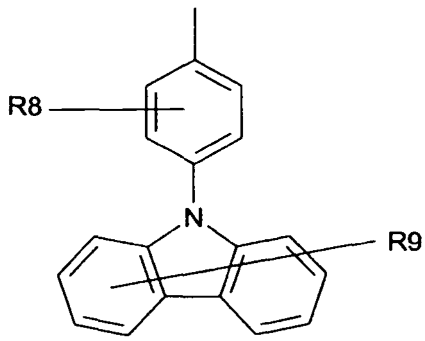

(Vc)

dans lesquelles R8 et R9, représentent, indépendamment l'un de l'autre, un groupement choisi parmi (i) les groupements hydrocarbonés ayant 1 à 30, de préférence 1-18, et mieux 1 à 12, voire 1-6, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl; (ii) les atomes d'halogène, et notamment Fl, Br et/ou Cl; (iii) les groupements -CN (nitrile), -COOH (carboxylate), $-NO_2$ (nitro); (iv) un atome d'hydrogène; (v) un groupement choisi parmi -C(O)NR2R3, -NR2R3, - OR4 ou -SR4 avec R2, R3 et R4 ayant les significations données ci-dessus;

- (v) un groupement hydrocarboné ayant 1 à 30, de préférence 2-18, et mieux 3 à 12, atomes de carbone, linéaire, ramifié ou cyclique, saturé ou insaturé, éventuellement comportant 1 à 5 hétéroatomes choisis parmi N, O, S, Si et P; et notamment un groupement choisi parmi $-C_6H_4$-CONR2R3, $-C_6H_4$-NR2R3 et -

$C_6H_4$-OR4 avec R2, R3 et R4 ayant les significations données ci-dessus;

* R7 représente un groupement choisi parmi :

- (i) les groupements hydrocarbonés ayant 1 à 30, de préférence 1-18, et mieux 1 à 12, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl;
- (ii) les atomes d'halogène, et notamment Fl, Br et/ou Cl;
- (iii) les groupements -CN (nitrile), -COOH (carboxylate), $-NO_2$ (nitro); -N=N-(azo); =NH (imino); $-CONH_2$ (amide);
- (iv) un atome d'hydrogène;
- (v) un groupement choisi parmi -C(O)NR2R3, -NR2R3, -OR4 ou -SR4 avec R2, R3 et R4 ayant les significations données ci-dessus;
- (vi) le radical R7 pouvant en outre former, avec l'une des liaisons "i", "j", "k", ou "g,h" prises avec le radical R1, ou "f" prise avec le radical R1, un cycle hydrocarboné saturé ayant au total 3 à 8, de préférence 4 à 7, et mieux 5 ou 6, atomes de carbone, éventuellement comportant 1 à 5 hétéroatomes choisis parmi N, O, S, Si et P;

* R'1 représente un groupement choisi parmi :

- (i) un atome d'hydrogène;
- (ii) un groupement hydrocarboné ayant 1 à 30, de préférence 1-18, et mieux 1 à 12, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl;
- (iii) un groupement choisi parmi -C(O)NR2R3, -NR2R3, -OR4 et -SR4, avec R2, R3 et R4 ayant les significations données ci-dessus;

* R'2 représente un groupement choisi parmi :

- (i) les groupements hydrocarbonés ayant 1 à 30, de préférence 1-18, et mieux 1 à 12, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl;
- (ii) les atomes d'halogène, et notamment Fl, Br et/ou Cl;
- (iii) les groupements -CN (nitrile), -COOH (carboxylate), $-NO_2$ (nitro); -N=N-(azo); =NH (imino); $-CONH_2$ (amide);
- (iv) un atome d'hydrogène;
- (v) un groupement choisi parmi -C(O)NR2R3, -NR2R3, -OR4 ou -SR4 avec R2, R3 et R4 ayant les significations données ci-dessus,

ledit colorant organique étant soluble dans la phase huileuse de la composition.

**2.** Composition selon la revendication 1 dans laquelle le colorant organique photochrome répond à l'une des formules (Ia) et (IIa) suivantes :

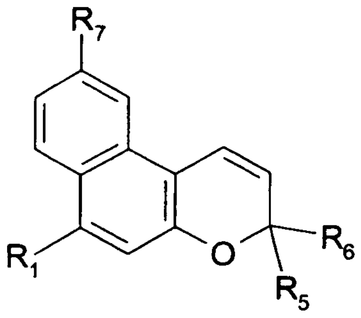

(Ia)

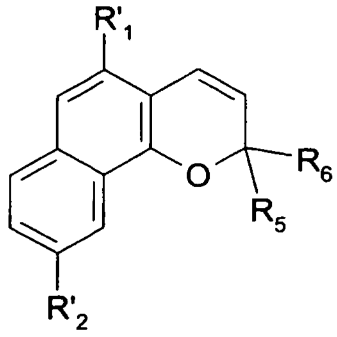

(IIa)

dans lesquelles R1, R5, R6, R7, R'1 et R'2 sont définis comme dans la revendication 1.

**3.** Composition selon l'une des revendications précédentes, dans laquelle R1 représente un cycle hydrocarboné avec l'une des liaisons "f" ou "gh" et le radical R7; ou un groupement choisi parmi -COOR4, -NR2R3, -OR4 et -SR4, dans lequel :

- R2 et R3 soit représentent, indépendamment l'un de l'autre, un groupement hydrocarboné ayant 1 à 20, de préférence 1-12, et mieux 1 à 6, atomes de carbone, linéaire, ramifié ou cyclique, saturé ou insaturé, éventuellement comportant 1 à 5 hétéroatomes choisis parmi N, O, S, Si et P,
soit pris ensemble avec l'atome d'azote auquel ils sont reliés, forment un hétérocycle hydrocarboné, saturé ou insaturé, comportant 3-10, de préférence 4-6, atomes de carbone et éventuellement 1-5 autres hétéroatomes choisis parmi N, O, S, Si et P, ledit cycle étant éventuellement substitué par au moins un radical hydrocarboné ayant 1 à 20, de préférence 1-15, et mieux 1 à 6, atomes de carbone, linéaire, ramifié ou cyclique, saturé ou insaturé, comportant éventuellement 1 à 5 hétéroatomes choisis parmi N, O, S, Si et P;
- R4 représente un groupement hydrocarboné ayant 1 à 20, de préférence 1-15, et mieux 1 à 6, atomes de carbone, linéaire, ramifié ou cyclique, saturé ou insaturé, éventuellement halogéné ou perhalogéné, notamment par Fl, Br et/ou Cl, et/ou éventuellement comportant 1 à 5 hétéroatomes choisis parmi N, O, S, Si et P.

**4.** Composition selon l'une des revendications précédentes, dans laquelle R5 et R6 représentent, indépendamment l'un de l'autre, un groupement choisi parmi :

- les groupements aminoaryles cycliques saturés de formule (IIa) ou (IIb) :

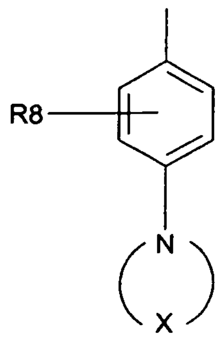

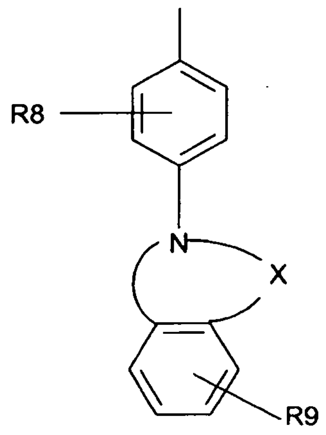

(IIa) (IIb)

dans lesquelles le cycle comportant N et X est un cycle saturé qui comprend au total 3 à 30 atomes, de préférence 4-10 et encore mieux 5, 6 ou 7 atomes, inclus l'azote, le reste étant des atomes de carbone et/ou des hétéroatomes choisis parmi O, S, Si, P et/ou des groupements choisis parmi -NH et -NR avec R représentant un radical hydrocarboné ayant 1 à 20, de préférence 1-15, et mieux 1 à 6, atomes de carbone, linéaire, ramifié ou cyclique, saturé ou insaturé, éventuellement comportant 1 à 5 hétéroatomes choisis parmi N, O, S, Si et P;
- un groupement hydrocarboné ayant 1 à 30, de préférence 2-18, et mieux 3 à 12, atomes de carbone, linéaire, ramifié ou cyclique, saturé ou insaturé, éventuellement comportant 1 à 5 hétéroatomes choisis parmi N, O, S, Si et P; et notamment un groupement choisi parmi -$C_6H_4$-CONR2R3, -$C_6H_4$-NR2R3 et -$C_6H_4$-OR4 avec R2, R3 et R4 ayant les significations données en revendication 1.

**5.** Composition selon l'une des revendications précédentes, dans laquelle R7 représente un groupement choisi parmi :

- (i) les groupements hydrocarbonés ayant 1 à 30, de préférence 1-18, et mieux 1 à 12, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl;
- (ii) les atomes d'halogène, et notamment Fl, Br et/ou Cl;
- (iii) les groupements -CN (nitrile), -COOH (carboxylate), -$NO_2$ (nitro); -N=N-(azo); =NH (imino); -$CONH_2$ (amide);
- (iv) un atome d'hydrogène;
- (v) un groupement choisi parmi -NR2R3, -OR4 ou -SR4 avec R2, R3 et R4 ayant les significations données en revendication 1;
- (vi) le radical R7 pouvant en outre former, avec l'une des liaisons "i", "j", "k", ou "g,h" prises avec le radical R1, ou "f" prise avec le radical R1, un cycle hydrocarboné saturé ayant au total 3 à 8, de préférence 4 à 7, et mieux 5 ou 6, atomes de carbone, éventuellement comportant 1 à 5 hétéroatomes choisis parmi N, O, S, Si et P.

**6.** Composition selon l'une des revendications précédentes, dans laquelle R'1 représente l'hydrogène ou un groupement hydrocarboné ayant 1 à 30, de préférence 1-18, et mieux 1 à 12, atomes de carbone, linéaires ramifiés ou cycliques, saturés ou insaturés, éventuellement comportant 1 à 5 hétéroatomes choisis parmi N, O, S, Si et P, et/ou éventuellement halogéné ou perhalogéné, notamment par Fl, Br et/ou Cl.

**7.** Composition selon l'une des revendications précédentes, dans laquelle R'2 représente l'hydrogène ou un groupement choisi parmi -$NO_2$, -NR2R3 et -C(O)NR2R3, dans lesquels R2 et R3, soit représentent, indépendamment l'un de l'autre, un groupement hydrocarboné ayant 1 à 20, de préférence 1-12, et mieux 1 à 6, atomes de carbone, linéaire, ramifié ou cyclique, saturé ou insaturé, éventuellement comportant 1 à 5 hétéroatomes choisis parmi N, O, S, Si et P; soit pris ensemble avec l'atome d'azote auquel ils sont reliés, forment un hétérocycle hydrocarboné, saturé ou insaturé, comportant 3-10, de préférence 4-6, atomes de carbone et éventuellement 1-5 autres hétéroatomes choisis parmi N, O, S, Si et P, ledit cycle étant éventuellement substitué par au moins un radical hydrocarboné ayant 1 à 20, de préférence 1-15, et mieux 1 à 6, atomes de carbone, linéaire, ramifié ou cyclique, saturé ou insaturé,

comportant éventuellement 1 à 5 hétéroatomes choisis parmi N, O, S, Si et P.

**8.** Composition selon l'une des revendications précédentes, dans laquelle le colorant organique est de formule (I) ou (Ia) pour lesquelles :

* R1 représente un groupement -COOR avec R étant un radical hydrocarboné saturé ayant 1 à 12, de préférence 1-6, atomes de carbone, et notamment un radical méthyle ou éthyle; ou un groupement

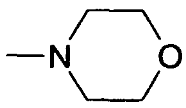

et/ou
* R5 et R6 représentent, indépendamment l'un de l'autre, soit (i) un groupement de formule (IIa) :

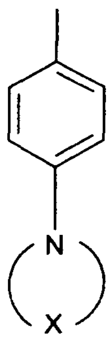

(IIa)

dans laquelle le cycle comportant N et X est un cycle saturé qui comprend au total 4 à 7 atomes, notamment 5 ou 6 atomes, inclus l'azote, et notamment 3-5 atomes de carbone et 0-1 atome d'oxygène; et en particulier un groupement de formule:

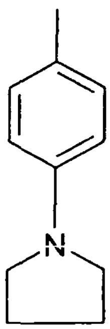 ou 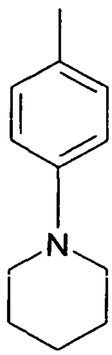 ou 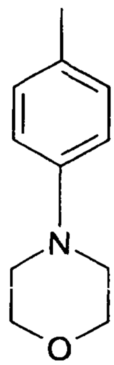

soit (ii) un groupement hydrocarboné ayant 5 à 14, de préférence 6 à 10 atomes de carbone, linéaire, ramifié ou cyclique, saturé ou insaturé, éventuellement comportant 1 à 2 hétéroatomes choisis parmi N, O ou S;
en particulier un groupement

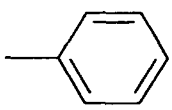 ou 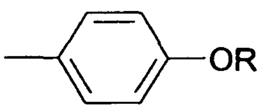 ou 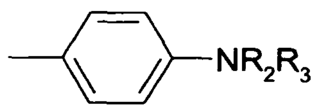

dans lesquelles R est un radical hydrocarboné saturé ayant 1 à 12, de préférence 1-6, atomes de carbone, et notamment un radical méthyle ou éthyle; et R2 et R3 sont, indépendamment l'un de l'autre, un groupement hydrocarboné ayant 1 à 20, de préférence 1-15, et mieux 1 à 6, atomes de carbone, linéaire, ramifié ou cyclique, saturé ou insaturé, éventuellement comportant 1 à 5 hétéroatomes choisis parmi N, O, S, Si et P;
et/ou

* R7 représente un atome d'hydrogène ou un groupement -NR2R3, avec R2 et R3 représentant, indépendamment l'un de l'autre, un groupement hydrocarboné saturé, linéaire ou ramifié, ayant 1 à 12, de préférence 1-6, atomes de carbone, et notamment un groupement méthyle et/ou éthyle.

**9.** Composition selon l'une des revendications 1 à 7, dans laquelle le colorant organique est de formule (IIa) pour laquelle :

* R'1 représente l'hydrogène ou un groupement -COOR avec R étant un radical hydrocarboné saturé ayant 1 à 12, de préférence 1-6, atomes de carbone, et notamment un radical méthyle ou éthyle;
et/ou
* R5 et R6 représentent, indépendamment l'un de l'autre, soit (i) un groupement de formule (IIa) :

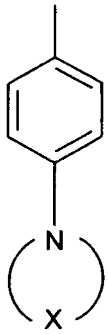

(IIa)

dans laquelle le cycle comportant N et X est un cycle saturé qui comprend au total 4 à 7 atomes, notamment 5 à 6 atomes, inclus l'azote, et notamment 4-5 atomes de carbone et 0-1 atome d'oxygène; et en particulier un groupement de formule:

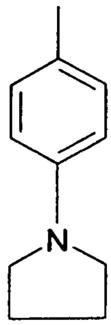

ou

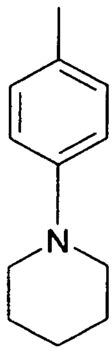

ou

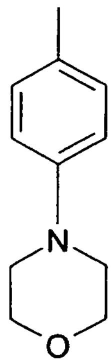

soit (ii) un groupement hydrocarboné ayant 5 à 14, de préférence 6 à 10 atomes de carbone, linéaire, ramifié ou cyclique, saturé ou insaturé, éventuellement comportant 1 à 2 hétéroatomes choisis parmi N, O ou S;
en particulier un groupement :

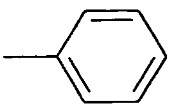

ou

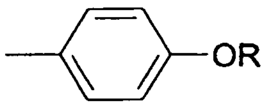

ou

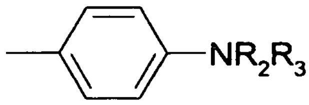

dans lesquelles R est un radical hydrocarboné saturé ayant 1 à 12, de préférence 1-6, atomes de carbone, et notamment un radical méthyle ou éthyle; et R2 et R3 sont, indépendamment l'un de l'autre, un groupement hydrocarboné ayant 1 à 20, de préférence 1-15, et mieux 1 à 6, atomes de carbone, linéaire, ramifié ou cyclique, saturé ou insaturé, éventuellement comportant 1 à 5 hétéroatomes choisis parmi N, O, S, Si et P;
et/ou
* R'2 représente l'hydrogène, ou un groupement -NR'R" avec R' et R", identiques ou différents, représentant un groupement hydrocarboné saturé, linéaire ou ramifié, ayant 1 à 12, de préférence 1-6, atomes de carbone, et notamment un groupement méthyle et/ou éthyle; ou un groupement

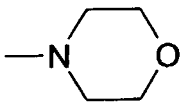

**10.** Composition selon l'une des revendications précédentes, dans laquelle le colorant organique est choisi parmi :

- le 3,3-di(4-méthoxyphényl)-6-morpholino-3H-naphto[2,1-b]pyran de formule :

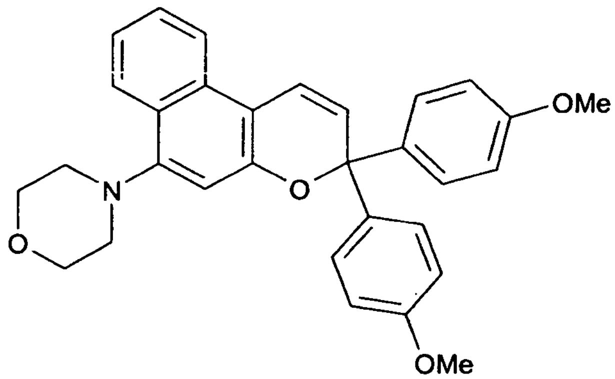

- le 3-phényl-3-(4-morpholinophényl)-6-morpholino-3H-naphto[2,1-b]pyran de formule :

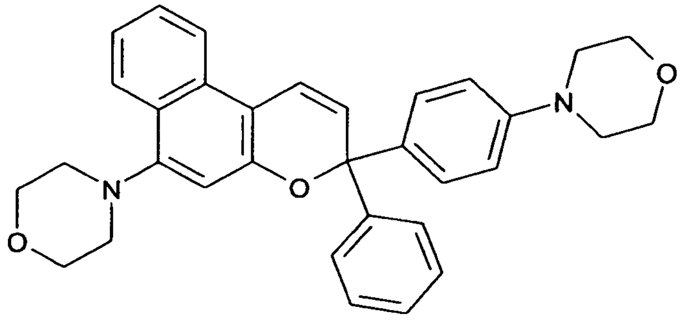

- le 3-phényl-3-(4-pipéridinophényl)-6-morpholino-3H-naphto[2,1-b]pyran de formule:

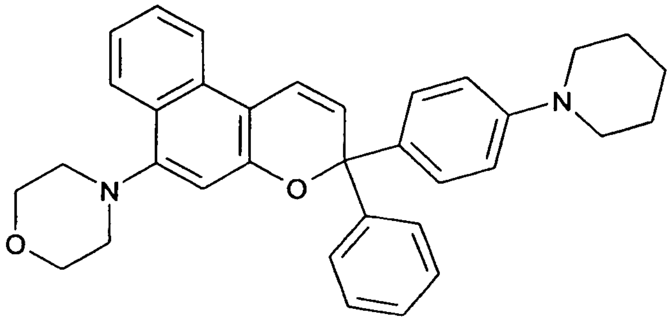

- le 3-phényl-3-(4-pipéridinophényl)-6-méthoxycarbonyl-9-N-diméthyl-3H-naphto[2,1-b]pyran de formule :

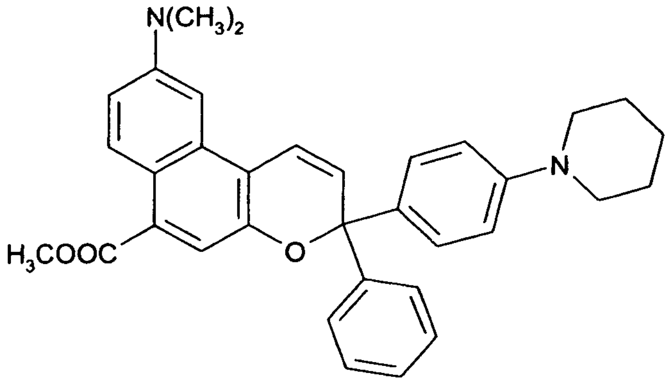

- le 2-phényl-2-(4-piperidinophényl)-5-méthoxycarbonyl-9-N-diméthyl-2H-naphto[1,2-b]pyran de formule :

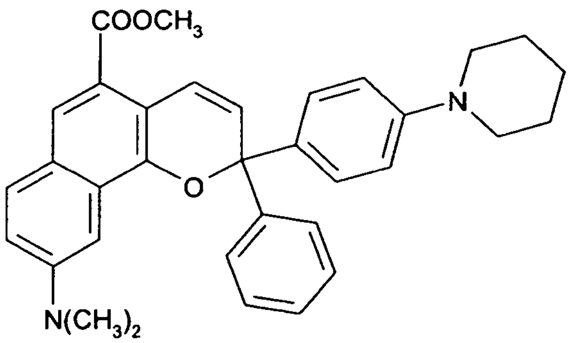

- leurs mélanges.

**11.** Composition selon l'une des revendications précédentes, dans laquelle le colorant organique selon l'invention est compris, seul ou en mélange, en une quantité de 0,001 à 20% en poids, notamment de 0,005 à 10 % en poids, de préférence 0,01 à 4,8% en poids, et encore mieux de 0,05 à 3% en poids, préférentiellement de 0,1 à 2% en poids, par rapport au poids total de la composition cosmétique.

**12.** Composition selon l'une des revendications précédentes, dans laquelle la phase huileuse est polaire et présente un paramètre moyen de solubilité $\delta_a$ selon l'espace de solubilité de Hansen, à 25°C, supérieur ou égal à 5,0 $(J/cm^3)^{½}$, notamment supérieur ou égal à 5,3, voire supérieur ou égal à 5,5 et encore mieux supérieur ou égal à 6,0 $(J/cm^3)^{½}$, voire supérieur ou égale à 7,0 $(J/cm^3)^{½}$.

**13.** Composition selon l'une des revendications précédentes dans laquelle la phase huileuse comprend 5 à 100% en poids, notamment 10-90%, voire 15-60% et encore mieux 20-50% en poids du poids total de ladite phase huileuse, d'une ou plusieurs huiles polaires ayant un paramètre moyen de solubilité $\delta_a$ selon l'espace de solubilité de Hansen, à 25°C, supérieur ou égal à 5,0 $(J/cm^3)^{½}$.

**14.** Composition selon l'une des revendications 1 à 11, dans laquelle la phase huileuse est apolaire et présente un paramètre moyen de solubilité $\delta_a$ selon l'espace de solubilité de Hansen, à 25°C, inférieur à 5,0, notamment inférieur ou égal à 4,9, encore mieux inférieur ou égal à 4,5 et encore mieux inférieur ou égal à 4,0 $(J/cm^3)^{½}$.

**15.** Composition selon l'une des revendications 1-11 et 14, dans laquelle la phase huileuse comprend 5 à 100% en poids, notamment 10-90%, voire 15-60% et encore mieux 20-50% en poids du poids total de ladite phase huileuse, d'une ou plusieurs huiles apolaires ayant un paramètre moyen de solubilité $\delta_a$ selon l'espace de solubilité de Hansen, à 25°C, inférieur à 5,0 $(J/cm^3)^{½}$.

**16.** Composition selon l'une des revendications précédentes, dans laquelle la phase huileuse comprend au moins une huile choisie parmi :

- les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule $R_1COOR_2$ dans laquelle $R_1$ représente le reste d'un acide gras supérieur comportant de 7 à 19 atomes de carbone et $R_2$ représente une chaîne hydrocarbonée ramifiée contenant de 3 à 20 atomes de carbone, par exemple, l'huile de Purcellin; l'huile de paraffine, de vaseline, de calophyllum, de macadamia, de colza, de coprah, d'arachide, de palme, de ricin, de jojoba, d'olive ou de germes de céréales; l'huile de beurre de karité; le perhydrosqualène;
- les esters et les éthers de synthèse notamment d'acides gras comme les huiles de formule $R_1COOR_2$ dans laquelle $R_1$ représente le reste d'un acide gras supérieur comportant de 7 à 29 atomes de carbone et $R_2$ représente une chaîne hydrocarbonée contenant de 3 à 30 atomes de carbone comme par exemple le myristate d'isopropyle, le palmitate d'éthyl-2hexyle, le stéarate d'octyl-2-dodécyle, l'érucate d'octyl-2-dodécyle, l'isononanoate d'isononyle, l'isostéarate d'isostéaryle; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol; les esters du pentaérythritol comme le tétraisostéarate de pentaérythrithyle ; des esters du type trimellitate de tridécyle;
- des alcools gras ayant de 12 à 26 atomes de carbone comme l'octyldodécanol, le 2-butyloctanol, le 2-hexyldécanol, le 2-undécylpentadécanol, l'alcool oléique.
- des hydrocarbures linéaires ou ramifiés, d'origine minérale ou synthétique tels que les huiles de paraffine et leurs dérivés, la vaseline, les polydécènes, le polyisobutène hydrogéné tel que le parléam; les isoparaffines comme l'isohexadécane et l'isodécane ;
- des glycérides et notamment des acétylglycérides ou des triglycérides d'acides gras ayant 4 à 10 atomes de carbone, comme les triglycérides des acides heptanoïque, octanoïque et des acides caprique/caprylique.

**17.** Composition selon l'une des revendications précédentes, dans laquelle la phase huileuse comprend au moins une huile choisie parmi l'octyldodécanol, l'hexyldécanol, l'octyldécanol, l'alcool oléique, l'huile de ricin, le malate de diisostéaryle, le triheptanoate de glycéryle, le trioctanoate de glycéryle, le triglycéride des acides caprique/caprylique, le triisononanoïn, le trimellitate de tridécyle, les hydrocarbures aliphatiques notamment en C6-C40 comme les huiles de paraffine, volatiles, telles que l'isohexadecane ou l'isododécane, ou non volatiles, et leurs dérivés; la vaseline, les polydécènes hydrogénés ou non, le polyisobutène hydrogéné tel que l'huile de Parléam, le squalane, les polybutylènes, l'isononanoate d'isononyle; les huiles fluorées notamment perfluorées, et leurs mélanges.

**18.** Composition selon l'une des revendications 1-11, dans laquelle la phase huileuse comprend au moins une huile de silicone phénylée de formule (VI), ou un mélange de telles huiles :

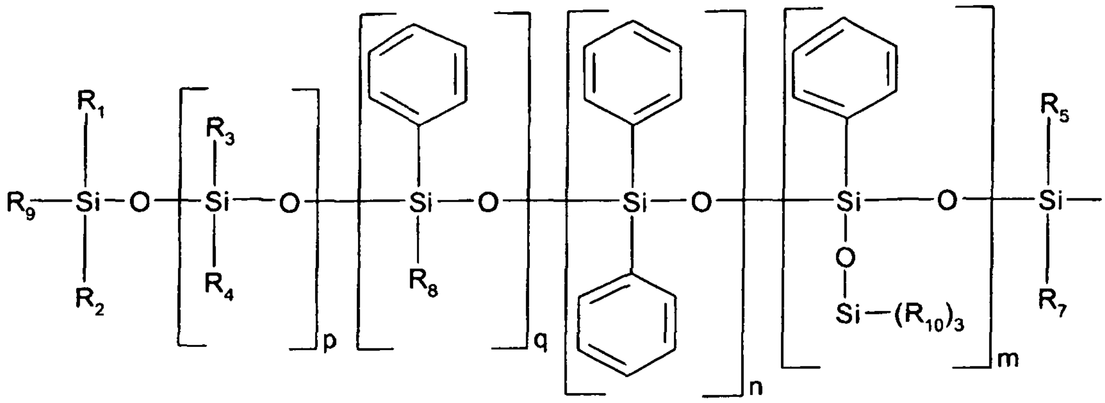

(VI)

dans laquelle

- R1 à R10, indépendamment les uns des autres, sont des radicaux hydrocarbonés, saturés ou insaturés, linéaires, cycliques ou ramifiés, en C1-C30,
- m, n, p et q sont, indépendamment les uns des autres, des nombres entiers compris entre 0 et 900, sous réserve que la somme 'm+n+q' est différente de 0.

**19.** Composition selon la revendication 18, dans laquelle dans l'huile de siliconée phénylée de formule (VI), la somme 'm+n+q' est comprise entre 1 et 100 et/ou la somme 'm+n+p+q' est comprise entre 1 et 900, encore mieux entre 1 et 800.

**20.** Composition selon l'une des revendications 18-19, dans laquelle l'huile de silicone phénylée est de formule (VII) :

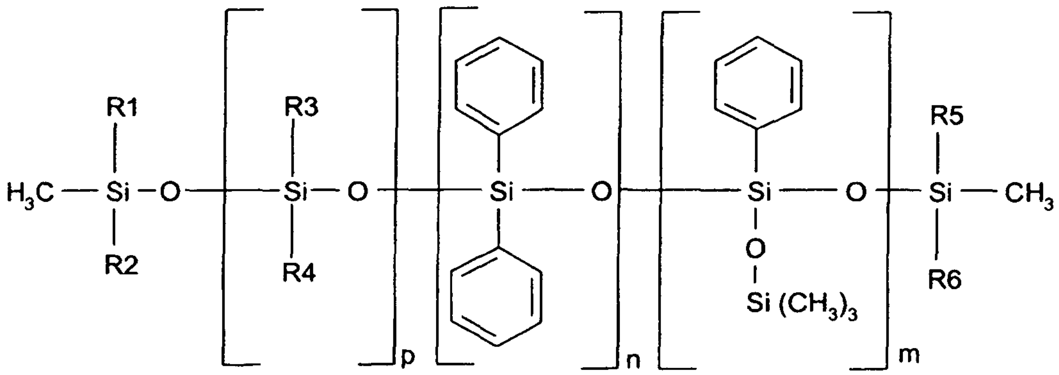

(VII)

dans laquelle :

- R1 à R6, indépendamment les uns des autres, sont des radicaux hydrocarbonés, saturés ou insaturés, linéaires cycliques ou ramifiés, en C1-C30,
- m, n et p sont, indépendamment les uns des autres, des nombres entiers compris entre 0 et 100, sous réserve que la somme 'n + m' est comprise entre 1 et 100.

**21.** Composition selon l'une des revendications 18 à 20, dans laquelle, dans les formules (VI) et (VII), R1 à R6, indépendamment les uns des autres, représentent un radical hydrocarboné saturé, linéaire ou ramifié, en C1-C30, notamment en C1-C12, et en particulier un radical méthyle, éthyle, propyle ou butyle.

**22.** Composition selon l'une des revendications 18 à 21, dans laquelle, dans les formules (VI) et (VII), R1 à R6 sont identiques et sont un radical méthyle.

**23.** Composition selon l'une des revendications 18 à 22, dans laquelle l'huile de silicone phénylée est choisie parmi les phényl triméthicones, les phényl diméthicones, les phényl triméthylsiloxy diphénylsiloxanes, les diphényl diméthicones, les diphényl méthyldiphényl trisiloxanes, et leurs mélanges.

**24.** Composition selon l'une des revendications 18 à 23, dans laquelle l'huile siliconée phénylée est présente en une quantité comprise entre 1-99% en poids, notamment 5-90% en poids, de préférence 7-50% en poids, voire 12-40% en poids, par rapport au poids total de la composition cosmétique.

**25.** Composition selon l'une des revendications 18 à 24, dans laquelle la phase huileuse de la composition comprend une ou plusieurs autres huiles additionnelles, cosmétiquement acceptables, polaires ou apolaires, volatiles ou non volatiles, siliconées ou hydrocarbonées, qui peut être présente en une quantité comprise entre 0,1-50% en poids, notamment 4-30% en poids, de préférence 10-25% en poids, par rapport au poids total de la composition cosmétique.

**26.** Composition selon l'une des revendications précédentes, dans laquelle la phase huileuse est comprise en une quantité de 1 à 99% en poids, notamment 10 à 90% en poids, de préférence 15 à 80% en poids, par rapport au poids total de la composition cosmétique.

**27.** Composition selon l'une des revendications précédentes, comprenant en outre d'autres corps gras choisis parmi les cires, les gommes et/ou les corps gras pâteux d'origine animale, végétale, minérale ou synthétique, ainsi que leurs mélanges; et/ou une phase particulaire, qui peut comprendre des pigments, des nacres, des charges, des colorants et/ou d'autres composés photochromes; et/ou des filtres UV; et/ou une phase aqueuse; et/ou un tensioactif et/ou un agent épaississant et/ou un polymère filmogène.

**28.** Composition selon l'une des revendications précédentes, présentant un $\Delta E$ supérieur ou égal à 5, de préférence supérieur ou égal à 10, mieux supérieur ou égal à 25, encore mieux supérieur ou égal 35, voire supérieur ou égal à 45.

**29.** Composition selon l'une des revendications précédentes, se présentant sous la forme d'un produit de soin et/ou de maquillage de la peau du corps ou du visage, des lèvres et des cheveux, d'un produit solaire ou autobronzant, d'un produit capillaire.

**30.** Composition selon l'une des revendications précédentes, se présentant sous la forme d'un rouge à lèvres, d'un fond de teint, d'un fard à joues ou à paupières, d'une poudre libre ou compacte, d'une crème teintée, d'un produit de maquillage du corps, d'un produit de coloration de la peau, d'un eye-liner ou d'un mascara.

**31.** Procédé de traitement cosmétique d'un support choisi parmi les muqueuses, les semi-muqueuses, la peau et/ou les phanères, dans lequel on applique sur ledit support une composition cosmétique telle que définie dans l'une des revendications 1 à 30.

**32.** Procédé de maquillage et/ou de coloration temporaire d'un support choisi parmi les muqueuses, les semi-muqueuses, la peau et/ou les phanères, dans lequel on applique sur ledit support une composition cosmétique telle que définie dans l'une des revendications 1 à 30.

**Claims**

**1.** Cosmetic composition comprising, in a cosmetically acceptable medium, at least one oily phase and at least one photochromic organic dye of formula (I) or (II):

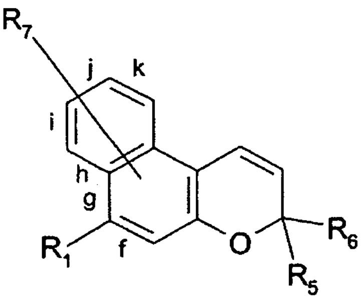

(I)

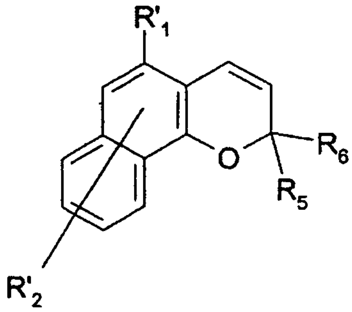

(II)

in which:

* R1 represents:

- (i) a linear, branched or cyclic, saturated or unsaturated hydrocarbon-based group containing from 1 to 30, preferably 1-18 and better still 1 to 12, or even 1 to 6, carbon atoms, optionally comprising 1 to 5 heteroatoms chosen from N, O, S, Si and P, and/or optionally halogenated or perhalogenated, especially with Fl, Br and/or Cl;
- (ii) a hydrocarbon-based ring formed with one of the bonds "f" or "gh" and the radical R7; or
- (iii) a group chosen from -COOR4, -C(O)NR2R3, -NR2R3, -OR4 and -SR4, in which:

- R2 and R3 either represent, independently of each other, a linear, branched or cyclic, saturated or unsaturated hydrocarbon-based group containing 1 to 20, preferably 1-12 and better still 1 to 6 carbon atoms, optionally comprising 1 to 5 heteroatoms chosen from N, O, S, Si and P,
or, taken together with the nitrogen atom to which they are attached, form a saturated or unsaturated hydrocarbon-based heterocycle containing 3-10 and preferably 4-6 carbon atoms and optionally 1-5 other heteroatoms chosen from N, O, S, Si and P, the said ring optionally being substituted with at least one linear, branched or cyclic, saturated or unsaturated hydrocarbon-based radical containing 1 to 20, preferably 1-15 and better still 1 to 6 carbon atoms, optionally comprising 1 to 5 heteroatoms chosen from N, O, S, Si and P;
- R4 represents a linear, branched or cyclic, saturated or unsaturated hydrocarbon-based group containing 1 to 20, preferably 1-15 and better still 1 to 6 carbon atoms, which is optionally halogenated or perhalogenated especially with Fl, Br and/or Cl, and/or optionally comprising 1 to 5 heteroatoms chosen from N, O, S, Si and P;

* R5 and R6 represent, independently of each other, a group chosen from:

- (i) the saturated cyclic aminoaryl groups of formula (IIa) or (IIb):

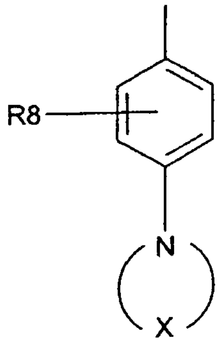 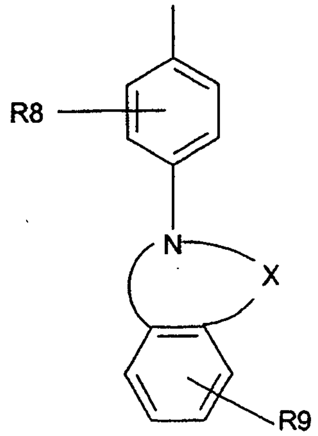

(IIa) (IIb)

in which the ring comprising N and X is a saturated ring containing in total 3 to 30 atoms, preferably 4-10 and better still 5, 6 or 7 atoms, including nitrogen, the remainder being carbon atoms and/or heteroatoms chosen from O, S, Si and P and/or groups chosen from -NH and -NR with R representing a linear, branched or cyclic, saturated or unsaturated hydrocarbon-based radical containing 1 to 20, preferably 1-15 and better still 1 to 6 carbon atoms, optionally comprising 1 to 5 heteroatoms chosen from N, O, S, Si and P;

- (ii) the indolinoaryl groups of formula (III):

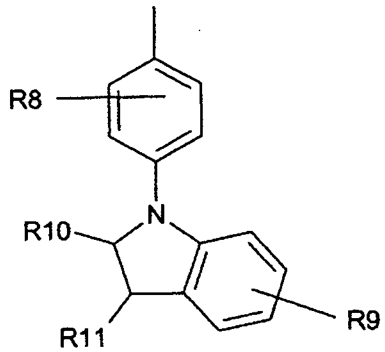

(III)

in which R10 and R11 represent, independently of each other, a group chosen from (i) linear, branched or cyclic, saturated or unsaturated hydrocarbon-based groups containing 1 to 30, preferably 1-18 and better still 1 to 12, or even 1-6, carbon atoms, optionally comprising 1 to 5 heteroatoms chosen from N, O, S, Si and P, and/or optionally halogenated or perhalogenated, especially with Fl, Br and/or Cl; (ii) halogen atoms, and especially Fl, Br and/or Cl; (iii) -CN (nitrile), -COOH (carboxylate) or -$NO_2$ (nitro) groups; (iv) a hydrogen atom; (v) a group chosen from -C(O)NR2R3, -NR2R3, -OR4 and -SR4 with R2, R3 and R4 having the meanings given above; (vi) the radicals R10 and R11 together possibly forming a saturated or unsaturated hydrocarbon-based ring containing in total 5 to 8 atoms (including the atoms of the indoline ring), the said atoms being chosen from C, O, S and/or NR with R representing H or a linear, branched or cyclic, saturated or unsaturated hydrocarbon-based radical containing 1 to 20 carbon atoms, optionally comprising 1 to 5 heteroatoms chosen from N, O, S, Si and P,

- (iii) the groups of formula (IV):

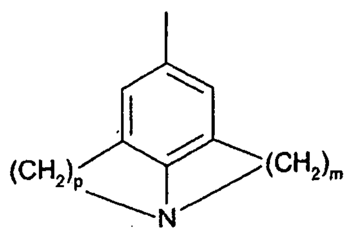

(IV)

in which m and p are, independently of each other, integers ranging from 2 to 5;
- (iv) the unsaturated cyclic aminoaryl groups of formula (Va), (Vb) or (Vc) :

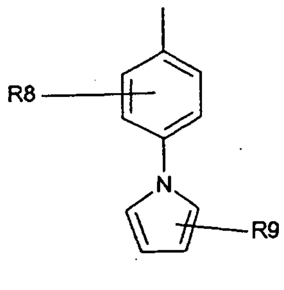

(Va)

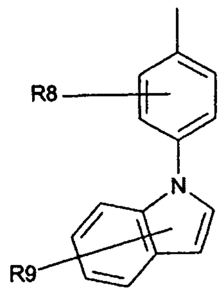

(Vb)

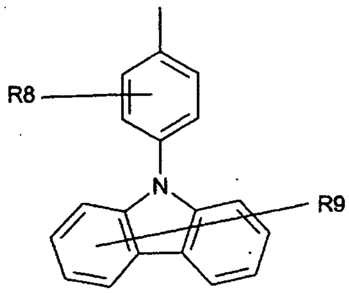

(Vc)

in which R8 and R9 represent, independently of each other, a group chosen from (i) linear, branched or cyclic, saturated or unsaturated hydrocarbon-based groups containing 1 to 30, preferably 1-18 and better still 1 to 12, or even 1-6, carbon atoms, optionally comprising 1 to 5 heteroatoms chosen from N, O, S, Si and P, and/or optionally halogenated or perhalogenated, especially with Fl, Br and/or Cl; (ii) halogen atoms, especially Fl, Br and/or Cl; (iii) -CN (nitrile), -COOH (carboxylate) or $-NO_2$ (nitro) groups; (iv) a hydrogen atom; (v) a group chosen from -C(O)NR2R3, -NR2R3, -OR4 and -SR4 with R2, R3 and R4 having the meanings given above;
- (v) a linear, branched or cyclic, saturated or unsaturated hydrocarbon-based group containing 1 to 30, preferably 2-18 and better still 3 to 12 carbon atoms, optionally comprising 1 to 5 heteroatoms chosen from N, O, S, Si and P; and especially a group chosen from $-C_6H_4$-CONR2R3, $-C_6H_4$-NR2R3 and $-C_6H_4$-OR4 with R2, R3 and R4 having the meanings given above;

* R7 represents a group chosen from:

- (i) linear, branched or cyclic, saturated or unsaturated hydrocarbon-based groups containing 1 to 30,

preferably 1-18 and better still 1 to 12 carbon atoms, optionally comprising 1 to 5 heteroatoms chosen from N, O, S, Si and P, and/or optionally halogenated or perhalogenated, especially with Fl, Br and/or Cl ;
- (ii) halogen atoms, and especially Fl, Br and/or Cl ;
- (iii) -CN (nitrile), -COOH (carboxylate), $-NO_2$ (nitro), -N=N- (azo), =NH (imino) or $-CONH_2$ (amide) groups;
- (iv) a hydrogen atom;
- (v) a group chosen from -C(O)NR2R3, -NR2R3, -OR4 and -SR4 with R2, R3 and R4 having the meanings given above;
- (vi) the radical R7 also possibly forming, with one of the bonds "i", "j", "k" or "g,h" taken with the radical R1, or "f" taken with the radical R1, a saturated hydrocarbon-based ring containing in total 3 to 8, preferably 4 to 7 and better still 5 or 6 carbon atoms, optionally comprising 1 to 5 heteroatoms chosen from N, O, S, Si and P;

* R'1 represents a group chosen from:

- (i) a hydrogen atom;
- (ii) a linear, branched or cyclic, saturated or unsaturated hydrocarbon-based group containing 1 to 30, preferably 1-18 and better still 1 to 12 carbon atoms, optionally comprising 1 to 5 heteroatoms chosen from N, O, S, Si and P, and/or optionally halogenated or perhalogenated, especially with Fl, Br and/or Cl;
- (iii) a group chosen from -C(O)NR2R3, -NR2R3, -OR4 and -SR4, with R2, R3 and R4 having the meanings given above;

* R'2 represents a group chosen from:

- (i) linear, branched or cyclic, saturated or unsaturated hydrocarbon-based groups containing 1 to 30, preferably 1-18 and better still 1 to 12 carbon atoms, optionally comprising 1 to 5 heteroatoms chosen from N, O, S, Si and P, and/or optionally halogenated or perhalogenated, especially with Fl, Br and/or Cl;
- (ii) halogen atoms, especially Fl, Br and/or Cl;
- (iii) -CN (nitrile), -COOH (carboxylate), $-NO_2$ (nitro), -N=N- (azo), =NH (imino) or $-CONH_2$ (amide) groups;
- (iv) a hydrogen atom;
- (v) a group chosen from -C(O)NR2R3, -NR2R3, -OR4 and -SR4, with R2, R3 and R4 having the meanings given above,

the said organic dye being soluble in the oily phase of the composition.

**2.** Composition according to Claim 1, in which the photochromic organic dye corresponds to one of the formulae (Ia) and (IIa) below:

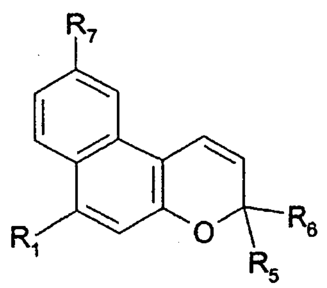

(Ia)

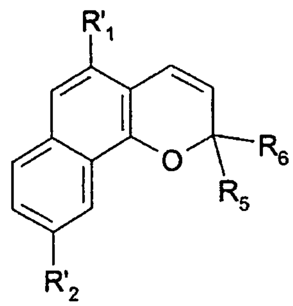

(IIa)

in which R1, R5, R6, R7, R'1 and R'2 are defined as in Claim 1.

**3.** Composition according to either of the preceding claims, in which R1 represents a hydrocarbon-based ring with one of the bonds "f" or "gh" and the radical R7; or a group chosen from -COOR4, -NR2R3, -OR4 and -SR4, in which:

- R2 and R3 either represent, independently of each other, a linear, branched or cyclic, saturated or unsaturated hydrocarbon-based group containing 1 to 20, preferably 1-12 and better still 1 to 6 carbon atoms, optionally comprising 1 to 5 heteroatoms chosen from N, O, S, Si and P,
or, taken together with the nitrogen atom to which they are attached, form a saturated or unsaturated hydrocarbon-based heterocycle containing 3-10 and preferably 4-6 carbon atoms and optionally 1-5 other heteroatoms chosen from N, O, S, Si and P, the said ring optionally being substituted with at least one linear, branched or cyclic, saturated or unsaturated hydrocarbon-based radical containing 1 to 20, preferably 1-15 and better still 1 to 6 carbon atoms, optionally comprising 1 to 5 heteroatoms chosen from N, O, S, Si and P;
- R4 represents a linear, branched or cyclic, saturated or unsaturated hydrocarbon-based group containing 1 to 20, preferably 1-15 and better still 1 to 6 carbon atoms, optionally halogenated or perhalogenated, especially with Fl, Br and/or Cl, and/or optionally comprising 1 to 5 heteroatoms chosen from N, O, S, Si and P.

**4.** Composition according to one of the preceding claims, in which R5 and R6 represent, independently of each other, a group chosen from:

- the saturated cyclic aminoaryl groups of formula (IIa) or (IIb) :

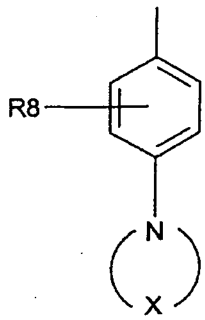

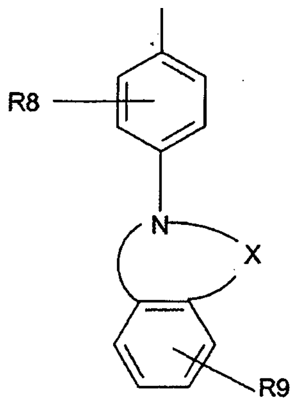

**(IIa)** **(IIb)**

in which the ring comprising N and X is a saturated ring which contains in total 3 to 30, preferably 4-10 and better still 5, 6 or 7 atoms, including nitrogen, the remainder being carbon atoms and/or heteroatoms chosen from O, S, Si and P and/or groups chosen from -NH and -NR with R representing a linear, branched or cyclic, saturated or unsaturated hydrocarbon-based radical containing 1 to 20, preferably 1-15 and better still 1 to 6 carbon atoms, optionally comprising 1 to 5 heteroatoms chosen from N, O, S, Si and P;
- a linear, branched or cyclic, saturated or unsaturated hydrocarbon-based group containing 1 to 30, preferably 2-18 and better still 3 to 12 carbon atoms, optionally comprising 1 to 5 heteroatoms chosen from N, O, S, Si and P; and especially a group chosen from $-C_6H_4-CONR2R3$, $-C_6H_4-NR2R3$ and $-C_6H_4-OR4$ with R2, R3 and R4 having the meanings given in Claim 1.

**5.** Composition according to one of the preceding claims, in which R7 represents a group chosen from:

- (i) linear, branched or cyclic, saturated or unsaturated hydrocarbon-based groups containing 1 to 30, preferably 1-18 and better still 1 to 12 carbon atoms, optionally comprising 1 to 5 heteroatoms chosen from N, O, S, Si and P, and/or optionally halogenated or perhalogenated, especially with Fl, Br and/or Cl;
- (ii) halogen atoms, especially Fl, Br and/or Cl;
- (iii) -CN (nitrile), -COOH (carboxylate), $-NO_2$ (nitro), -N=N- (azo), =NH (imino) or $-CONH_2$ (amide) groups;
- (iv) a hydrogen atom;
- (v) a group chosen from -NR2R3, -OR4 and -SR4, with R2, R3 and R4 having the meanings given in Claim 1;
- (vi) the radical R7 also possibly forming, with one of the bonds "i", "j", "k" or "g,h" taken with the radical R1, or "f" taken with the radical R1, a saturated hydrocarbon-based ring containing in total 3 to 8, preferably 4 to 7 and better still 5 or 6 carbon atoms, optionally comprising 1 to 5 heteroatoms chosen from N, O, S, Si and P.

**6.** Composition according to one of the preceding claims, in which R'1 represents hydrogen or a linear, branched or cyclic, saturated or unsaturated hydrocarbon-based group containing 1 to 30, preferably 1-18 and better still 1 to 12 carbon atoms, optionally comprising 1 to 5 heteroatoms chosen from N, O, S, Si and P, and/or optionally halogenated or perhalogenated, especially with Fl, Br and/or Cl.

**7.** Composition according to one of the preceding claims, in which R'2 represents hydrogen or a group chosen from $-NO_2$, -NR2R3 and -C(O)NR2R3, in which R2 and R3 either represent, independently of each other, a linear, branched or cyclic, saturated or unsaturated hydrocarbon-based group containing 1 to 20, preferably 1-12 and better still 1 to 6 carbon atoms, optionally comprising 1 to 5 heteroatoms chosen from N, O, S, Si and P; or, taken together with the nitrogen atom to which they are attached, form a saturated or unsaturated hydrocarbon-based heterocycle containing 3-10 and preferably 4-6 carbon atoms and optionally 1-5 other heteroatoms chosen from N, O, S, Si and P, the said ring optionally being substituted with at least one linear, branched or cyclic, saturated or unsaturated hydrocarbon-based radical containing 1 to 20, preferably 1-15 and better still 1 to 6 carbon atoms, optionally comprising 1 to 5 heteroatoms chosen from N, O, S, Si and P.

**8.** Composition according to one of the preceding claims, in which the organic dye is of formula (I) or (Ia) for which:

* R1 represents a group -COOR with R being a saturated hydrocarbon-based radical containing 1 to 12 and preferably 1-6 carbon atoms, and especially a methyl or ethyl radical; or a group

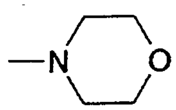

and/or
* R5 and R6 represent, independently of each other, either (i) a group of formula (IIa):

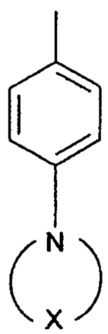

(IIa)

in which the ring comprising N and X is a saturated ring containing in total 4 to 7 atoms and especially 5 or 6 atoms, including nitrogen, and especially 3-5 carbon atoms and 0-1 oxygen atom; and in particular a group of formula:

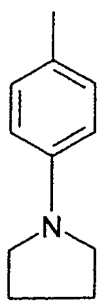

or

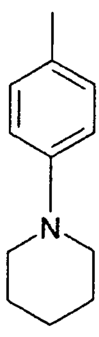

or

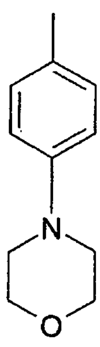

or (ii) a linear, branched or cyclic, saturated or unsaturated hydrocarbon-based group containing 5 to 14 and preferably 6 to 10 carbon atoms, optionally comprising 1 or 2 heteroatoms chosen from N, O and S;
in particular a group

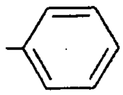 or 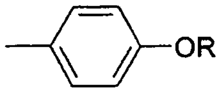 or 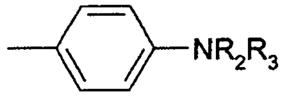

in which R is a saturated hydrocarbon-based radical containing 1 to 12 and preferably 1-6 carbon atoms, and especially a methyl or ethyl radical; and R2 and R3 are, independently of each other, a linear, branched or cyclic, saturated or unsaturated hydrocarbon-based group containing 1 to 20, preferably 1-15 and better still 1 to 6 carbon atoms, optionally comprising 1 to 5 heteroatoms chosen from N, O, S, Si and P;
and/or
* R7 represents a hydrogen atom or a group -NR2R3, with R2 and R3 representing, independently of each other, a linear or branched, saturated hydrocarbon-based group containing 1 to 12 and preferably 1-6 carbon atoms, and especially a methyl and/or ethyl group.

**9.** Composition according to one of Claims 1 to 7, in which the organic dye is of formula (IIa) for which:

* R'1 represents hydrogen or a group -COOR with R being a saturated hydrocarbon-based radical containing 1 to 12 and preferably 1-6 carbon atoms, and especially a methyl or ethyl radical;
and/or
* R5 and R6 represent, independently of each other, either (i) a group of formula (IIa):

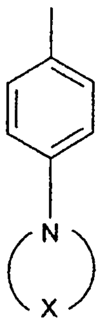

(IIa)

in which the ring comprising N and X is a saturated ring containing in total 4 to 7 atoms and especially 5 to 6 atoms, including nitrogen, and especially 4-5 carbon atoms and 0-1 oxygen atom; and in particular a group of formula:

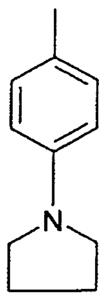 or 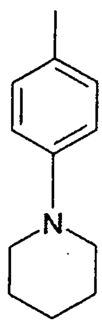 or 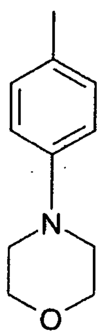

or (ii) a linear, branched or cyclic, saturated or unsaturated hydrocarbon-based group containing 5 to 14 and preferably 6 to 10 carbon atoms, optionally comprising 1 or 2 heteroatoms chosen from N, O and S;

in particular a group:

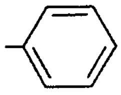 or 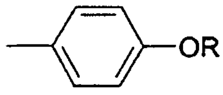 or 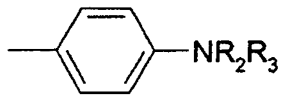

in which R is a saturated hydrocarbon-based radical containing 1 to 12 and preferably 1-6 carbon atoms, and especially a methyl or ethyl radical; and R2 and R3 are, independently of each other, a linear, branched or cyclic, saturated or unsaturated hydrocarbon-based group containing 1 to 20, preferably 1-15 and better still 1 to 6 carbon atoms, optionally comprising 1 to 5 heteroatoms chosen from N, O, S, Si and P;
and/or
* R'2 represents hydrogen or a group -NR'R", with R' and R", which may be identical or different, representing a linear or branched, saturated hydrocarbon-based group containing 1 to 12 and preferably 1-6 carbon atoms, and especially a methyl and/or ethyl group; or a group

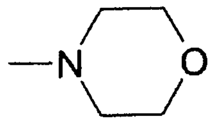

**10.** Composition according to one of the preceding claims, in which the organic dye is chosen from:

- 3,3-bis(4-methoxyphenyl)-6-morpholino-3H-naphtho-[2,1-b]pyran of formula:

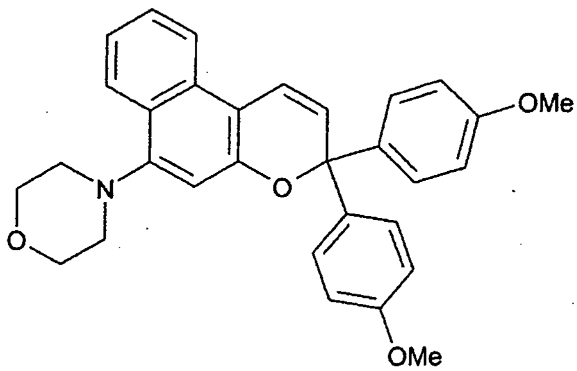

- 3-phenyl-3-(4-morpholinophenyl)-6-morpholino-3H-naphtho[2,1-b]pyran of formula:

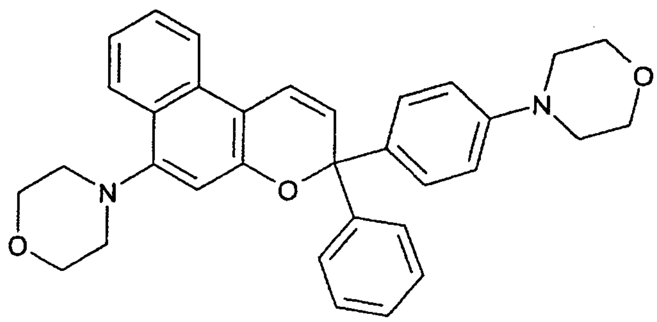

- 3-phenyl-3-(4-piperidinophenyl)-6-morpholino-3H-naphtho [2,1-b] pyran of formula:

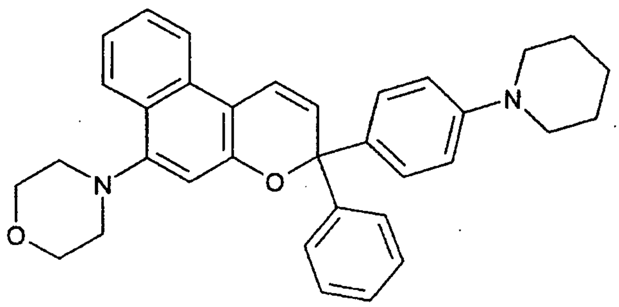

- 3-phenyl-3-(4-piperidinophenyl)-6-methoxycarbonyl-9-N-dimethyl-3H-naphtho[2,1-b]pyran of formula:

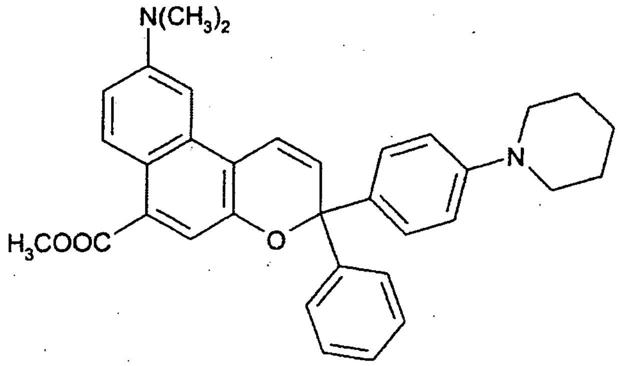

- 2-phenyl-2-(4-piperidinophenyl)-5-methoxycarbonyl-9-N-dimethyl-2H-naphtho[1,2-b]pyran of formula:

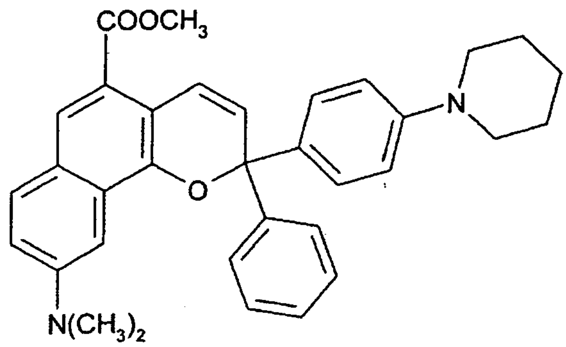

- mixtures thereof.

**11.** Composition according to one of the preceding claims, in which the organic dye according to the invention is included, alone or as a mixture, in an amount of from 0.001% to 20% by weight, especially from 0.005% to 10% by weight, preferably 0.01% to 4.8% by weight, better still from 0.05% to 3% by weight, preferentially from 0.1% to 2% by weight, relative to the total weight of the cosmetic composition.

**12.** Composition according to one of the preceding claims, in which the oily phase is polar and has a mean solubility parameter $\delta_a$ according to the Hansen solubility space, at 25°C, of greater than or equal to 5.0 $(J/cm^3)^{1/2}$, especially greater than or equal to 5.3, or even greater than or equal to 5.5 and better still greater than or equal to 6.0 $(J/cm^3)^{1/2}$, or even greater than or equal to 7.0 $(J/cm^3)^{1/2}$.

**13.** Composition according to one of the preceding claims, in which the oily phase comprises 5% to 100% by weight, especially 10-90%, or even 15-60% and better still 20-50% by weight, relative to the total weight of the said oily phase, of one or more polar oils with a mean solubility parameter $\delta_a$ according to the Hansen solubility space, at

25°C, of greater than or equal to 5.0 $(J/cm^3)^{1/2}$.

**14.** Composition according to one of Claims 1 to 11, in which the oily phase is apolar and has a mean solubility parameter $\delta_a$ according to the Hansen solubility space, at 25°C, of less than 5.0, especially less than or equal to 4.9, better still less than or equal to 4.5 and even better still less than or equal to 4.0 $(J/cm^3)^{1/2}$.

**15.** Composition according to one of Claims 1-11 and 14, in which the oily phase comprises 5% to 100% by weight, especially 10-90%, or even 15-60% and better still 20-50% by weight, relative to the total weight of the said oily phase, of one or more apolar oils with a mean solubility parameter $\delta_a$ according to the Hansen solubility space, at 25°C, of less than 5.0 $(J/cm^3)^{1/2}$.

**16.** Composition according to one of the preceding claims, in which the oily phase comprises at least one oil chosen from:

- animal or plant oils formed from fatty acid esters of polyols, in particular liquid triglycerides, for example sunflower oil, corn oil, soybean oil, marrow oil, grapeseed oil, sesame seed oil, hazelnut oil, apricot oil, almond oil or avocado oil; fish oils or glyceryl tricaprocaprylate, or plant or animal oils of formula $R_1COOR_2$ in which $R_1$ represents a higher fatty acid residue containing from 7 to 19 carbon atoms and $R_2$ represents a branched hydrocarbon-based chain containing from 3 to 20 carbon atoms, for example Purcellin oil; liquid paraffin, liquid petroleum jelly, beauty-leaf oil, macadamia oil, rapeseed oil, coconut oil, groundnut oil, palm oil, castor oil, jojoba oil, olive oil or cereal germ oil; shea butter oil; perhydrosqualene;
- synthetic esters and ethers, especially of fatty acids, for instance the oils of formula $R_1COOR_2$ in which $R_1$ represents a higher fatty acid residue containing from 7 to 29 carbon atoms and $R_2$ represents a hydrocarbon-based chain containing from 3 to 30 carbon atoms, for instance isopropyl myristate, 2-ethylhexyl palmitate, 2-octyldodecyl stearate, 2-octyldodecyl erucate, isononyl isononanoate or isostearyl isostearate; hydroxylated esters, for instance isostearyl lactate, octyl hydroxystearate, octyldodecyl hydroxystearate, diisostearyl malate, triisocetyl citrate, and fatty alkyl heptanoates, octanoates and decanoates; polyol esters, for instance propylene glycol dioctanoate, neopentyl glycol diheptanoate or diethylene glycol diisononanoate; pentaerythritol esters, for instance pentaerythrityl tetraisostearate; esters of the type such as tridecyl trimellitate;
- fatty alcohols containing from 12 to 26 carbon atoms, for instance octyldodecanol, 2-butyloctanol, 2-hexyldecanol, 2-undecylpentadecanol or oleyl alcohol;
- linear or branched hydrocarbons of mineral or synthetic origin, such as liquid paraffins and derivatives thereof, petroleum jelly, polydecenes, and hydrogenated polyisobutene such as parleam; isoparaffins, for instance isohexadecane and isodecane;
- glycerides and especially acetylglycerides or triglycerides of fatty acids containing 4 to 10 carbon atoms, for instance heptanoic, octanoic and capric/ caprylic acid triglycerides.

**17.** Composition according to one of the preceding claims, in which the oily phase comprises at least one oil chosen from octyldodecanol, hexyldecanol, octyldecanol, oleyl alcohol, castor oil, diisostearyl malate, glyceryl triheptanoate, glyceryl trioctanoate, capric/caprylic acid triglyceride, triisononanoin, tridecyl trimellitate, aliphatic hydrocarbons, especially of C6-C40, for instance volatile liquid paraffins, such as isohexadecane or isododecane, or non-volatile liquid paraffins, and derivatives thereof; petroleum jelly, hydrogenated or non-hydrogenated polydecenes, hydrogenated polyisobutene such as parleam oil, squalane, polybutylenes and isononyl isononanoate; fluoro oils and especially perfluoro oils, and mixtures thereof.

**18.** Composition according to one of Claims 1-11, in which the oily phase comprises at least one phenylsilicone oil of formula (VI), or a mixture of such oils:

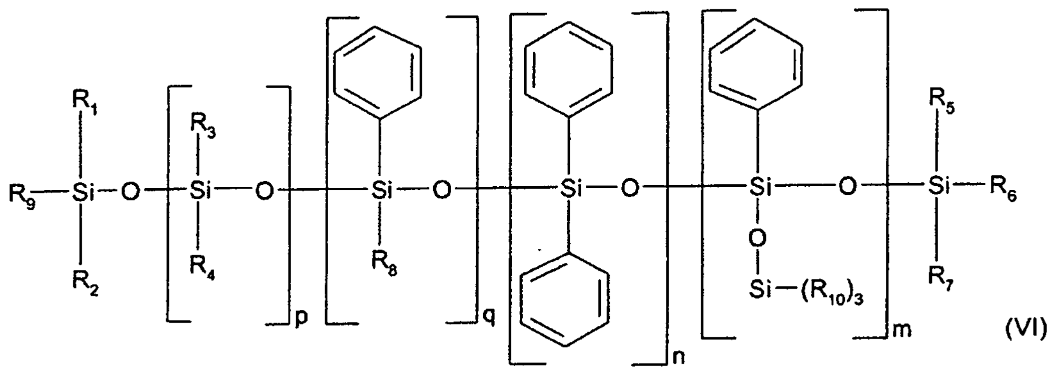

(VI)

in which

- R1 to R10, independently of each other, are saturated or unsaturated, linear, cyclic or branched C1-C30 hydrocarbon-based radicals,
- m, n, p and q are, independently of each other, integers between 0 and 900, with the proviso that the sum "m+n+q" is other than 0.

**19.** Composition according to Claim 18, in which, in the phenylsilicone oil of formula (VI), the sum "m+n+q" is between 1 and 100 and/or the sum "m+n+p+q" is between 1 and 900 and better still between 1 and 800.

**20.** Composition according to either of Claims 18 and 19, in which the phenylsilicone oil is of formula (VII) :

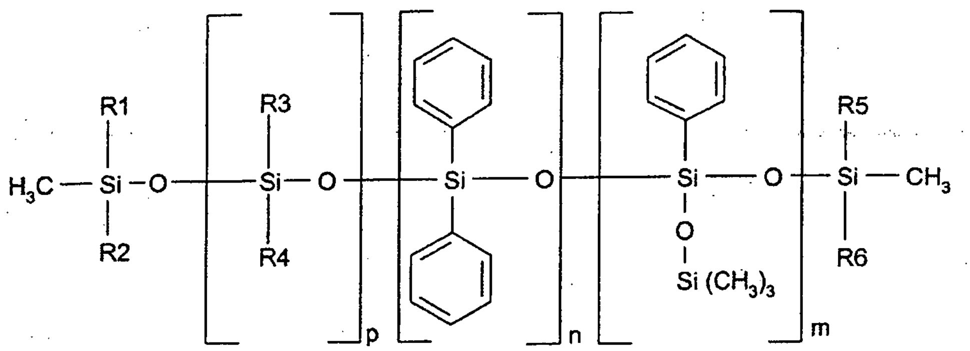

(VII)

in which:

- R1 to R6, independently of each other, are saturated or unsaturated, linear, cyclic or branched C1-C30 hydrocarbon-based radicals,
- m, n and p are, independently of each other, integers between 0 and 100, with the proviso that the sum "n+m" is between 1 and 100.

**21.** Composition according to one of Claims 18 to 20, in which, in formulae (VI) and (VII), R1 to R6, independently of each other, represent a saturated linear or branched C1-C30 and especially C1-C12 hydrocarbon-based radical, and in particular a methyl, ethyl, propyl or butyl radical.

**22.** Composition according to one of Claims 18 to 21, in which, in formulae (VI) and (VII), R1 to R6 are identical and are a methyl radical.

**23.** Composition according to one of Claims 18 to 22, in which the phenylsilicone oil is chosen from phenyl trimethicones, phenyl dimethicones, phenyltrimethylsiloxydiphenylsiloxanes, diphenyl dimethicones and diphenylmethyldiphenyltrisiloxanes, and mixtures thereof.

**24.** Composition according to one of Claims 18 to 23, in which the phenylsilicone oil is present in an amount of between 1-99% by weight, especially 5-90% by weight, preferably 7-50% by weight, or even 12-40% by weight, relative to the total weight of the cosmetic composition.

**25.** Composition according to one of Claims 18 to 24, in which the oily phase of the composition comprises one or more other additional cosmetically acceptable, polar or apolar, volatile or non-volatile, silicone-based or hydrocarbon-based oils, which may be present in an amount of between 0.1-50% by weight, especially 4-30% by weight and preferably 10-25% by weight, relative to the total weight of the cosmetic composition.

**26.** Composition according to one of the preceding claims, in which the oily phase is included in an amount of from 1% to 99% by weight, especially 10% to 90% by weight and preferably 15% to 80% by weight, relative to the total weight of the cosmetic composition.

**27.** Composition according to one of the preceding claims, also comprising other fatty substances chosen from waxes, gums and/or pasty fatty substances of animal, plant, mineral or synthetic origin, and also mixtures thereof; and/or a particulate phase, which may comprise pigments, nacres, fillers, dyes and/or other photochromic compounds; and/or UV-screening agents; and/or an aqueous phase; and/or a surfactant and/or a thickener and/or a film-forming polymer.

**28.** Composition according to one of the preceding claims, with a ΔE value of greater than or equal to 5, preferably greater than or equal to 10, better still greater than or equal to 25 and even better still greater than or equal to 35, or even greater than or equal to 45.

**29.** Composition according to one of the preceding claims, which is in the form of a care and/or makeup product for body or facial skin, for the lips and for the hair, an antisun product or self-tanning product, or a haircare product.

**30.** Composition according to one of the preceding claims, which is in the form of a lipstick, a foundation, a makeup rouge, an eyeshadow, a loose or compact powder, a tinted cream, a body makeup product, a skin-colouring product, an eyeliner or a mascara.

**31.** Cosmetic process for treating a support chosen from mucous membranes, semi-mucous membranes, the skin and/or the integuments, in which a cosmetic composition as defined in one of Claims 1 to 30 is applied to the said support.

**32.** Process for making up and/or temporarily colouring a support chosen from mucous membranes, semi-mucous membranes, the skin and/or the integuments, in which a cosmetic composition as defined in one of Claims 1 to 30 is applied to the said support.

**Patentansprüche**

**1.** Kosmetische Zusammensetzung, die in einem kosmetisch akzeptablen Medium mindestens eine Ölphase und mindestens einen photochromen organischen Farbstoff der Formel (I) oder (II)

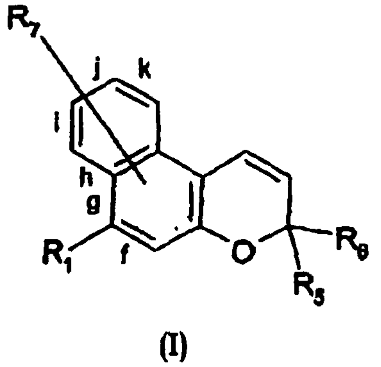

(I)

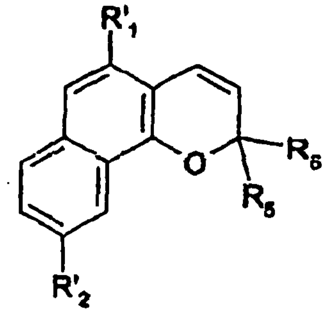

(IIa)

enthält, in denen bedeuten:

* $R_1$:

- (i) eine geradkettige, verzweigte oder cyclische, gesättigte oder ungesättigte Kohlenwasserstoffgruppe, die 1 bis 30, vorzugsweise 1 bis 18 und besser 1 bis 12 oder sogar 1 bis 6 Kohlenstoffatome aufweist, die gegebenenfalls 1 bis 5 Heteroatome enthält, die unter N, O, S, Si und P ausgewählt werden, und/oder die gegebenenfalls halogeniert oder perhalogeniert ist, insbesondere mit F, Br und/oder Cl;
- (ii) einen Kohlenwasserstoffring, der mit einer der Bindungen "f" oder "gh" und dem Rest $R_7$ gebildet wird; oder
- (iii) eine Gruppe, die unter $-COOR_4$, $-C(O)NR_2R_3$, $-NR_2R_3$, $-OR_4$ und $-SR_4$ ausgewählt wird, worin bedeuten:

  - $R_2$ und $R_3$ unabhängig voneinander eine geradkettige, verzweigte oder cyclische, gesättigte oder ungesättigte Kohlenwasserstoffgruppe, die 1 bis 20, vorzugsweise 1 bis 12 und besser 1 bis 6 Kohlenstoffatome aufweist, die gegebenenfalls 1 bis 5 Heteroatome enthält, die unter N, O, S, Si und P ausgewählt werden,
  oder die zusammengenommen mit dem Stickstoffatom, mit dem sie verbunden sind, einen gesättigten oder ungesättigten Kohlenwasserstoffheterocylus bilden, der 3 bis 10, vorzugsweise 4 bis 6 Kohlenstoffatome und gegebenenfalls 1 bis 5 weitere Heteroatome enthält, die unter N, O, S, Si und P ausgewählt werden, wobei der Ring gegebenenfalls mit mindestens einem geradkettigen, verzweigten oder cyclischen, gesättigten oder ungesättigten Kohlenwasserstoffrest substituiert ist, der 1 bis 20, vorzugsweise 1 bis 15 und besser 1 bis 6 Kohlenstoffatome aufweist, der gegebenenfalls 1 bis 5 Heteroatome enthält, die unter N, O, S, Si und P ausgewählt werden;
  - $R_4$ eine geradkettige, verzweigte oder cyclische, gesättigte oder ungesättigte Kohlenwasserstoffgruppe, die 1 bis 20, vorzugsweise 1 bis 15 und besser 1 bis 6 Kohlenstoffatome aufweist, die gegebenenfalls halogeniert oder perhalogeniert ist, insbesondere mit F, Br und/oder Cl, und/ oder die gegebenenfalls 1 bis 5 Heteroatome enthält, die unter N, O, S, Si und P ausgewählt werden;

* $R_5$ und $R_6$ unabhängig voneinander eine Gruppe, die ausgewählt wird unter:

- (i) den gesättigten cyclischen Aminoarylgruppen der Formel (IIa) oder (IIb):

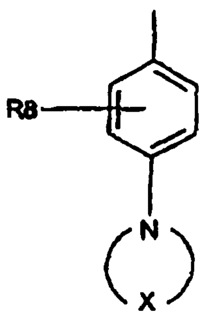
(IIa)

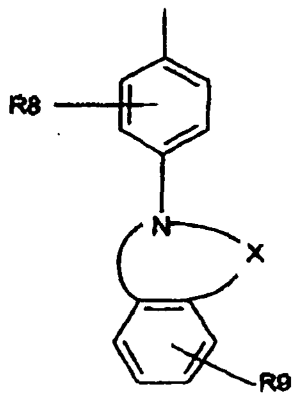
(IIb)

in denen der Ring, der N und X enthält, ein gesättigter Ring ist, der insgesamt 3 bis 30 Atome, vorzugsweise 4 bis 10 Atome und noch besser 5, 6 oder 7 Atome, der Stickstoff eingeschlossen, wobei der Rest aus Kohlenstoffatomen und/oder Heteroatomen, die unter O, S, Si, P ausgewählt werden, und/oder Gruppen besteht, die unter -NH und -NR ausgewählt werden, wobei R einen geradkettigen, verzweigten oder cyclischen, gesättigten oder ungesättigten Kohlenwasserstoffrest darstellt, der 1 bis 20, vorzugsweise 1 bis 15 und besser 1 bis 6 Kohlenstoffatome aufweist, der gegebenenfalls 1 bis 5 Heteroatome enthält, die unter N, O, S, Si und P ausgewählt werden;
- (ii) den Indolinoarylgruppen der Formel (III):

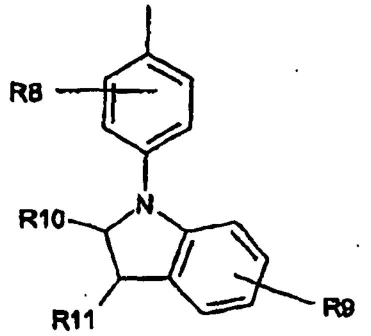

(III),

in der $R_{10}$ und $R_{11}$ unabhängig voneinander eine Gruppe darstellen, die ausgewählt wird unter (i) den geradkettigen, verzweigten oder cyclischen, gesättigten oder ungesättigten Kohlenwasserstoffgruppen, die 1 bis 30, vorzugsweise 1 bis 18 und besser 1 bis 12, sogar 1 bis 6 Kohlenstoffatome aufweisen, die gegebenenfalls 1 bis 5 Heteroatome enthalten, die unter N, O, S, Si und P ausgewählt werden, und/oder die gegebenenfalls halogeniert oder perhalogeniert sind, insbesondere mit F, Br und/oder Cl; (ii) den Halogenatomen und insbesondere F, Br und/oder Cl; (iii) den Gruppen -CN (Nitril), -COOH (Carboxylat), $-NO_2$ (Nitro); (iv) einem Wasserstoffatom; (v) einer Gruppe, die unter - $C(O)NR_2R_3$, $-NR_2R_3$, $-OR_4$ oder $-SR_4$ ausgewählt wird, worin $R_2$, $R_3$ und $R_4$ die weiter oben angegebenen Bedeutungen haben; (vi) wobei die Reste $R_{10}$ und $R_{11}$ miteinander einen gesättigten oder ungesättigten Kohlenwasserstoffring bilden können, der insgesamt 5 bis 8 Atome (eingeschlossen die Atome des Indolinrings) aufweisen, wobei diese Atome unter C, O, S und/oder NR ausgewählt werden, wobei R Wasserstoff oder einen geradkettigen, verzweigten oder cyclischen, gesättigten oder ungesättigten Kohlenwasserstoffrest darstellt, der gegebenenfalls 1 bis 5 Heteroatome enthält, die unter N, O, S, Si und P ausgewählt werden,

- (iii) den Gruppen der Formel (IV):

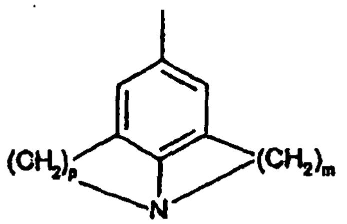

(IV),

worin m und p unabhängig voneinander ganze Zahlen sind, die im Bereich von 2 bis 5 liegen;

- (iv) den ungesättigten cyclischen Aminoarylgruppen der Formeln (Va), (Vb) oder (Vc):

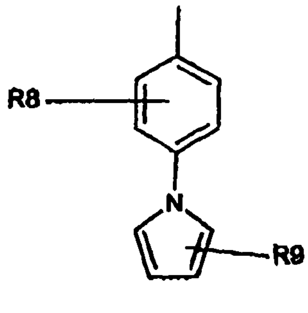

(Va)

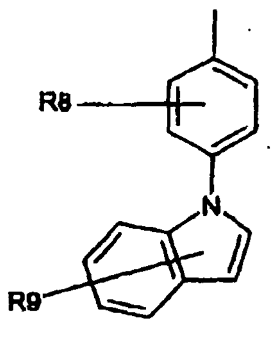

(Vb)

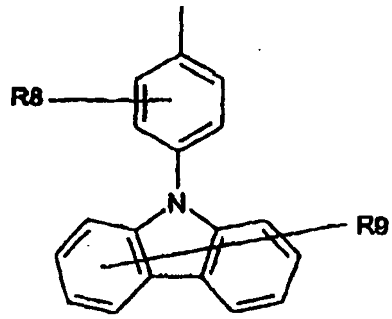

(Vc),

in denen $R_8$ und $R_9$ unabhängig voneinander eine Gruppe darstellen, die ausgewählt wird unter (i) den geradkettigen, verzweigten oder cyclischen, gesättigten oder ungesättigten Kohlenwasserstoffgruppen, die 1 bis 30, vorzugsweise 1 bis 18 und besser 1 bis 12, sogar 1 bis 6 Kohlenstoffatome aufweisen, die gegebenenfalls 1 bis 5 Heteroatome enthalten, die unter N, O, S, Si und P ausgewählt werden, und/oder die gegebenenfalls halogeniert oder perhalogeniert sind, insbesondere mit F, Br und/oder Cl; (ii) den Halogenatomen, insbesondere F, Br und/oder Cl; (iii) den Gruppen -CN (Nitril), -COOH (Carboxylat), $-NO_2$ (Nitro); (iv) einem Wasserstoffatom; (v) einer Gruppe, die unter - $C(O)NR_2R_3$, $-NR_2R_3$, $OR_4$ oder $SR_4$ ausgewählt wird, worin $R_2$, $R_3$ und $R_4$ die weiter oben angegebenen Bedeutungen haben;
- (v) einer geradkettigen, verzweigten oder cyclischen, gesättigten oder ungesättigten Kohlenwasserstoffgruppe, die 1 bis 30, vorzugsweise 2 bis 18 und besser 3 bis 12 Kohlenstoffatome aufweist, die gegebenenfalls 1 bis 5 Heteroatome enthält, die unter N, O, S, Si und P ausgewählt werden; und insbesondere einer Gruppe, die unter $-C_6H_4-CONR_2R_3$,
- $C_6H_4-NR_2R_3$ und $-C_6H_4-OR_4$ ausgewählt wird, worin $R_2$, $R_3$ und $R_4$ die weiter oben angegebenen Bedeutungen haben;

* $R_7$ eine Gruppe, die ausgewählt wird unter:

- (i) den geradkettigen, verzweigten oder cyclischen, gesättigten oder ungesättigten Kohlenwasserstoffgruppen, die 1 bis 30, vorzugsweise 1 bis 18 und besser 1 bis 12 Kohlenstoffatome aufweisen, die gegebenenfalls 1 bis 5 Heteroatome enthalten, die unter N, O, S, Si und P ausgewählt werden, und/ oder die gegebenenfalls halogeniert oder perhalogeniert sind, insbesondere mit F, Br und/ oder Cl;
- (ii) den Halogenatomen und insbesondere F, Br und/oder Cl;
- (iii) den Gruppen -CN (Nitril), -COOH (Carboxylat), $-NO_2$ (Nitro); -N=N- (Azo); -NH (Imino); $-CONH_2$;
- (iv) einem Wasserstoffatom;
- (v) einer Gruppe, die ausgewählt wird unter $-C(O)NR_2R_3$, $-NR_2R_3$, $OR_4$ oder $-SR_4$, wobei $R_2$, $R_3$ und $R_4$ die weiter oben angegebenen Bedeutungen haben;
- (vi) wobei der Rest $R_7$ außerdem mit einer der Bindungen "i", "j", "k" oder "g,h", zusammen genommen mit dem Rest $R_1$, oder "f", zusammen genommen mit dem Rest $R_1$, einen gesättigten Kohlenwasserstoüring bilden kann, der insgesamt 3 bis 8, vorzugsweise 4 bis 7 und besser 5 oder 6 Kohlenstoffatome aufweist, der gegebenenfalls 1 bis 5 Heteroatome enthält, die unter N, O, S, Si und P ausgewählt werden;

* $R'_1$ eine Gruppe, die ausgewählt wird unter;

- (i) einem Wasserstoffatom;
- (ii) einer geradkettigen, verzweigten oder cyclischen, gesättigten oder ungesättigten Kohlenwasserstoffgruppe, die 1 bis 30, vorzugsweise 1 bis 18 und besser 1 bis 12 Kohlenstoffatome aufweist, die gegebenenfalls 1 bis 5 Heteroatome enthält, die unter N, O, S, Si und P ausgewählt werden, und/oder die gegebenenfalls halogeniert oder perhalogeniert ist, insbesondere mit F, Br und/oder Cl;
- (iii) einer Gruppe, die ausgewählt wird unter $-C(O)NR_2R_3$, $-NR_2R_3$, $-OR_4$ und $-SR_4$, wobei $R_2$, $R_3$ und $R_4$ die weiter oben angegebenen Bedeutungen haben;

* $R'_2$ eine Gruppe, die ausgewählt wird unter:

- (i) den geradkettigen, verzweigten oder cyclischen, gesättigten oder ungesättigten Kohlenwasserstoffgruppen, die 1 bis 30, vorzugsweise 1 bis 18 und besser 1 bis 12 Kohlenstoffatome aufweisen, die gegebenenfalls 1 bis 5 Heteroatome enthalten, die unter N, O, S, Si und P ausgewählt werden, und/oder die gegebenenfalls halogeniert oder perhalogeniert sind, insbesondere mit F, Br und/oder Cl;
- (ii) den Halogenatomen und insbesondere F, Br und/oder Cl;
- (iii) den Gruppen -CN (Nitril), -COOH (Carboxylat), $-NO_2$ (Nitro); -N=N- (Azo); =NH (Imino); $-CONH_2$ (Amid);
- (iv) einem Wasserstoffatom;
- (v) einer Gruppe, die unter $-C(O)NR_2R_3$, $-NR_2R_3$, $-OR_4$ oder $SR_4$ ausgewählt wird, wobei $R_2$, $R_3$ und $R_4$ die weiter oben angegebenen Bedeutungen haben,

wobei der organische Farbstoff in der Ölphase der Zusammensetzung löslich ist.

**2.** Zusammensetzung nach Anspruch 1, in der der photochrome organische Farbstoff einer der folgenden Formeln (Ia) und (IIa)

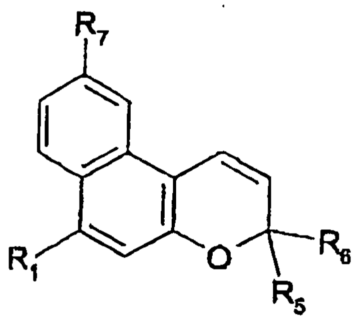

(Ia)

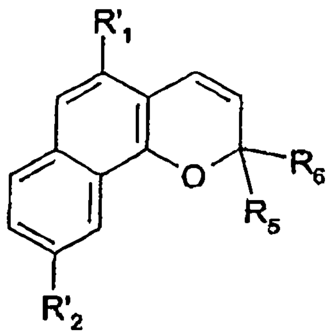

(IIa)

entspricht, worin $R_1$, $R_5$, $R_6$, $R_7$, $R'_1$ und $R'_2$ wie in Anspruch 1 definiert sind.

**3.** Zusammensetzung nach einem der vorhergehenden Ansprüche, in der $R_1$ mit einer der Bindungen "f" oder "gh" und dem Rest $R_7$ einen Kohlenwasserstoffring oder eine Gruppe darstellt, die unter $-COOR_4$,

- $NR_2R_3$, $-OR_4$ und $-SR_4$ ausgewählt wird, worin bedeuten:
- $R_2$ und $R_3$ unabhängig voneinander eine geradkettige, verzweigte oder cyclische, gesättigte oder ungesättigte Kohlenwasserstoffgruppe, die 1 bis 20, vorzugsweise 1 bis 12 und besser 1 bis 6 Kohlenstoffatome aufweist, die gegebenenfalls 1 bis 5 Heteroatome enthält, die unter N, O, S, Si und P ausgewählt werden, oder die zusammen mit dem Stickstoffatom, mit dem sie verbunden sind, einen gesättigten oder ungesättigten Kohlenwasserstoffheterocyclus bilden, der 3 bis 10, vorzugsweise 4 bis 6 Kohlenstoffatome und gegebenenfalls 1 bis 5 weitere Heteroatome enthält, die unter N, O, S, Si und P ausgewählt werden, wobei der Ring gegebenenfalls mit mindestens einem geradkettigen, verzweigten oder cyclischen, gesättigten oder ungesättigten Kohlenwasserstoffrest substituiert ist, der 1 bis 20, vorzugsweise 1 bis 15 und besser 1 bis 6 Kohlenstoffatome aufweist, der gegebenenfalls 1 bis 5 Heteroatome enthält, die unter N, O, S, Si und P ausgewählt werden;
- $R_4$ eine geradkettige, verzweigte oder cyclische, gesättigte oder ungesättigte Kohlenwasserstoffgruppe, die 1 bis 20, vorzugsweise 1 bis 15 und besser 1 bis 6 Kohlenstoffatome aufweist, die gegebenenfalls halogeniert oder perhalogeniert ist, insbesondere mit F, Br und/oder Cl, und/oder die gegebenenfalls 1 bis 5 Heteroatome enthält, die unter N, O, S, Si und P ausgewählt werden.

**4.** Zusammensetzung nach einem der vorhergehenden Ansprüche, in der $R_5$ und $R_6$ unabhängig voneinander eine Gruppe darstellen, die ausgewählt wird unter:

- den gesättigten cyclischen Aminoarylgruppen der Formel (IIa) oder (IIb)

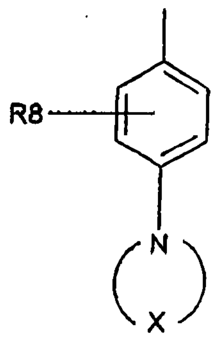

(IIa)

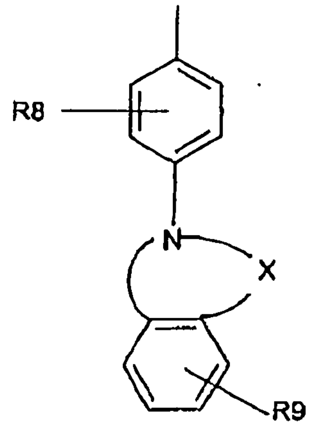

(IIb)

in denen der Ring, der N und X enthält, ein gesättigter Ring ist, der insgesamt 3 bis 30 Atome, vorzugsweise 4 bis 10 Atome und noch besser 5, 6 oder 7 Atome, der Stickstoff eingeschlossen, enthält, wobei der Rest Kohlenstoffatome und/oder Heteroatome, die unter O, S, Si, P ausgewählt werden, und/oder Gruppen sind, die unter -NH und -NR ausgewählt werden, wobei R einen geradkettigen, verzweigten oder cyclischen, gesättigten oder ungesättigten Kohlenwasserstoffrest darstellt, der 1 bis 20, vorzugsweise 1 bis 15 und besser 1 bis 6 Kohlenstoffatome aufweist, der gegebenenfalls 1 bis 5 Heteroatome enthält, die unter N, O, S, Si und P ausgewählt werden;

- einer geradkettigen, verzweigten oder cyclischen, gesättigten oder ungesättigten Kohlenwasserstoffgruppe, die 1 bis 30, vorzugsweise 2 bis 18 und besser 3 bis 12 Kohlenstoffatome aufweist, die gegebenenfalls 1 bis 5 Heteroatome enthält, die unter N, O, S, Si und P ausgewählt werden; und insbesondere einer Gruppe, die unter $-C_6H_4-CONR_2R_3$, $-C_6H_4-NR_2R_3$ und $-C_6H_4-OR_4$ ausgewählt wird, wobei $R_2$, $R_3$ und $R_4$ die in Anspruch 1 angegebenen Bedeutungen haben.

**5.** Zusammensetzung nach einem der vorhergehenden Ansprüche, in der $R_7$ eine Gruppe darstellt, die ausgewählt wird unter:

- (i) den geradkettigen, verzweigten oder cyclischen, gesättigten oder ungesättigten Kohlenwasserstoffgruppen, die 1 bis 30, vorzugsweise 1 bis 18 und besser 1 bis 12 Kohlenstoffatome aufweisen, die gegebenenfalls 1 bis 5 Heteroatome enthalten, die unter N, O, S, Si und P ausgewählt werden, und/ oder die gegebenenfalls halogeniert oder perhalogeniert sind, insbesondere mit F, Br und/ oder Cl;
- (ii) den Halogenatomen und insbesondere F, Br und/oder Cl;
- (iii) den Gruppen -CN (Nitril), -COOH (Carboxylat), $-NO_2$ (Nitro); - N=N- (Azo); =NH (Imino); $-CONH_2$;
- (iv) einem Wasserstoffatom;
- (v) einer Gruppe, die unter $-C(O)NR_2R_3$, $-NR_2R_3$, $OR_4$ oder $-SR_4$ ausgewählt wird, wobei $R_2$, $R_3$ und $R_4$ die in Anspruch 1 angegebenen Bedeutungen haben;
- (vi) wobei der Rest $R_7$ außerdem mit einer der Bindungen "i", "j", "k" oder "g,h", zusammen genommen mit dem Rest $R_1$, oder "f", zusammen genommen mit dem Rest $R_1$, einen gesättigten Kohlenwasserstoffring bilden kann, der insgesamt 3 bis 8, vorzugsweise 4 bis 7 und besser 5 oder 6 Kohlenstoffatome aufweist, der gegebenenfalls 1 bis 5 Heteroatome enthält, die unter N, O, S, Si und P ausgewählt werden.

**6.** Zusammensetzung nach einem der vorhergehenden Ansprüche, in der $R'_1$ ein Wasserstoffatom oder eine geradkettige, verzweigte oder cyclische, gesättigte oder ungesättigte Kohlenwasserstoffgruppe darstellt, die 1 bis 30, vorzugsweise 1 bis 18 und besser 1 bis 12 Kohlenstoffatome aufweist, die gegebenenfalls 1 bis 5 Heteroatome enthält, die unter N, O, S, Si und P ausgewählt werden, und/oder die gegebenenfalls halogeniert oder perhalogeniert ist, insbesondere mit F, Br und/oder Cl.

**7.** Zusammensetzung nach einem der vorhergehenden Ansprüche, in der $R'_2$ ein Wasserstoffatom oder eine Gruppe

darstellt, die unter - $NO_2$, -$NR_2R_3$ und -C(O)$NR_2R_3$ ausgewählt wird, in denen $R_2$ und $R_3$ unabhängig voneinander eine geradkettige, verzweigte der cyclische, gesättigte oder ungesättigte Kohlenwasserstoffgruppe darstellen, die 1 bis 20, vorzugsweise 1 bis 12 und besser 1 bis 6 Kohlenstoffatome aufweist, die gegebenenfalls 1 bis 5 Heteroatome enthält, die unter N, O, S, Si und P ausgewählt werden; oder die zusammen genommen mit dem Stickstoffatom, mit dem sie verbunden sind, einen gesättigten oder ungesättigten Kohlenwasserstoffheterocyclus bilden, der 3 bis 10, vorzugsweise 4 bis 6 Kohlenstoffatome und gegebenenfalls 1 bis 5 weitere Heteroatome enthält, die unter N, O, S, Si und P ausgewählt werden, wobei der Ring gegebenenfalls mit mindestens einem geradkettigen, verzweigten oder cyclischen, gesättigten oder ungesättigten Kohlenwasserstoffrest substituiert ist, der 1 bis 20, vorzugsweise 1 bis 15 und besser 1 bis 6 Kohlenstoffatome aufweist, der gegebenenfalls 1 bis 5 Heteroatome enthält, die unter N, O, S, Si und P ausgewählt werden.

**8.** Zusammensetzung nach einem der vorhergehenden Ansprüche, in der der organische Farbstoff der Formel (I) oder (Ia) entspricht, in denen bedeuten:

* $R_1$ eine Gruppe -COOR, wobei R ein gesättigter Kohlenwasserstoffrest ist, der 1 bis 12, vorzugsweise 1 bis 6 Kohlenstoffatome aufweist und insbesondere ein Methyl- oder Ethylrest ist, oder eine Gruppe

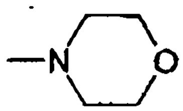

und/oder

* $R_5$ und $R_6$ unabhängig voneinander (i) eine Gruppe der Formel (IIa)

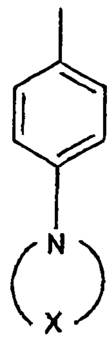

(IIa),

in der der Ring, der N und X enthält, ein gesättigter Ring ist, der insgesamt 4 bis 7, insbesondere 5 oder 6 Atome, der Stickstoff eingeschlossen, und insbesondere 3 bis 5 Kohlenstoffatome und 0 bis 1 Sauerstoffatome enthält; und vor allem eine Gruppe der Formel:

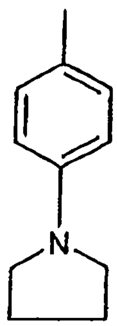

oder

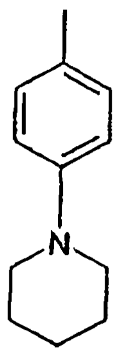

odeı

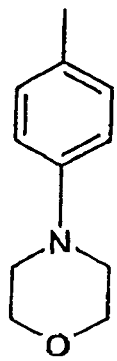

oder (ii) eine geradkettige, verzweigte oder cyclische, gesättigte oder ungesättigte Kohlenwasserstoffgruppe, die 5 bis 14, vorzugsweise 6 bis 10 Kohlenstoffatome aufweist, die gegebenenfalls 1 bis 2 Heteroatome enthält, die unter N, O und S ausgewählt werden;
vor allem eine Gruppe

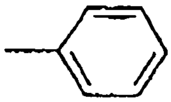 oder 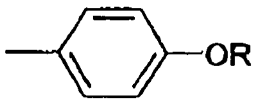 oder 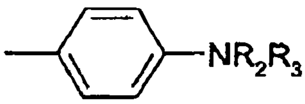

in denen R ein gesättigter Kohlenwasserstoffrest ist, der 1 bis 12, vorzugsweise 1 bis 6 Kohlenstoffatome aufweist, und insbesondere ein Methylrest oder Ethylrest; und worin $R_2$ und $R_3$ unabhängig voneinander eine geradkettige, oder verzweigte oder cyclische, gesättigte oder ungesättigte Kohlenwasserstoffgruppe bedeuten, die gegebenenfalls 1 bis 5 Heteroatome enthält, die unter N, O, S, Si und P ausgewählt werden;
und/oder
* $R_7$ ein Wasserstoffatom oder eine Gruppe $-NR_2R_3$, wobei $R_2$ und $R_3$ unabhängig voneinander eine gesättigte, geradkettige oder verzweigte Kohlenwasserstoffgruppe darstellen, die 1 bis 12, vorzugsweise 1 bis 6 Kohlenstoffatome aufweist, und insbesondere eine Methyl- und/ oder Ethylgruppe.

**9.** Zusammensetzung nach einem der Ansprüche 1 bis 7, in der der organische Farbstoff der Formel (IIa) entspricht, in der bedeuten:

* $R'_1$ Wasserstoff oder eine Gruppe -COOR, wobei R ein gesättigter Kohlenwasserstoffrest, der 1 bis 12, vorzugsweise 1 bis 6 Kohlenstoffatome aufweist, und insbesondere ein Methylrest oder Ethylrest ist;
und/oder
* $R_5$ und $R_6$ unabhängig voneinander (i) eine Gruppe der Formel (I-Ia)

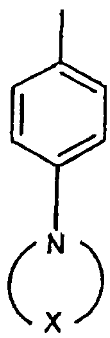

(IIa),

in der der Ring, der N und X enthält, ein gesättigter Ring ist, der insgesamt 4 bis 7 Atome, insbesondere 5 bis 6 Atome, der Stickstoff eingeschlossen, und insbesondere 4 bis 5 Kohlenstoffatome und 0 bis 1 Sauerstoffatome enthält; und vor allem eine Gruppe der Formel:

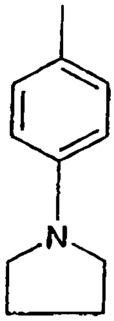

oder

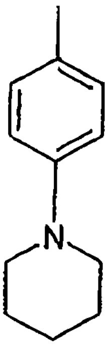

oder

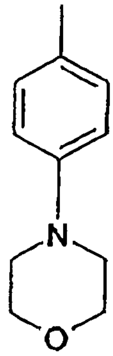

oder (ii) eine geradkettige, verzweigte oder cyclische, gesättigte oder ungesättigte Kohlenwasserstoffgruppe, die 5 bis 14, vorzugsweise 6 bis 10 Kohlenstoffatome aufweist, die gegebenenfalls 1 bis 2 Heteroatome enthält, die unter N, O und S ausgewählt werden;
vor allem eine Gruppe:

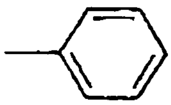

oder

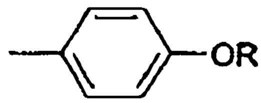

oder

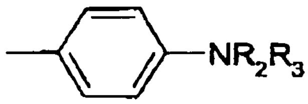

in denen R ein gesättigter Kohlenwasserstoffrest, der 1 bis 12, vorzugsweise 1 bis 6 Kohlenstoffatome aufweist und insbesondere ein Methylrest oder Ethylrest ist; wobei $R_2$ und $R_3$ unabhängig voneinander eine geradkettige, verzweigte oder cyclische, gesättigte oder ungesättigte Kohlenwasserstoffgruppe, die gegebenenfalls 1 bis 5 Heteroatome enthält, die unter N, O, S, Si und P ausgewählt werden;
und/oder
* $R'_2$ Wasserstoff oder eine Gruppe -NR'R", wobei R' und R", gleich oder verschieden, eine gesättigte, geradkettige oder verzweigte Kohlenwasserstoffgruppe, die 1 bis 12, vorzugsweise 1 bis 6 Kohlenstoffatome aufweist, und insbesondere eine Methylgruppe und/oder Ethylgruppe bedeuten; oder eine Gruppe

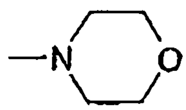 .

**10.** Zusammensetzung nach einem der vorhergehenden Ansprüche, in der der organische Farbstoff ausgewählt wird unter:

- dem 3,3-Di-(4-methoxyphenyl)-6-morpholino-3H-naphtho[2,1-b]-pyran der Formel:

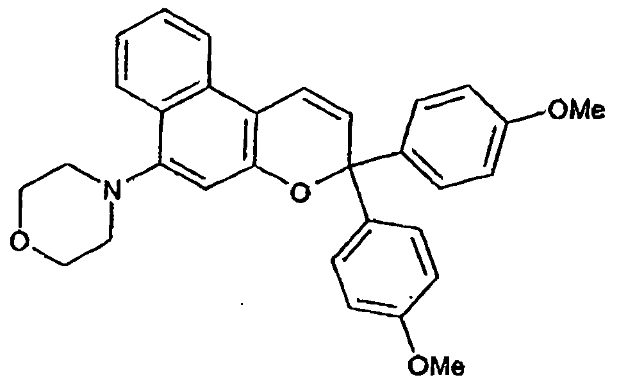

- dem 3-Phenyl-3-(4-morpholinophenyl)-6-morpholino-3H-naphtho[2,1-b]pyran der Formel:

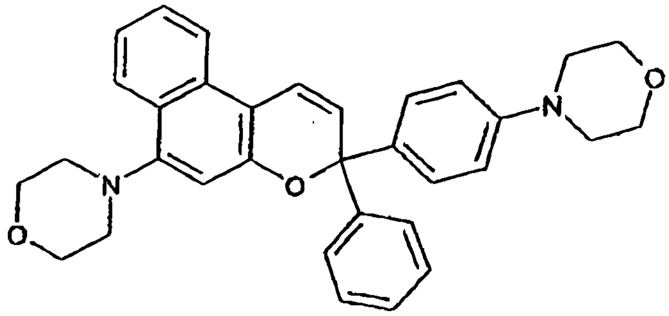

- dem 3-Phenyl-3-(4-piperidinophenyl)-6-morpholino-3H-naphtho[2,1-b]pyran der Formel:

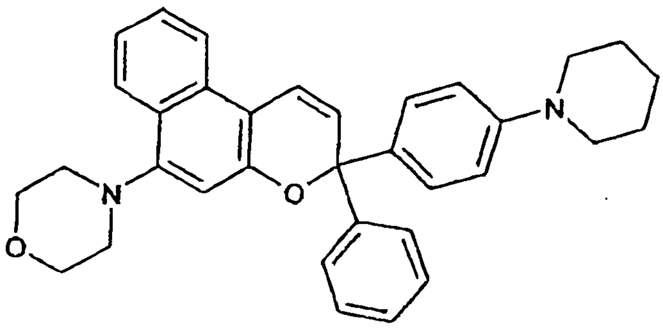

- dem 3-Phenyl-3-(4-piperidinophenyl)-5-methoxycarbonyl-9-N-dimethyl-3H-naphtho[2,1-b]pyran der Formel:

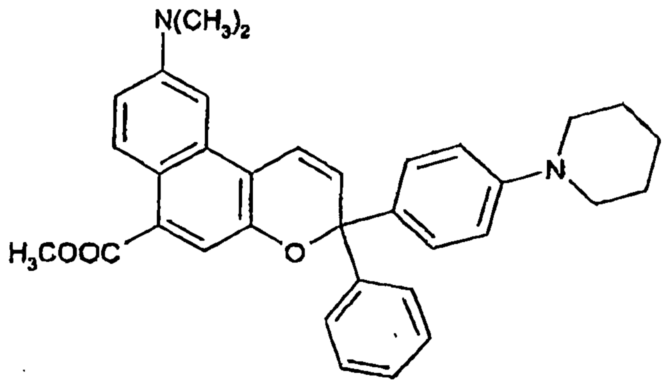

- dem 2-Phenyl-2-(4-piperidinophenyl)-5-methoxycarbonyl-9-N-dimethyl-2H-naphto[1,2-b]pyran der Formel:

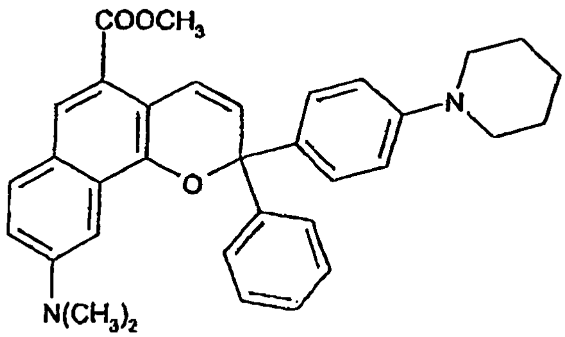

- und ihren Gemischen.

**11.** Zusammensetzung nach einem der vorhergehenden Ansprüche, in der der erfindungsgemäße organische Farbstoff, allein oder im Gemisch, in einer Menge von 0,001 bis 20 Gew.-%, insbesondere 0,005 bis 10 Gew.-%, vorzugsweise 0,01 bis 4,8 Gew.-% und noch besser 0,05 bis 3 Gew.-%, vorzugsweise 0,1 bis 2 Gew.-%, bezogen auf das Gesamtgewicht der kosmetischen Zusammensetzung, enthalten ist.

**12.** Zusammensetzung nach einem der vorhergehenden Ansprüche, in der die Ölphase polar ist und bei 25 °C einen mittleren Löslichkeitsparameter $\delta_a$ gemäß dem Hansen-Löslichkeitsraum von 5,0 $(J/cm^3)^{1/2}$ oder darüber, insbesondere von 5,3 oder darüber, besser von 5,5 oder darüber und vor allem von 6,0 $(J/cm^3)^{1/2}$ oder darüber und noch besser von 7,0 $(J/cm^3)^{1/2}$ oder darüber aufweist.

**13.** Zusammensetzung nach einem der vorhergehenden Ansprüche, in der die Ölphase 5 bis 100 Gew.-%, insbesondere 10 bis 90 Gew.-%, besser 15 bis 60 Gew.-% und noch besser 20 bis 50 Gew.-% des Gesamtgewichts der Ölphase eines oder mehrerer polarer Öle enthält, die bei 25 °C einen mittleren Löslichkeitsparameter $\delta_a$ gemäß dem Hansen-Löslichkeitsraum von 5,0 $(J/cm^3)^{1/2}$ oder darüber aufweisen.

**14.** Zusammensetzung nach einem der Ansprüche 1 bis 11, in der die Ölphase unpolar ist und bei 25 °C einen mittleren Löslichkeitsparameter $\delta_a$ gemäß dem Hansen-Löslichkeitsraum kleiner als 5,0, insbesondere kleiner als oder gleich 4,9, noch besser kleiner als oder gleich 4,5 und noch besser kleiner als oder gleich 4,0 $(J/cm^3)^{1/2}$ aufweist.

**15.** Zusammensetzung nach einem der Ansprüche 1 bis 11 und 14, in der die Ölphase 5 bis 100 Gew.-%, insbesondere 10 bis 90 %, besser 15 bis 60 % und noch besser 20 bis 50 Gew.-% des Gesamtgewichts der Ölphase eines oder mehrerer unpolarer Öle enthält, die bei 25 °C einen mittleren Löslichkeitsparameter $\delta_a$ gemäß dem Hansen-Löslichkeitsraum kleiner als 5,0 $(J/cm^3)^{1/2}$ aufweisen.

**16.** Zusammensetzung nach einem der vorhergehenden Ansprüche, in wobei die Ölphase mindestens ein Öl enthält, das ausgewählt wird unter:

- den tierischen oder pflanzlichen Ölen, die aus den Estern einer Fettsäure und von Polyolen gebildet werden, vor allem den flüssigen Triglyceriden, zum Beispiel Sonnenblumenöl, Maisöl, Sojaöl, Kürbiskernöl, Traubenkernöl, Sesamöl, Haselnussöl, Aprikosenöl, Mandelöl oder Avocadoöl, den Fischölen, Glycerintricaprocaprylat, oder den pflanzlichen oder tierischen Ölen der Formel $R_1COOR_2$, in der $R_1$ den Rest einer höheren Fettsäure darstellt, die 7 bis 19 Kohlenstoffatome aufweist, und $R_2$ eine verzweigte Kohlenwasserstoffkette darstellt, die 3 bis 20 Kohlenstoffatome enthält, zum Bespiel Purcellinöl; Paraffinöl, Vaselineöl, Calophyllumöl, Macadamiaöl, Rapsöl, Kopraöl, Erdnussöl, Palmöl, Ricinusöl, Jojobaöl, Olivenöl oder Getreidekeimöl; Sheabutteröl; Perhydrosqualen;
- den synthetischen Estern und Ethern insbesondere von Fettsäuren, wie den Ölen der Formel $R_1COOR_2$, in der $R_1$ den Rest einer höheren Fettsäure darstellt, die 7 bis 29 Kohlenstoffatome aufweist, und $R_2$ eine Kohlenwasserstoffkette darstellt, die 3 bis 30 Kohlenstoffatome enthält, wie zum Beispiel Isopropylmyristat, 2-Ethylhexylpalmitat, 2-Octyldodecylstearat, 2-Octyldodecylerucat, Isononylisononanoat, Isostearylisostearat, den hydroxylierten Estern, wie Isostearyllactat, Octylhydroxystearat, Octyldodecylhydroxystearat, Düsostearylmalat, Triisocetylcitrat, die Heptanoate, Octanoate, Decanoate von Fettalkoholen; den Estern von Polyolen, wie Propylenglykoldioctanoat, Neopentylglykoldiheptanoat, Diethylenglykoldiisononanoat; den Estern von Pentaerythrit, wie Pentyaerythrityltetraisostearat; den Estern vom Tridecyltrimellitattyp;
- den Fettalkoholen, die 12 bis 26 Kohlenstoffatome aufweisen, wie Octyldodecanol, 2-Butyloctanol, 2-Hexyldecanol, 2-Undecylpentadecanol, Oleylalkohol;
- den geradkettigen oder verzweigten Kohlenwasserstoff mineralischer oder synthetischer Herkunft, wie den Paraffinölen und ihren Derivaten, Vaseline, den Polydecenen, hydriertem Polyisobuten, wie Parleam; den Isoparaffinen, wie Isohexadecan und Isodecan;
- den Glyceriden und insbesondere den Acetylglyceriden oder Triglyceriden von Fettsäuren, die 4 bis 10 Kohlenstoffatome aufweisen, wie den Triglyceriden von Heptansäure, Octansäure und Caprinsäure/ Caprylsäure.

**17.** Zusammensetzung nach einem der vorhergehenden Ansprüche, in der die Ölphase mindestens ein Öl enthält, das unter Octyldodecanol, Hexyldecanol, Octyldecanol, Oleylalkohol, Ricinusöl, Diisostearylmalat, Glycerintriheptanoat, Glycerintrioctanoat, dem Triglycerid von Caprinsäure/Caprylsäure, Triisononanoin, Tridecyltrimellitat, den aliphatischen Kohlenwasserstoffen, insbesondere mit 6 bis 40 Kohlenstoffatomen, wie den flüchtigen Paraffinölen, wie Isohexadecan oder Isododecan, oder den nicht flüchtigen Paraffinölen, und ihren Derivaten; Vaseline, den hydrierten oder nicht hydrierten Polydecenen, hydriertem Polyisobuten, wie Parleamöl, Squalan, den Polybutylenen, Isononylisononanoat; den fluorierten Ölen, insbesondere perfluorierten Ölen, und deren Gemischen ausgewählt wird.

**18.** Zusammensetzung nach einem der Ansprüche 1 bis 11, in der die Ölphase mindestens ein phenyliertes Siliconöl der Formel (VI)

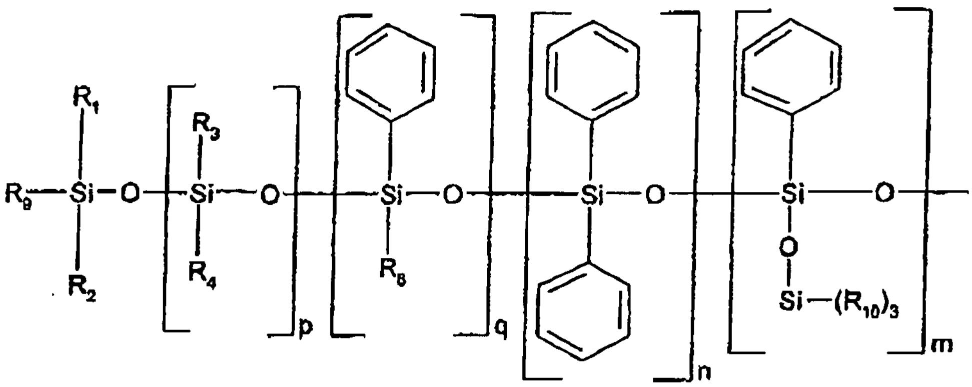

(VI)

oder ein Gemisch derartiger Öle enthält, in der bedeuten:

- $R_1$ bis $R_{10}$ unabhängig voneinander gesättigte oder ungesättigte, geradkettige, cyclische oder verzweigte $C_{1-30}$-Kohlenwasserstoffreste,

- m, n, p und q unabhängig voneinander ganze Zahlen, die im Bereich von 0 bis 900 liegen, mit der Maßgabe, dass die Summe 'm+n+q' verschieden von 0 ist.

**19.** Zusammensetzung nach Anspruch 18, in der in dem phenylierten Siliconöl der Formel (VI) die Summe 'm+n+q' im Bereich von 1 bis 100 liegt und/oder die Summe 'm+n+q' im Bereich von 1 bis 900 oder besser 1 bis 800 liegt.

**20.** Zusammensetzung nach einem der Ansprüche 18 bis 19, in der das phenylierte Siliconöl die Formel (VII)

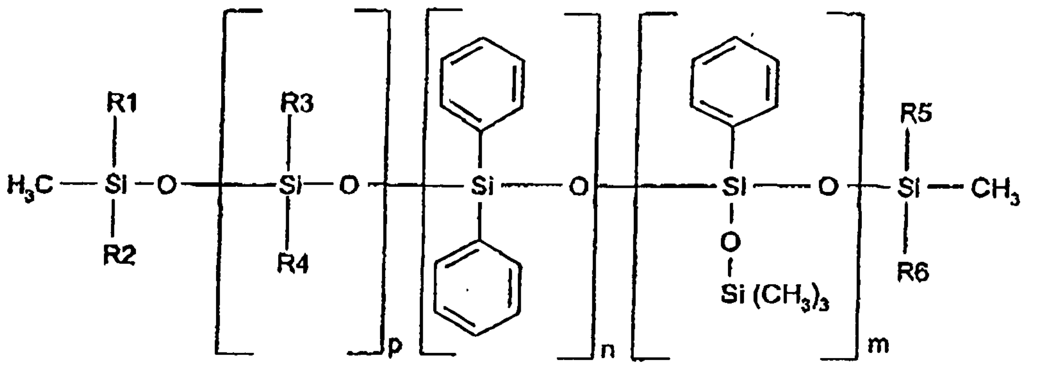

(VII)

aufweist, in der bedeuten:

- $R_1$ bis $R_6$ unabhängig voneinander gesättigte oder ungesättigte, geradkettige, cyclische oder verzweigte $C_{1-30}$-Kohlenwasserstoffreste,
- m, n und p unabhängig voneinander ganze Zahlen, die im Bereich von 0 bis 100 liegen, mit der Maßgabe, dass die Summe 'n + m' im Bereich von 1 bis 100 liegt.

**21.** Zusammensetzung nach einem der Ansprüche 18 bis 20, wobei in den Formeln (VI) und (VII) die Reste $R_1$ bis $R_6$ unabhängig voneinander einen gesättigten, geradkettigen oder verzweigten $C_{1-30}$-Kohlenwasserstoffrest, insbesondere $C_{1-12}$-Kohlenwasserstoffrest und vor allem Methyl, Ethyl, Propyl oder Butyl bedeuten.

**22.** Zusammensetzung nach einem der Ansprüche 18 bis 21, wobei die Reste $R_1$ bis $R_6$ in den Formeln (VI) und (VII) identisch sind und Methyl bedeuten.

**23.** Zusammensetzung nach einem der Ansprüche 18 bis 22, wobei das phenylierte Siliconöl unter den Phenyltrimethiconen, den Phenyldimethiconen, den Phenyltrimethylsiloxydiphenylsiloxanen, den Diphenyldimethiconen, den Diphenylmethyldiphenyltrisiloxanen und deren Gemischen ausgewählt wird.

**24.** Zusammensetzung nach einem der Ansprüche 18 bis 23, wobei das phenylierte Siliconöl in einer Menge enthalten ist, die im Bereich von 1 bis 99 Gew.-%, insbesondere 5 bis 90 Gew.-%, vorzugsweise 7 bis 50 Gew.-%, besser 12 bis 40 Gew.-%, bezogen auf das Gesamtgewicht der kosmetischen Zusammensetzung, liegt.

**25.** Zusammensetzung nach einem der Ansprüche 18 bis 24, wobei die Ölphase der Zusammensetzung ein oder mehrere weitere zusätzliche, kosmetisch akzeptable, polare oder unpolare, flüchtige oder nicht flüchtige, siliconhaltige oder kohlenwasserstoffhaltige Öle enthält, das in einer Menge enthalten sein kann, die im Bereich von 0,1 bis 50 Gew.-%, insbesondere 4 bis 30 Gew.-%, vorzugsweise 10 bis 25 Gew.-%, bezogen auf das Gesamtgewicht der kosmetischen Zusammensetzung, liegt.

**26.** Zusammensetzung nach einem der vorhergehenden Ansprüche, wobei die Ölphase in einer Menge enthalten ist, die im Bereich von 1 bis 99 Gew.-%, insbesondere 10 bis 90 Gew.-%, vorzugsweise 15 bis 80 Gew.-%, bezogen auf das Gesamtgewicht der kosmetischen Zusammensetzung, enthalten ist.

**27.** Zusammensetzung nach einem der vorhergehenden Ansprüche, die außerdem andere Fettsubstanzen, die unter den Wachsen, den Gummen und/oder den pastösen Fettsubstanzen tierischer, pflanzlicher, mineralischer oder synthetischer Herkunft sowie ihren Gemischen ausgewählt werden; und/oder eine Partikelphase, die Pigmente,

Perlglanzpigmente, Füllstoffe, Farbstoffe und/oder andere photochrome Verbindungen enthalten kann; und/oder UV-Filter; und/oder eine wässrige Phase; und/oder einen grenzflächenaktiven Stoff und/oder ein Verdickungsmittel und/oder ein filmbildendes Polymer enthält.

**28.** Zusammensetzung nach einem der vorhergehenden Ansprüche, die ein ΔE von 5 oder darüber, vorzugsweise 10 oder darüber, besser 25 oder darüber, noch besser 35 oder darüber oder sogar von 45 oder darüber aufweist.

**29.** Zusammensetzung nach einem der vorhergehenden Ansprüche, die in Form eines Produkts zum Pflegen und/oder Schminken der Haut des Körpers oder des Gesichts, der Lippen und der Haare, in Form eines Sonnenschutzmittels oder Selbstbräunungsmittels, eines Produkts zur Haarbehandlung vorliegt.

**30.** Zusammensetzung nach einem der vorhergehenden Ansprüche, die in Form eines Lippenstifts, eines Make-ups, eines Rouge oder eines Lidschatten, eines losen oder kompakten Pulvers, einer gefärbten Creme, eines Produkts zum Schminken des Körpers, eines Produkts zum Färben der Haut, eines Eyeliners oder eines Mascaras vorliegt.

**31.** Verfahren zur kosmetischen Behandlung eines Trägers, der unter den Schleimhäuten, den Halbschleimhäuten, der Haut und/oder den Hautanhangsgebilden ausgewählt wird, bei dem eine kosmetische Zusammensetzung, die wie in einem der Ansprüche 1 bis 30 definiert ist, auf diesen Träger aufgetragen wird.

**32.** Verfahren zum Schminken und/oder vorübergehenden Färben eines Trägers, der unter den Schleimhäuten, den Halbschleimhäuten, der Haut und/oder den Hautanhangsgebilden ausgewählt wird, bei dem eine kosmetische Zusammensetzung, die wie in einem der Ansprüche 1 bis 30 definiert ist, auf diesen Träger aufgetragen wird.